# EUROPÄISCHE PATENTSCHRIFT

(11) **EP 1 246 825 B1**
(45) Veröffentlichungstag und Bekanntmachung des Hinweises auf die Patenterteilung: **17.12.2003**
(21) Anmeldenummer: 01902310.0
(22) Anmeldetag: 12.01.2001
(51) Int. Cl.: C07D 487/04, A61K 31/407, A61P 29/00

(54) **ANELLIERTE PYRROLVERBINDUNGEN, DIESE ENTHALTENDE PHARMAZEUTISCHE MITTEL UND DEREN VERWENDUNG**
FUSED PYRROLE COMPOUNDS, PHARMACEUTICAL AGENTS CONTAINING THE SAME, AND THE USE THEREOF
COMPOSES DE PYRROLE ANNELES, AGENTS PHARMACEUTIQUES LES CONTENANT ET LEUR UTILISATION

(30) Priorität: 13.01.2000 DE 10001166
(43) Veröffentlichungstag der Anmeldung: 09.10.2002
(73) Patentinhaber: MERCKLE GMBH, 89143 Blaubeuren (DE)
(72) Erfinder: STRIEGEL, Hans-Günter, 89134 Blaustein (DE); LAUFER, Stefan, 89143 Blaubeuren (DE); TOLLMANN, Karola, 65611 Brechen (DE); TRIES, Susanne, 89584 Ehingen (DE)
(74) Vertreter: Riedl, Peter, Dr.
(86) Internationale Anmeldenummer: EP0100332
(87) Internationale Veröffentlichungsnummer: WO01051491

(56) Entgegenhaltungen:
- DE-A- 19 624 289
- DANNHARDT, G. ET AL.: "E-2-Pyrrolizin-5-yl Acrylic Acids ..." ARCH. PHARM. (WEINHEIM), Bd. 328, 1995, Seiten 681-686, XP001000482
- WILKERSON, W. W. ET AL.: "Antiinflammatory 4,5-Diarylpyrroles ..." J. MED. CHEM., Bd. 38, 1995, Seiten 3895-3901, XP002920001 in der Anmeldung erwähnt

## Beschreibung

Die vorliegende Erfindung betrifft anellierte Pyrrolverbindungen, insbesondere Pyrrolizine, Indolizine und Heteroanaloga, mit selektiver inhibitorischer Wirkung auf das Isoenzym-2 der Prostaglandin-H-synthase (COX-2); pharmazeutische Mittel, die diese Verbindungen enthalten; und die Verwendung dieser Verbindungen zur Behandlung von Erkrankungen des rheumatischen Formenkreises.

Anerkanntermaßen greifen die Arachidonsäuremetabolite Prostaglandin E₂ (PGE₂), Prostaglandin I₂ (PGI₂) und Thromboxan B₂ (TXB₂) tief ins Entzündungsgeschehen ein. Schlüsselenzym für die Prostaglandinsynthese ist die Prostaglandin-H-synthase. Als Cyclooxygenase erzeugt sie über das Intermediat Prostaglandin G₂ (PGG₂) aus Arachidonsäure Prostaglandin H₂ (PGH₂). Das Enzym existiert in zwei Isoformen, nämlich Cyclooxygenase-1 und Cyclooxygenase-2. Da die Strukturen der beiden Isoezyme bereits aufgeklärt werden konnten, sind auch Strukturunterschiede zwischen den beiden Enzymen genauer untersucht worden.

Cyclooxygenase-1 existiert in nahezu allen Zellen und erzeugt intra- wie extrazellulär ubiquitäre Prostaglandin-Mediatoren, die in physiologischen Mengen für die Funktion von Organen, wie Magen und Nieren, erforderlich sind.

Die Hemmung dieses Enzymes unterbindet die Synthese des gefäßerweiternden PGE₂ ebenso wie die des cytoprotektiven PGI₂. Verringerte Durchblutung und fehlender protektiver Effekt können zu Schädigungen der Magenschleimhaut und in der Folge zu Ulzerationen führen. Ein verringerter renaler Blutfluß kann in Folge einer Ischämie zu Schädigungen des Nierenparenchyms und weiter zu Niereninsuffizienz führen.

Cyclooxygenase-2 dagegen wird nur in spezialisierten Zellen, z.B. in Blutzellen, wie Monozyten und Granulozyten, in Synovialzellen, Gefäßendothelzellen (venös) und lokal in entzündetem Gewebe angetroffen. Experimentell wird die Bildung (Expression) des Isoenzyms-2 durch Phorbolester, Lipopolysaccharide und andere Streßfaktoren, die lokale Entzündungen provozieren können, ausgelöst.

Nichtsteroidale Antiphlogistika (NSAIDs), wie Acetylsalicylsäure, Mefenaminsäure, Diclofenac, Indomethacin, Ibuprofen und Naproxen, befinden sich klinisch in breiter Anwendung. Pharmakologisch wirken sie über eine Hemmung der Cyclooxygenase.

Pharmakologisch ähnlich wirkende Pyrrolizinverbindungen sind aus zahlreichen Publikationen bekannt. Beispielsweise werden antiphlogistisch wirksame Pyrrolizinverbindungen beschrieben in Arch. Pharm. 319, 65-69 (1986); 319, 231-234 (1986); 318, 661-663 (1985); 318, 663-664 (1985); 319, 500-505 (1986); 319, 749-755 (1986); 327, 509-514 (1994); 328, 681-686 (1995); 330, 307-312 (1997) sowie in J. Med. Chem. 1987, 30, 820-823 und 1994, 37, 1894-1897.

Weitere Pyrrolizinverbindungen sind in der US 5,260,451 (entsprechend EP 0397175) sowie in WO 95/32970; WO 95/32971; und WO 95/32972 beschrieben. Diese Verbindungen besitzen die Strukturformel

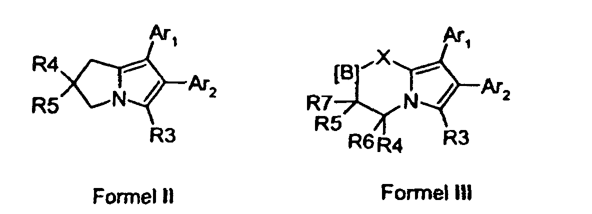

Allen Verbindungen ist ein anelliertes Diarylpyrrol-Strukturelement und ein dritter acider Rest R³ gemeinsam. Die Verbindungen zeichnen sich durch hohe Lipophilie, gute Bioverfügbarkeit und mittlere Halbwertzeiten aus, s. Drugs of the Future, 1995, 20 (10):1007-1009.

Weitere Pyrrolizinverbindungen ähnlicher Konstitution sind in der DE 198 45 446.6 und der PCT/EP 99/09057 beschreiben. Auch Alkylsulfinylbenzoyl- und Alkylsulfonylbenzoyl-substituierte Pyrrolizine sollen der US 4,232,038 zufolge antiinflammatorische, analgetische und antipyretische Eigenschaften aufweisen. Gemäß der DE 196 24 290.8 und der DE 196 24 289.4 besitzen bestimmte Verbindungen dieses Typs lipidsenkende Wirkung.

ML 3000 (1) beispielsweise, ein vicinal-diarylsubstituiertes Pyrrolderivat, wird als dualer Inhibitor der Cyclooxygenase (COX) und der 5-Lipoxygenase (LOX) beschrieben.

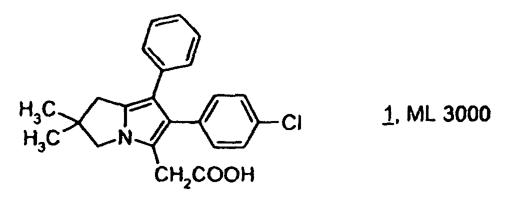

Allerdings ergeben sich über die Hemmung der Cyclooxygenase auch toxische Nebenwirkungen, die vor allem mit der Hemmung der konstitutiven, physiologischen Cyclooxygenase-1 zusammenhängen. Neuere Untersuchungen zeigen, daß die klassischen NSAIDs eine unzureichende Selektivität besitzen und häufig sogar das Isoenzym-1 der Cyclooxygenase bevorzugen.

Eine selektive Hemmung der spezifisch in entzündetem Gewebe überexprimierten und durch die Bildung unphysiologischer Gewebespiegel von Prostaglandinen zu Gewebsschädigungen führenden Cyclooxygenase-2 hingegen sollte zu einem überlegenen, nämlich nebenwirkungsärmeren, antiinflammatorischen Prinzip führen.

Substanzen, die selektiv die Cyclooxygenase-2 hemmen, wurden ebenfalls schon beschrieben.

Hierzu gehören vor allem 5-gliedrige heterocyclische Ringe mit Bisarylsubstitution. Beispielsweise nennt die WO 94/15932 3,4-Diarylthiophene, -furane und -pyrrole, mitunter das 3-(4-Methylsulfonylphenyl)-4-(4-fluorphenyl)-thiophen. Die WO 94/27980 beschreibt 4,5-Diaryloxazole, beispielsweise 4-(4-Fluorphenyl)-2-methyl-5-[4-(methylsulfonyl)-phenyl]-oxazol. In der EP 0087629 sind 2,3-Diaryl-5-halothiophene, beispielsweise 5-Brom-2,3-bis(4-fluorphenyl)thiophen, genannt und die WO 95/15315 gibt 1,5-Diphenylpyrazole, beispielsweise 5-(4-Fluorphenyl)-1-[4-(methylsulfonyl)-phenyl]-3-(trifluormethyl)-pyrazol, an. 2,3-Diarylpyrrole werden in der WO 95/00501 genauso genannt wie die unten am Beispiel der Laktone 4 und 5 veranschaulichten 3,4-Diarylfuranone.

Ferner finden sich auch cycloaliphatische oder aromatische nichtheterocyclische Ringe mit Bisarylsubstitution. Beispielsweise werden in der WO 96/10012 auch Bisarylphenyle, beispielsweise 2-[4-Methylthio)phenyl]-1-biphenyl, beschrieben. 5-gliedrige Ringe wiederum lassen sich in der WO 95/11883 als unten am Beispiel des Cyclopentens 6, (SC57666, SC 57949) veranschaulichte 1,2-Diarylcyclopentene nachlesen, an die in Anlehnung an die WO 95/21817 in 4-Position eine Spiroeinheit geknüpft, sein kann, beispielsweise wie es im 5-(4-Fluorphenyl)-6-[4-(methylsulfonyl)phenyl]spiro[2.4]hept-5-en der Fall ist. Schließlich sind auch die in WO 95/30656 beschriebenen 2,3-Diarylcyclopentadiene, beispielsweise 1-Methylsulfonyl-4-[1,1-dimethyl-4-(4-fluorphenyl)cyclopenta-2,4-dien-3-yl]-benzol zu erwähnen.

Weitere Pyrrole sind von Wilkerson et al. beschrieben in J. Med. Chem 1994, 37, 988-998 und J. Med. Chem. 1995, 38, 3895-3901. Auch die WO 99/61016 gibt bestimmte substituierte Pyrrole als Cyclooxygenase-2-Hemmer an.

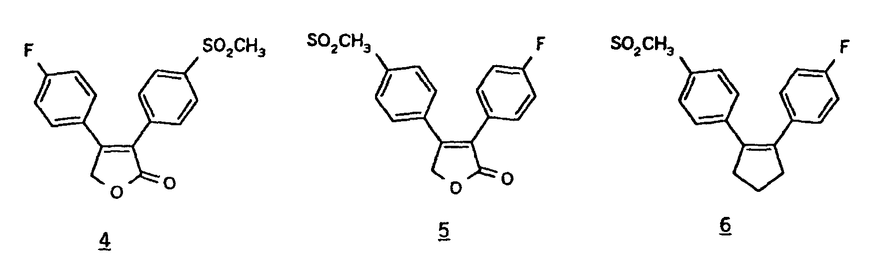

Im Fall der Lactone 4 und 5 zeigt nur Verbindung 5 eine hohe Aktivität an Cyclooxygenase-2 und hohe Selektivität für diese Isoform.

Aufgabe der vorliegenden Erfindung war es, neue Verbindungen zur Verfügung zu stellen, welche die Cyclooxygenase-2 stärker hemmen als die Cyclooxygenase-1.

Gelöst wird diese Aufgabe überraschenderweise durch [α]-anellierte Pyrrolverbindungen, die eine in para-Stellung mit bestimmten schwefelhaltigen Gruppen substituierte Phenylgruppe tragen.

Gegenstand der vorliegenden Erfindung sind daher [α]-anellierte Pyrrolverbindungen der Formel I 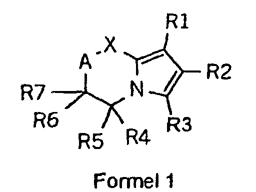 worin
- X: für CR8R9, S, O, NR12 oder C(O) steht;
- A: für CR10R11 oder eine Bindung zwischen X und dem die Reste R6 und R7 tragenden Atom steht;

der erste der Reste R1, R2, R3 für 4-substituiertes Phenyl steht, wobei der Substituent ausgewählt ist unter C₁₋₄-Alkylthio, C₁₋₄-Alkylsulfinyl, C₁₋₄-Alkylsulfonyl, Sulfamoyl, N-C₁₋₄-Alkylsulfamoyl, N,N-Di-C₁₋₄-Alkylsulfamoyl, C₁₋₄-Alkylsulfonamido oder C₁₋₄-Alkylsulfon-N-C₁₋₄-alkylamido;
der zweite der Reste R1, R2, R3 für
   C₁₋₆-Alkyl, das gegebenenfalls einfach, zweifach oder dreifach bzw. mit Halogen auch mehrfach mit unter Halogen, C₃₋₇-Cycloalkyl, C₁₋₄-Alkoxy, Trifluormethoxy, Hydroxy oder Trifluormethyl ausgewählten, gleichen oder verschiedenen Substituenten substituiert ist;
   C₃₋₇-Cycloalkyl, das gegebenenfalls mit unter Halogen, C₁₋₄-Alkyl, Halogen-C₁₋₄-alkyl, C₃₋₇-Cycloalkyl, C₁₋₄-Alkoxy, Halogen- C₁₋₄-alkoxy oder Hydroxy ausgewählten, gleichen oder verschiedenen Substituenten substituiert ist;
   Phenyl, das gegebenenfalls einfach, zweifach oder dreifach bzw. mit Halogen auch mehrfach mit unter Halogen, C₁₋₄-Alkyl, Halogen-C₁₋₄-alkyl, C₁₋₄-Alkoxy, Halogen- C₁₋₄-alkoxy, C₁₋₄-Alkylthio, Hydroxy, Nitro, C₁₋₄-Alkylsulfinyl, C₁₋₄-Alkylsulfonyl, Sulfamoyl, N-C₁₋₄-Alkylsulfamoyl, N,N-Di- C₁₋₄-Alkylsulfamoyl, C₁₋₄-Alkylsulfonamido oder C₁₋₄-Alkylsulfon-N-C₁₋₄-alkylamido ausgewählten, gleichen oder verschiedenen Substituenten substituiert ist;
      oder
   einen 5- oder 6-gliedrigen heterocyclischen Rest, der aromatisch oder nicht-aromatisch, monooder bicyclisch, und gegebenenfalls benzoanelliert sein kann und der 1, 2 oder 3, unter N, O und S unabhängig voneinander ausgewählte Heteroatome aufweist und gegebenenfalls einfach, zweifach oder dreifach bzw. mit Halogen auch mehrfach mit unter Halogen, C₁₋₄-Alkyl, Halogem-C₁₋₄-alkyl, C₁₋₄-Alkoxy, Halogen-C₁₋₄-alkoxy, C₁₋₄-Alkylthio, Hydroxy, Nitro, C₁₋₄-Alkylsulfinyl, C₁₋₄-Alkylsulfonyl, Sulfamoyl, N-C₁₋₄-Alkylsulfamoyl, N,N-Di- C₁₋₄-Alkylsulfamoyl, C₁₋₄-Alkylsulfonamido oder C₁₋₄-Alkylsulfon-N-C₁₋₄-alkylamido ausgewählten, gleichen oder verschiedenen Substituenten substituiert ist, steht;
der dritte der Reste R1, R2, R3 für
   H, C₁₋₆-Alkyl, Halogen-C₁₋₄-alkyl, Hydroxy-C₁₋₆-alkyl, -CHO, -COOH, -COCOOH, -COO-C₁₋₄-Alkyl, -COO-C₁₋₄-AlkPhenyl, -COCOO-C₁₋₄-Alkyl, Halogen, Cyano, C₁₋₄-Alkylsulfonyl, Sulfamoyl oder B-Y steht;
      worin
   B für C₁₋₈-Alkylen oder C₂₋₈-Alkenylen steht, die gegebenenfalls durch Hydroxyl oder C₁₋₄-Alkoxy substituiert sein können;
   Y für -COOH, -COO-C₁₋₄-Alkyl, -SO₃-C₁₋₄-Alkyl, -CHO oder Hydroxy steht; oder der zweite und der dritte der Reste R1, R2, R3 zusammen mit den C-Atomen, an die sie gebunden sind, für gesättigtes oder ungesättigtes C₃₋₇-Cycloalkyl stehen;
   R4-R11, die gleich oder verschieden sein können, für Wasserstoff C₁₋₆-Alkyl, Hydroxy-C₁₋₆-alkyl, C₁₋₄-Alkoxy-C₁₋₆-alkyl, Hydroxyl, COOH oder Acyloxy stehen, wobei vicinale Reste auch für Bindungen oder geminale Reste auch zusammen mit dem C-Atom, an das sie gebunden sind, für Carbonyl oder für C₃₋₇-Cycloalkyl stehen können;
   R12 für Wasserstoff, C₁₋₆-Alkyl oder Phenyl steht,
und optische Isomere, physiologisch verträgliche Salze und physiologisch hydrolysierbare Ester davon, wobei die physiologisch hydrolysierbaren Ester ausgewählt sind unter Alkyl-, Pivaloyloxymethyl-, Acetoxymethyl-, Phthalidyl-, Indanyl- und Methoxymethylester.

Bevorzugte Ausführungsformen dieses Gegenstandes sind in den anliegenden Ansprüchen beschrieben.

Die physiologisch verträglichen Salze können im vorliegenden Fall Säureadditions- oder Basenadditionssalze sein.

Für Säureadditionssalze verwendet man anorganische Säuren, wie Salzsäure, Schwefelsäure, Salpetersäure oder Phosphorsäure, oder organische Säuren, insbesondere Carbonsäuren, z.B. Essigsäure, Weinsäure, Milchsäure, Citronensäure, Äpfelsäure, Mandelsäure, Ascorbinsäure, Maleinsäure, Fumarsäure, Gluconsäure oder Sulfonsäuren, z. B. Methansulfonsäure, Benzolsulfonsäure und Toluolsulfonsäure, und dergleichen.

Zu Basenadditionssalzen zählen Salze der Verbindungen der Formel I mit anorganischen Basen, wie Natrium- oder Kaliumhydroxid oder mit organischen Basen, wie Mono-, Di- oder Triethanolamin.

Soweit die erfindungsgemäßen Verbindungen Asymmetriezentren aufweisen, sind Racemate sowie optische Isomere als Gemische oder in Reinform (Enantiomere, Diastereomere) umfasst.

Der Begriff "Alkyl, Alkoxy etc." umfasst geradkettige oder verzweigte Alkylgruppen, wie CH₃, C₂H₅, n-Propyl, CH(CH₃)₂, n-Butyl, CH(CH₃)-C₂H₅, Isobutyl, C(CH₃)₃, n-Pentyl oder n-Hexyl, insbesondere CH₃, C₂H₅ oder CH(CH₃)₂, vorzugsweise mit - soweit nicht anderes angegeben ist - 1 bis 8, insbesondere 1 bis 6 und besonders bevorzugt 1 bis 4 Kohlenstoffatomen; als Substituent einer der Reste R1 bis R12 umfaßt "Alkyl, Alkoxy etc." vorzugsweise 1 bis 4 Kohlenstoffatome.

### Zu substituiertem "Alkyl, Alkoxy etc." gehören insbesondere:

Halogenalkyl, d.h. Alkyl, das partiell oder vollständig durch Fluor, Chlor, Brom und/oder Iod substituiert ist, also z.B. CH₂F, CHF₂, CF₃, CH₂Cl, 2-Fluorethyl, 2-Chlorethyl oder 2,2,2-Trifluorethyl; als Substituent einer der Reste R1 bis R12 bedeutet Halogenalkyl vorzugsweise CHF₂ und vor allem CF₃;

Halogenalkoxy, d.h. Alkoxy, das partiell oder vollständig durch Fluor, Chlor, Brom und/oder Iod substituiert ist, also z.B die den vorstehend aufgezählten Halogenalkylresten entsprechenden Halogenalkoxyreste; als Substituent einer der Reste R1 bis R12 bedeutet Halogenalkoxy vorzugsweise OCHF₂ und vor allem OCF₃;

Alkoxyalkyl, d.h. durch Alkoxy substituiertes Alkyl, also z.B. -CH₂-OCH₃ oder 2-Methoxyethyl;

Hydroxyalkyl, d.h. Alkyl, das vorzugsweise einfach durch Hydroxy substituiert ist, z.B. Hydroxymethyl oder 2-Hydroxyethyl;

Trifluormethylalkyl, d.h. Alkyl, das vorzugsweise einfach durch Trifluormethyl substituiert ist, z.B. die unter Hydroxyalkyl beschriebenen Reste, die mit Trifluormethyl anstatt mit Hydroxy substituiert sind;

Trifluormethoxyalkyl, d.h. Alkyl, das vorzugsweise einfach durch Trifluormethoxy substituiert ist, z.B. die unter Hydroxyalkyl beschriebenen Reste, die mit Trifluormethoxy anstatt mit Hydroxy substituiert sind;

Cycloalkylalkyl, d.h. Alkyl, das vorzugsweise einfach durch Cycloalkyl substituiert ist, z.B. die unter Hydroxyalkyl beschriebenen Reste, die mit Cyclopropyl, Cyclobutyl, Cyclopentyl oder Cyclohexyl anstatt mit Hydroxy substituiert sind.

Der Begriff "Cycloalkyl" umfasst mono- oder bicyclische Alkylgruppen, wie Cyclopropyl, Cyclobutyl, Cyclopentyl, Cyclohexyl, etc., vorzugsweise mit - soweit nicht anderes angegeben ist - 3 bis 9, insbesondere 3 bis 7 und besonders bevorzugt 5 oder 6 Kohlenstoffatomen.

Der Begriff "Alkylen" umfasst geradkettige oder verzweigte Alkylengruppen, wie Methylen und Ethylen, vorzugsweise mit - soweit nicht anderes angegeben ist - 1 bis 8, insbesondere 1 bis 6 und besonders bevorzugt 1 bis 4 Kohlenstoffatomen. Ist Alkylen durch Hydroxyl oder Alkoxy substituiert, so ist eine einfache Substitution bevorzugt.

Der Begriff "Alkenylen" umfasst geradkettige oder verzweigte, einfach oder mehrfach ungesättigte Alkylengruppen, wie Ethenylen, vorzugsweise mit - soweit nicht anderes angegeben ist - 2 bis 8, insbesondere 2 bis 6 und besonders bevorzugt 2 bis 4 Kohlenstoffatomen. Ist Alkenylen durch Hydroxyl oder Alkoxy substituiert, so ist eine einfache Substitution bevorzugt.

Acyloxy meint -OCOR, worin R für Alkyl oder Aryl stehen kann. Insbesondere sind Acetyloxy und Benzoyloxy zu nennen.
-COOAlkyl meint Alkoxycarbonyl, wie CO-OCH₃, CO-OC₂H₅, CO-OCH₂-C₂H₅, CO-OCH(CH₃)₂, n-Butoxycarbonyl, CO-OCH(CH₃)-C₂H₅, CO-OCH₂-CH(CH₃)₂, CO-OC(CH₃)₃, insbesondere CO-OCH₃, CO-OC₂H₅, CO-OCH(CH₃)₂ oder CO-OCH₂-CH(CH₃)₂.
-COOAlkPhenyl meint eine am Alkylrest mit Phenyl substituierte Alkoxycarbonylgruppe, wie Benzyloxycarbonyl.
-COCOOAlkyl meint einen Alkylester einer Carboxycarbonylgruppe.

Alkylthio meint -S-Alkyl und wird auch als Alkylsulfanyl oder Alkylmercapto bezeichnet, wie SCH₃, SC₂H₅, SCH₂-C₂H₅, SCH(CH₃)₂, n-Butylthio, 1-Methylpropylthio, 2-Methylpropylthio, SC(CH₃)₃. Bevorzugt ist Methylthio.

Alkylsulfinyl meint -S(O)-Alkyl und wird auch als Alkylsulfoxo bezeichnet, wie SO-CH₃, SO-C₂H₅, n-Propylsulfinyl, 1-Methylethylsulfinyl, n-Butylsulfinyl, 1-Methylpropylsulfinyl, 2-Methylpropylsulfinyl, 1,1-Dimethylethylsulfinyl, Bevorzugt ist Methylsulfinyl.

Alkylsulfonyl meint -S(O)₂-Alkyl und wird auch als Alkylsulfon bezeichnet, wie SO₂-CH₃, SO₂-C₂H₅, n-Propylsulfonyl, SO₂-CH(CH₃)₂, n-Butylsulfonyl, 1-Methylpropylsulfonyl, 2-Methylpropylsulfonyl, SO₂-C(CH₃)₃. Bevorzugt ist Methylsulfonyl.

Sulfamoyl meint -S(O)₂NH₂ und wird auch als Amidosulfonyl oder Sulfonsäureamid bezeichnet.

N-Alkylsulfamoyl meint monosubstituiertes Sulfamoyl -S(O)₂NH-Alkyl, z.B. -S(O)₂NH-CH₃.

N,N-Dialkylsulfamoyl meint disubstituiertes Sulfamoyl -S(O)₂N-(Alkyl)₂, wobei die beiden stickstoffbegundenen Alkylreste gleich oder verschieden sein können, z.B. -S(O)₂N(CH₃)₂.

Alkylsulfonamido meint -NHS(O)₂-Alkyl, wie NHSO₂-CH₃, NHSO₂-C₂H₅, n-Propylsulfonamido, NHSO₂-CH(CH₃)₂, n-Butylsulfonamido, 1-Methylpropylsulfonamido, 2-Methylpropylsulfonamido, NHSO₂-C(CH₃)₃. Bevorzugt ist Methylsulfonamido.

Alkylsulfon-N-alkylamido meint -N(Alkyl)S(O)₂-Alkyl, wobei der stickstoffgebundene und der schwefelgebundene Alkylrest gleich oder verschieden sein können, z.B. N(CH₃)SO₂-CH₃.

Carbonyl, CHO, -COOH, -COCOOH, -SO₃H meint >C=O, Formyl, Carboxy, Carboxycarbonyl bzw. Sulfo.

"Aryl" steht vorzugsweise für Naphthyl und insbesondere für Phenyl.

Der Begriff "Halogen" umfasst ein Fluor-, Chlor-, Brom- oder Iodatom und insbesondere ein Fluor-, Chlor- oder Bromatom. Bevorzugt sind in der Regel Fluor- und Chloratome, gegebenenfalls auch Bromatome.

Bei dem "heterocyclischen Rest" handelt es sich insbesondere um einen 5- oder 6-gliedrigen heterocyclischen Rest, der aromatisch oder nicht-aromatisch, mono- oder bicyclisch, und/oder benzoanelliert sein kann. Zu den aromatischen Resten gehören stickstoffhaltige heterocyclische Reste, wie Pyrrolyl, Imidazolyl, Pyrazolyl, Pyridazinyl, Pyrazinyl, Indolyl, Chinolinyl, vor allem Pyridyl, Pyrimidyl und Isochinolinyl. Zu den aromatischen Resten gehören auch diejenigen heterocyclischen Reste, die ein Sauerstoffatom oder ein Schwefelatom enthalten, wie Thienyl, Benzothienyl, Furanyl und vor allem Benzofuranyl. Auch gehören dazu heterocyclische Reste, die zwei oder mehrere verschiedene Heteroatome enthalten, wie Thiazolyl, Isothiazolyl, Thiadiazolyl, Isoaazolyl und Oxazolyl. Bevorzugte aromatische heterocyclische Reste sind Thienyl, Pyridyl und Thiazolyl.
Zu den nicht-aromatischen Resten gehören stickstoffhaltige heterocyclische Reste, wie Piperidinyl und Piperazinyl. Hierzu gehören auch heterocyclische Reste, die zwei oder mehrere verschiedene Heteroatome enthalten, wie Morpholinyl.

Substituierte Reste, insbesondere Alkyl, Cycloalkyl, Aryl und Heteroaryl, sind vorzugsweise einfach, zweifach oder dreifach substituiert.

Für die Substitution mit Halogen gilt darüber hinaus, daß auch poly- und insbesondere perhalogenierte, vor allem poly- und insbesondere perfluorierte Reste erfaßt sein sollen. Bevorzugte Reste dieser Art sind poly- und insbesondere perhalogneierte aliphatische Reste, bei denen 2 oder mehrere bzw. insbesondere sämtliche C-gebundene Wasserstoffatome durch ein Halogen- und insbesondere ein Fluortom ersetzt sind. Hier sind vor allem poly- und insbesondere perhalogeniertes Alkly zu nennen, wie Trifluormethyl, 2,2,2-Trifluorethyl, Perfluorethyl, 3,3,3-Trifluorpropyl, 2,2,3,3,3-Pentafluorpropyl, Perfluorpropyl, 1,1,1-Trifluorisopropyl, 1,1,1,3,3,3-Hexafluorisopropyl, Perfluorisopropyl, 4,4,4-Trifluorbutyl, 3,3,4,4,4-Pentafluorbutyl, 2,2,3,3,4,4,4-Heptafluorbutyl, Perfluorbutyl, Perfluorisobutyl, Perfluor-secbutyl und Perfluor-t-butyl bzw. die entsprechenden poly- und insbesondere perchlorierten Reste.

Der [α]-Anelland kann 6- oder vor allem 5-gliedrig, heterocyclisch oder vor allem alicyclisch, falls alicyclisch, dann ungesättigt oder vor allem gesättigt, und/oder mithin substituiert oder unsubstituiert sein.

Zu den erfindungsgemäßen [α]-anellierten Pyrrolverbindungen der Formel I gehören insbesondere diejenigen, worin X für CR8R9 und A für eine Bindung zwischen X und dem die Reste R6 und R7 tragenden Atom stehen (Pyrrolizine); X für CR8R9 und A für CR10R11 stehen (Indolizine); X für NR12 und A für eine Bindung zwischen X und dem die Reste R6 und R7 tragenden Atom stehen (Pyrsolo[1,2-a]imidazole); X für S und A für eine Bindung zwischen X und dem die Reste R6 und R7 tragenden Atom stehen (Pyrrolo[2,1-b]thiazole); X für S und A für CR10R11 stehen (Pyrrolo[2,1-b]1,3-thiazine); X für O und A für CR10R11 stehen (Pyrrolo[2,1-b]1,3-oxazine); X für O und A für eine Bindung zwischen X und dem die Reste R6 und R7 tragenden Atom stehen (Pyrrolo[2,1-b]oxazole), wobei die nicht genannten Reste die oben angegebenen Bedeutungen haben können.

Ist der [α]-Anelland ein 5-gliedriger ungesättigter Rest, so stehen vornehmlich R4 und R6 für eine Bindung, wie beispielsweise im Pyrrolizin, Pyrrolo[2,1-b]imidazol und Pyrrolo[2,1-b]thiazol. Ist der [α]-Anelland ein 6-gliedriger ungesättigter Rest, so stehen vornehmlich R4 und R6, wie beispielsweise im Pyrrolo[2,1-b]1,3-thiazin, Pyrrolo[2,1-b]1,3-oxazin oder 5,6-Dihydroindolizin, und gegebenfalls auch R8 und R10 für eine Bindung, wie beispielsweise im Indolizin.

Ohne an einen bestimmten [α]-Anellanden gebunden zu sein, stehen gemäß einer besonderen Ausführungsform der vorliegenden Erfindung R4-R11, die gleich oder verschieden sein können, für Wasserstoff oder Alkyl. Gemäß einer anderen besonderen Ausführungsform steht wenigstens einer der Reste R4, R5, R6 und R7 für Hydroxyalkyl, insbesondere Hydroxymethyl, und die anderen der Reste R4, R5, R6 und R7 stehen unabhängig voneinander für H oder Alkyl, wobei vorzugsweise R4 für Hydroxyalkyl, insbesondere Hydroxymethyl, R5 für H oder Alkyl und R6 und R7 unabhängig voneinander für H oder Alkyl stehen. Gemäß einer weiteren besonderen Ausführungesform steht einer der Reste R8 und R9 für H, Alkyl, Hydroxyalkyl oder Alkoxyalkyl und der andere für Hydroxyl, Alkoxy, Carboxyl oder Acyloxy, oder R8 und R9 stehen zusammen mit dem Kohlenstoffatom, an das sie gebunden sind, für eine Carbonylgruppe.

Bevorzugt sind 6,7-Dihydro-5H-pyrrolizine, d.h. Verbindungen der Formel I, worin X für CR8R9, A für eine Bindung zwischen X und dem die Reste R6 und R7 tragenden Atom und R4, R5, R6, R7, R8, R9, die gleich oder verschieden sein können, die oben genannten Bedeutungen haben und vorzugsweise für Wasserstoff oder Alkyl stehen. Insbesondere sind 6,7-Dihydro-5H-pyrrolizine zu nennen, in denen R4 bis R9 allesamt Wasserstoff sind oder wenigstens einer der Reste R4 bis R9, beispielsweise R6 und/oder R8 für Alkyl, insbesondere für Methyl, stehen.

Erfindungsgemäß steht einer der Reste R1, R2, R3 für 4-substituiertes Phenyl (para-substituiertes Phenyl), wobei der Substituent ausgewählt ist unter bestimmten schwefelhaltigen Gruppen, nämlich unter Alkylthio, Alkylsulfinyl, Alkylsulfonyl, Sulfamoyl, N-Alkylsulfamoyl, N,N-Dialkylsulfamoyl, Alkylsulfonamido und Alkylsulfon-N-alkylamido. Bevorzugte Substituenten sind Alkylthio, Alkylsulfinyl, Alkylsulfonyl, Sulfamoyl und Alkylsulfonamido, insbesondere Methylthio, Methylsulfinyl, Methylsulfonyl, Sulfamoyl und Methylsulfonamido, ganz besonders Methylsulfonyl und Sulfamoyl.

Ein - vorteilhafterweise lipophiler - zweiter der Reste R1, R2, R3 kann linear oder verzweigt, alicyclisch oder heterocyclisch, nicht-aromatisch oder aromatisch sein. Bevorzugt sind einerseits Arylreste, insbesondere Phenylreste, andererseits aliphatische und cycloaliphatische, insbesondere Alkyl, Cycloalkyl- und Cycloalkylalkylreste. Diese Reste können den obigen Angaben entsprechend substituiert sein. Eine Substitution ist inbesondere für den ersten Fall, d.h. der Arylsubstitution, von Vorteil. Dementsprechend steht der zweite der Reste R1, R2, R3 insbesondere für einfach in Para- oder Meta-Stellung substituiertes Phenyl, insbesondere für 4-substituiertes Phenyl. Gegebenenfalls vorhandene Substituenten sind vorzugsweise ausgewählt unter Alkylgruppen, insbesondere Methyl, und vor allem elektronegative Gruppen, wie Halogenatomen, insbesondere Fluor, und Halogenalkylgruppen, insbesondere Trifluormethyl.

Ist der zweite der Reste R1, R2, R3 ein Alkylrest, so sind einerseits Alkylreste mit einer relativ großen Anzahl an Kohlenstoffatomen, insbesondere die o.g. C₄₋₆-Alkylreste, und von diesen vorteilhafterweise die verzweigten und insbesondere die einfach verzweigten, andererseits Alkylreste mit einer relativ geringen Anzahl von Kohlenstoffatomen, vor allem Methyl, bevorzugt.

Ist der zweite der Reste R1, R2, R3 ein Cycloalkylrest, so ist insbesondere Cycloheayl von Vorteil.

Ist der zweite der Reste R1, R2, R3 ein Cycloalkylalkylrest, so sind Cycloalkylmethylreste bevorzugt, wobei Cyclopropylmethyl von Vorteil ist.

Gemäß einer bevorzugten Ausführungsform ist der zweite der Reste R1, R2, R3 ein fluoraliphatischer, insbesondere ein poly- oder insbesondere perfluorierter aliphatischer Rest. Insbesondere sind hier polyund perfluorierte Alkylreste zu nennen, d.h. vor allem die zuvor beschriebenen Alkylreste, in denen 2 oder mehrere und insbesondere sämtliche H-Atome durch Fluoratome ersetzt sind. Dies gilt analog für Cycloalkyl- und Cycloalkylalkytreste.

Gemäß einer besonderen Ausführungsform der vorliegenden Erfindung steht ein dritter der Reste R1, R2, R3 für Wasserstoff, Alkyl, Halogenalkyl, Hydroxyalkyl, Halogen, Cyano, Alkylsulfonyl, Sulfamoyl oder B-Y, worin B für Alkylen oder Alkenylen, die gegebenenfalls durch Hydroxyl oder Alkoxy substituiert sein können, und Y für Hydroxy stehen; im Rahmen dieser Ausführungsform sind Wasserstoff, Alkyl, Halogenalkyl, insbesondere CF₃ oder Halogen bevorzugt. Steht der dritte der Reste für B-Y, so steht B vorzugsweise für Alkylen, das gegebenenfalls durch Hydroxy substituiert ist.

Ist der dritte Rest ein Alkylrest, so sind hier die relativ kurzen Reste bevorzugt, wobei insbesondere Methyl zu nennen ist. Bevorzugte Alkoxycarbonylreste sind Methoxy- und Ethoxycarbonyl. Von den Halogenatomen sind in diesem Fall Chlor und gegebenenfalls Brom bevorzugt.

Auch können ein zweiter und ein dritter der Reste R1, R2, R3, sofern sie vicinal zueinander stehen, zusammen mit den C-Atomen, an die sie gebunden sind, einen cycloaliphatischen Rest bilden. Sind R2 und R3 beteiligt, so handelt es sich bei diesem Ring für sich genommen um einen ungesättigten Ring, beispielsweise einen Cyclopentenyl- oder Cyclohexenyl-Ring, wie es im vorliegenden Beispiel 32 veranschaulicht ist. Sind R1 und R2 beteiligt, so weist der Ring zwei exocyclische Doppelbindungen auf.

Vorzugsweise stehen der erste und der zweite der Reste R1, R2, R3 vicinal zueinander, d.h. es sind R1 und R2 oder R2 und R3 beteiligt. Dies bringt insbesondere für die erfindungsgemäßen [α]-anellierten Pyrrolverbindungen mit Bisarylsubstitution Vorteile mit sich.

Grundsätzlich sind die Positionen der als erste, zweite und dritte Reste bezeichneten Gruppen variabel. Als vorteilhaft hat sich erwiesen, wenn der erste der Reste R1, R2, R3 an der Position von R1 oder R3 sitzt. Ebenfalls als vorteilhaft hat sich erwiesen, wenn der zweite der Reste R1, R2, R3 an der Position von R2 sitzt.

Verbindungen, in denen R3 für CHO, -COOH, -COCOOH, -COOAlkyl, -COOAlkPhenyl, -COCOOAlkyl oder B-Y steht, worin B für Alkylen oder Alkenylen, die gegebenenfalls durch Hydroxyl oder Alkoxy substituiert sein können, und Y für -COOH, -COO-Alkyl oder -CHO steht, können als Synthese-Zwischenprodukte Anwendung finden. Im Rahmen dieser besonderen Ausführungsform bevorzugte Reste B-Y sind 2-Essigsäure- und 3-Propionsäurereste.

Die erfindungsgemäßen Verbindungen können folgendermaßen hergestellt werden (in den Schemata haben die angezeigten Reste die zuvor genannten Bedeutungen, sofern nichts anderes angegeben ist; Angaben in Klammern dienen als erläuternde Beispiele). In den Reaktionsschemata hat Acyl die oben angegebenen Bedeutungen. R steht für einen Alkylrest sofern nicht anderes angegeben ist.

Die Synthese von Sulfoxiden und Sulfonen kann über die Reduktion von Sulfonsäurechloriden mit unedlen Metallen, wie Eisen und Zink, in sauren, wäßrigen Systemen zu geeigneten Thiolen geführt werden, die dann zunächst zu Schwefelethem alkyliert und anschließend mit Peroxyden schrittweise oxydiert werden.

Eine weitere Möglichkeit zur Synthese von Sulfonen geht über eine Reduktion von Sulfonsäurechloriden mit unedlen Metallen, wie Eisen und Zink, in neutralen, gepufferten, wäßrigen Systemen, oder mit Sulfiten, zu Sulfinaten, die anschließend zu Alkylsulfinaten alkyliert und schließlich thermisch zu den Sulfonen umgelagert werden.

Beispielsweise kann 6-(4-Fluorphenyl)-5-methyl-7-phenyl-2,3-dihydro-1H-pyrrolizin, das nach dem von Laufer et al. ( J. Med. Chem. 1994, 37, 1894-1897) beschriebenen Verfahren aus 2-Benzylpyrrolin und 2-Brom-1-(4-fluorphenyl)-ethanon hergestellt werden kann, in Chlorsulfonsäure sulfoniert werden und durch ein geeignetes Chlorierungsmittel, wie Phosphorpentachlorid, aber auch durch überschüssige Chlorsulfonsäure, bevorzugt in der Wärme, zum Sulfonsäurechlorid umgewandelt werden. Anschließend wird das Sulfonsäurechlorid in einer wäßrigen Lösung von Natriumsulfit und einer geeigneten Base, z.B. Natriumhydrogencarbonat bei erhöhter Temperatur, z.B. 70 °C, reduziert. Das in Lösung befindliche Anion der gebildeten sulfinigen Säure wird in z.B. ethanolisch-wäßrigem System mit einem Alkyljodid alkyliert und der primär entstandene Sulfinigsäure-Alkylester durch mehrstündiges Erhitzen (z.B.100 °C) in das gewünschte Alkylsulfon umgewandelt (Reaktion 1).

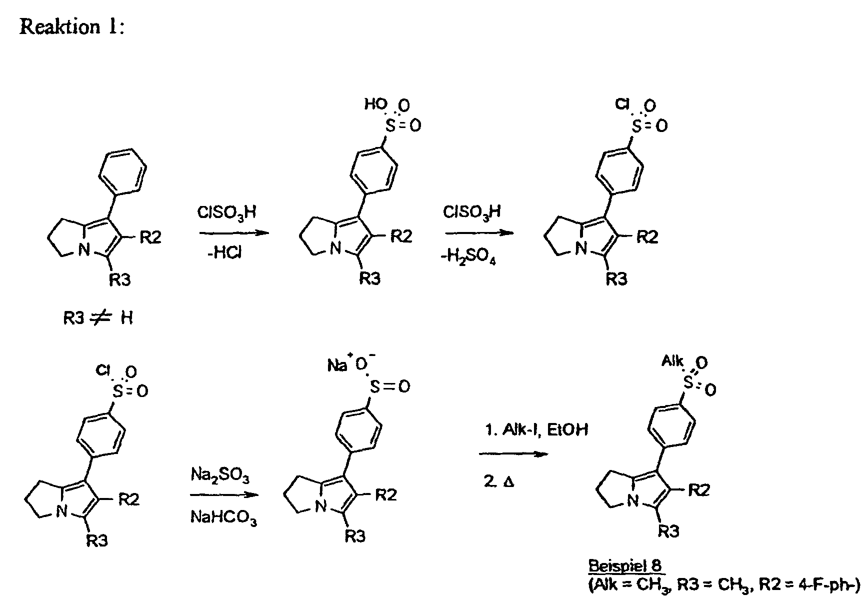

Erforderlichenfalls wird zunächst eine Schutzgruppe für die äußerst reaktive Position 5 der Ausgangsverbindung, z.B. 6-(4-Fluorphenyl)-7-phenyl-2,3-dihydro-1H-pyrrolizin eingeführt. Als Schutzgruppe für reaktive Positionen an Pyrrolen eignen sich Carbonsäureestergruppen, die über die Umsetzung dieser Pyrrole beispielsweise mit Phosgen, Diphosgen oder Triphosgen und die anschließende Alkoholyse der als Zwischenstoffe gebildeten Carbonsäurechloride nahezu quantitativ hergestellt werden können. Nach erfolgter Einführung der gewünschten Gruppe werden die Ester verseift und die isolierten Pyrrolcarbonsäuren thermisch zu den Pyrrolen decarboxyliert.

Vorteilhafterweise können die erforderlichen Schwefelfunktionen tragenden Aromaten bereits beim Aufbau des Pyrrolizingerüstes durch geeignete Vorstufen eingeführt werden, wodurch sich eine Verkürzung der Synthesefolge ergibt. Beispielsweise ist das zum Aufbau der Verbindung aus Beispiel 6 nach der geschilderten Technik erforderliche 4-Methylsulfanylbenzyl-2-pyrrolin (7) über die Grignardreaktion des 4-Methylsulfanylbenzylbromids mit Magnesium und die nachfolgende Addition an 4-Chlorbutyronitril (Reaktion 2) zugänglich.

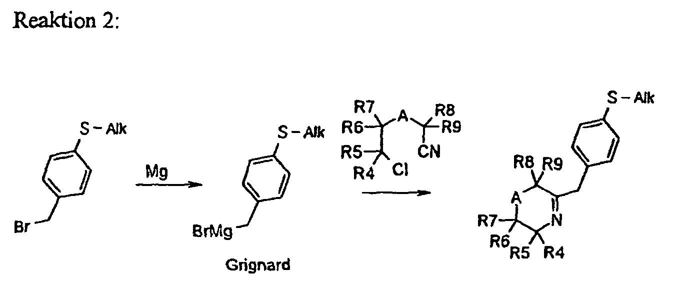

Vorzugsweise erfolgt die Darstellung des Zwischenproduktes 7 über die Kondensation eines 4-Alkylsulfanylphenylessigsäureesters mit N-Vinyl-2-pyrrolidon in Anwesenheit starker Basen, z.B. NaH, K-tert.Butylat, etc. und die nachfolgende hydrolytische Spaltung und Decarboxylierung des in hoher Ausbeute als Zwischenprodukt gebildeten 1-Vinyl-3-acyl-2-pyrrolidons (Reaktion 3). Beispielsweise liefert die Umsetzung des Zwischenproduktes 7 mit 2-Brom-1-(4-fluorphenyl)-ethanon das nachfolgend gezeigte Pyrrolizin (Beispiel 6).

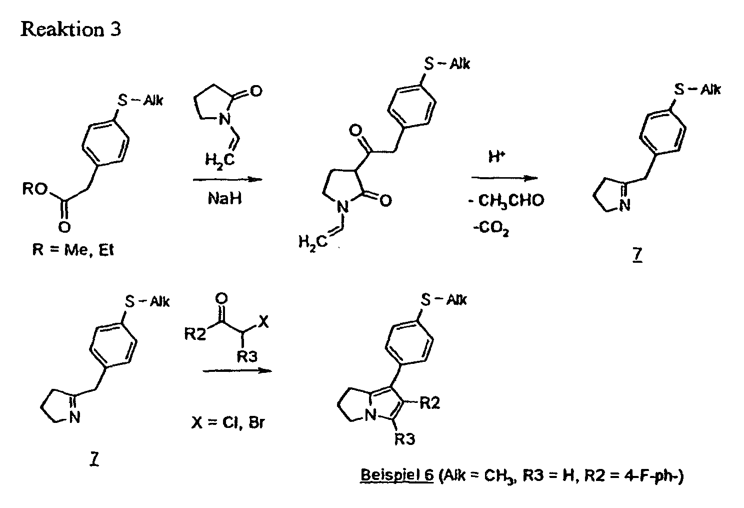

Die notwendigen Ausgangsverbindungen 4-Alkylmercapto-phenylessigsäureester, hier 4-Methylmercapto-phenylessigsäuremethylester, können ausgehend von Thioanisol via Friedel-Crafts-Acylierung mit Acetylchlorid und Willgerodt-Kindler-Umlagerung mit anschließender Hydrolyse und Esterbildung bequem hergestellt werden. In guten Ausbeuten können aus den 4-Alkylmercaptophenylessigsäureestern durch Persäuren-Oxidation, z.B. mit m-Chlorperbenzoesäure oder H₂O₂ in Eisessig, die 4-Alkylsulfonyl-phenylessigsäureester erhalten werden, die analog der Sulfanylverbindung zu einem Pyrrolin 9 kondensiert und zum Pyrrolizin des Beispiels 7 cyclisiert werden kann (Reaktion 4).

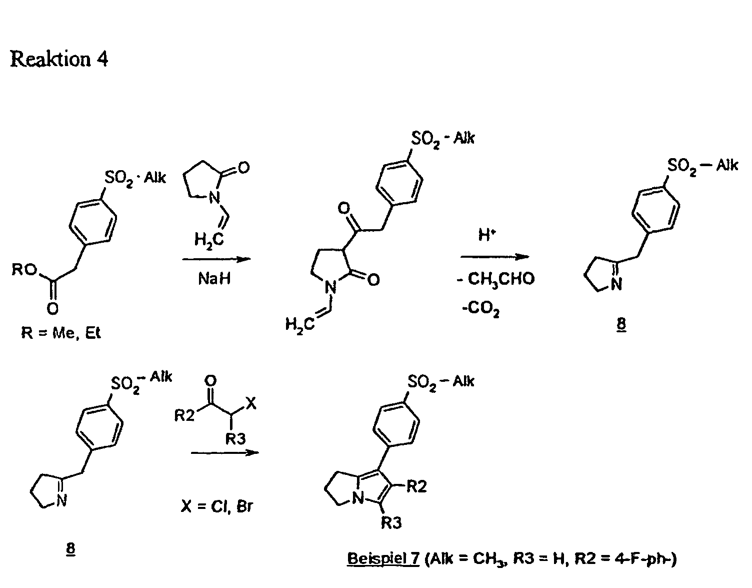

Erfindungsgemäße Diarylpyrrolizin-Verbindungen der 6,7- (bzw 1,2-) -Diarylreihe, bei denen die Schwefelfunktion in der para-Position am Aromaten der Position 6 (bzw 2) des Pyrrolizins positioniert ist, können sowohl im Fall der Sulfanyl-, Sulfoxid-, sowie der Sulfonyl-, als auch der Sulfamoyl-Verbindungen auch aus den entsprechenden Phenacylbromiden, die diese Schwefelfunktionen tragen, hergestellt werden (Reaktion 4b).

So liefert beispielsweise die Umsetzung von 2-Brom-1-(4-methylsulfanyl-phenyl)-1-ethanon mit 2-Benzyl-1-pyrrolin die Verbindung des Beispiels 1, mit 2-(4-Fluorbenzyl)-1-pyrrolin die des Beispiels 4, die Umsetzung von 2-Brom-1-(4-Methylsulfinyl-phenyl)-1-ethanon mit 2-Benzyl-1-pyrrolin die Verbindung des Beispiels 2, die Umsetzung von 2-Brom-1-(4-Methylsulfonyl-phenyl)-1-ethanon mit 2-Benzyl-1-pyrrolin die des Beispiels 3, mit 2-(4-Fluorbenzyl)-1-pyrrolin die Verbindung aus Beispiel 5.

Auch können in Position 5 des Pyrrolizins bei besagten 6,7- (bzw. 1,2) Diarylpyrrolizin-Verbindungen durch Acylierung in Position 5 (bzw 3) Acyl-Seitenketten eingeführt und diese durch geeignete Reduktionsmethoden (HJ-Reduktion, NaCNBH₃ -Reduktion, Vitride-Reduktion etc. ) in Alkylseitenketten umgewandelt werden.

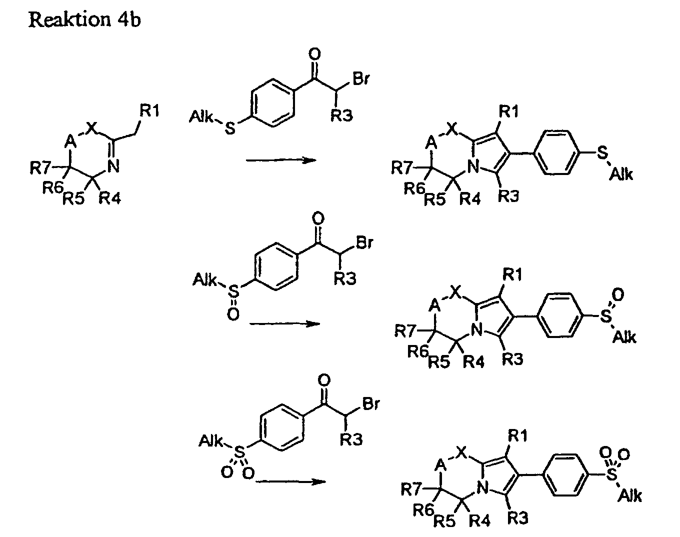

Erfindungsgemäße 5,6- (bzw 2,3) Diarylpyrrolizin-Verbindungen und 6,7- (bzw 2,3) Diarylindolizin-Verbindungen können auf dem Weg einer 1,3-dipolaren Cycloaddition aus entsprechenden Münchnon- bzw. Sydnon-Vorläuferverbindungen und geeigneten Dienophilen oder Dipolarophilen hergestellt werden. Dabei kann eine Schwefelfunktion einerseits über die Münchnon-/Sydnon-Komponente, andererseits über die Dipolarophilkomponente eingeführt werden. Als Dipolarophil finden Dehydrozimtsäuren oder andere Acetylencarbonsäuren, 2-halogensubstituierte Zimtsäuren oder 2-Halogenacrylate und Nitrostyrole Verwendung. In der Reihe der Pyrrolizine werden zur Sydnonbildung N-Acylderivate des Prolins (Pyrrolidin-2-carbonsäure) und in der Reihe der Indolizine N-Acylderivate der homologen Piperidin-2-carbonsäure, eingesetzt.

Beispielsweise führt die Cycloaddition des 2-Brom-3-(4-methylsulfanylphenyl)-propensäureethylesters (10) an das in situ erzeugte Münchnon des N-(4-Fluorbenzoyl)-prolin (11, Reaktion 5) zum Ethylester der Pyrrolizincarbonsäure des Beispiels 11, die des 2-Brom-3-(4-methylsulfonylphenyl)-propensäureethylesters (12) zum Ester aus Beispiel 12, und die Cycloaddition eines 2-(4-Methylsulfonylphenyl)-1-nitro-ethens (13) an dasselbe Münchnon führt zur Pyrrolizin-Verbindung aus Beispiel 13A.

Durch den Einsatz von beispielsweise Dehydrozimtsäuremethylester (14), 1-Nitrosiyrol bzw. N-Benzoyl-Prolin (15) gelangt man zunächst zu unsubstituierten 5,6- (bzw 2,3) Diarylpyrrolizin-Verbindungen (Reaktion 6), in die beispielsweise durch Chlorsulfonierung nachträglich eine Schwefelfunktion in die Para-Ringposition eingeführt werden kann.

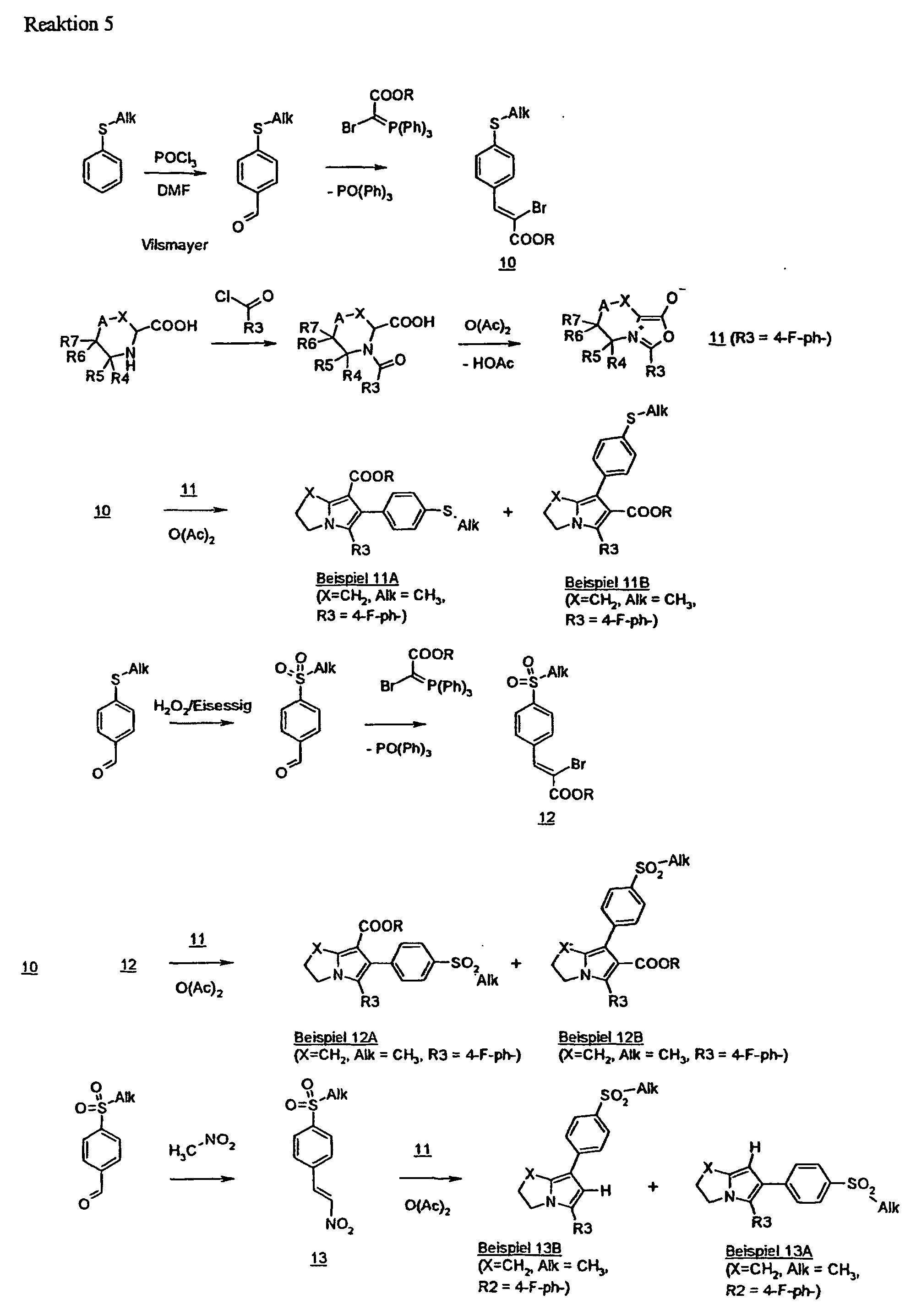

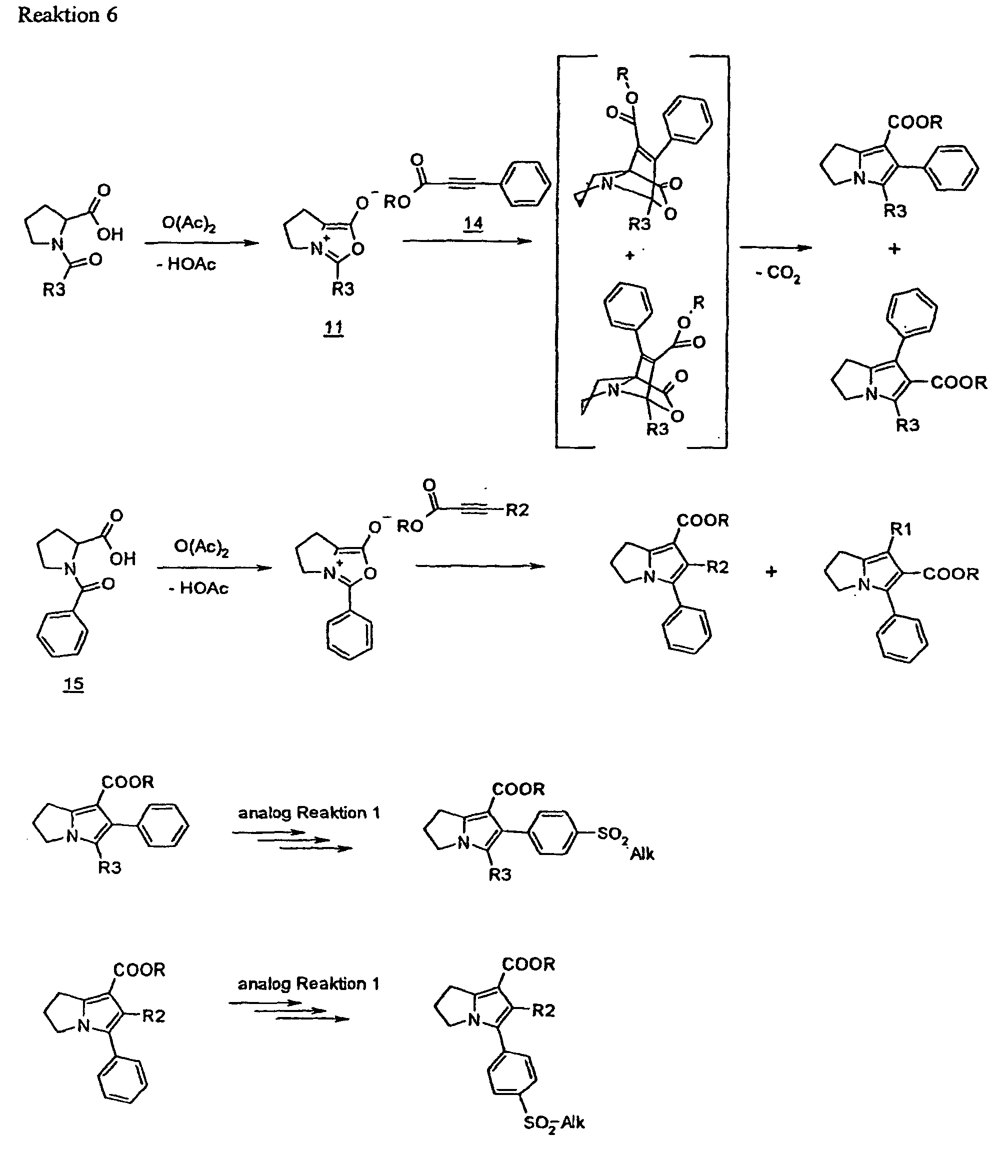

Dies erfolgt in Analogie zu den 6-Aryl und 6,7-Diarylverbindungen nach den oben (s.o. Reaktion 1, Beispiel 7) aufgeführten Techniken: die Umsetzung mit Chlorsulfonsäure im Überschuß führt zu den Sulfonsäurechloriden, die einerseits durch Na₂SO₃-Reduktion und Alkylierung mit anschließender Umlagerung in die Sulfone umgewandelt werden können (Reaktion 7) und andererseits in wäßrigen und nichtwäßrigen Lösungen von Ammoniak in die erfindungsgemäßen Sulfonamide umgewandelt werden können. Beispielsweise führt die Reaktion des 3-(4-Fluorphenyl)-2-phenyl-6,7-dihydro-5H-pyrrolizin-1-yl-carbonsäuremethylesters mit Chlorsulfonsäure im Überschuß, sowohl in der Kälte als auch in der Wärme, unter gleichzeitigem Verlust der Carbonsäureesterfunktion zum 4-[3-(4-Fluorphenyl)-6,7-dihydro-5H-pyrrolizin-2-yl]-benzol-sulfonsäurechlorid, das anschließend mit Ammoniak in das Sulfonamid des Beispiels 10 umgewandelt werden kann.

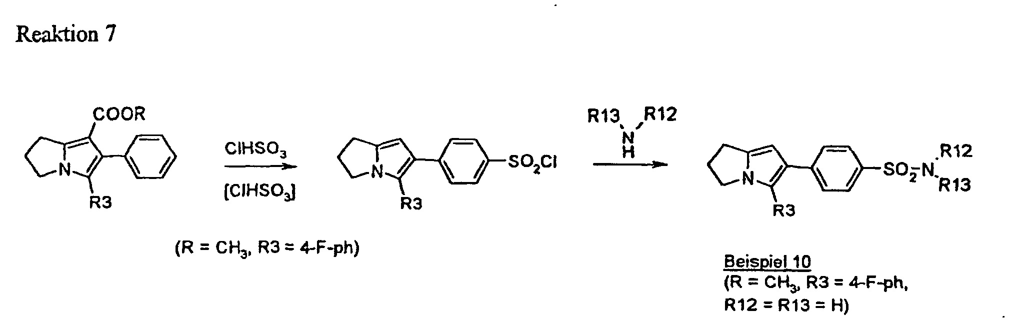

Eine geeignete Alternative für einen Zugang zu 5,6-diarylsubstituierten Pyrrolizinen bietet die Arylierung nach Suzuki vor allem dann, wenn der schwefelhaltige Aromat an Position-5 eingeführt wird.

Beispielsweise liefert die Umsetzung eines 6-(4-Fluorphenyl)-7-methyl-2,3-dihydro-1H-pyrrolizins, das aus 2-Ethyl-1-pyrrolin und 2-Brom-1-(4-fluorphenyl)-1-ethanon erhalten wurde, mit N-Bromsuccinimid in THF bei niedrigen Temperaturen (-70 °C bis 25 °C) ein 5-Brom-6-(4-fluorphenyl)-7-methyl-2,3-dihydro-1H-pyrrolizin, das in Anwesenheit von Tetrakis-triphenylphosphin-Palladium-(0) in wässrigalkalischem (z.B. Na₂CO₃/CH₂Cl₂) Milieu mit 4-Methylsulfanyl-phenylboronsäure nach Suzuki zur Methylsulfanyl-Verbindung aus Beispiel 9 kondensiert werden kann (Reaktion 8).

Als Ausgangspunkt für die 6-Alkyl-7-(4-sulfamoyl-phenyl) substituierten Pyrrolizine eignet sich auch das 7-Phenyl-2,3-dihydro-pyrrolizin, das durch NBS in THF in Position 5 halogeniert oder durch Umsetzung mit acylierenden Verbindungen wie Säurechloriden und Säureanhydriden in Position 5 acyliert werden kann (Reaktion 9).

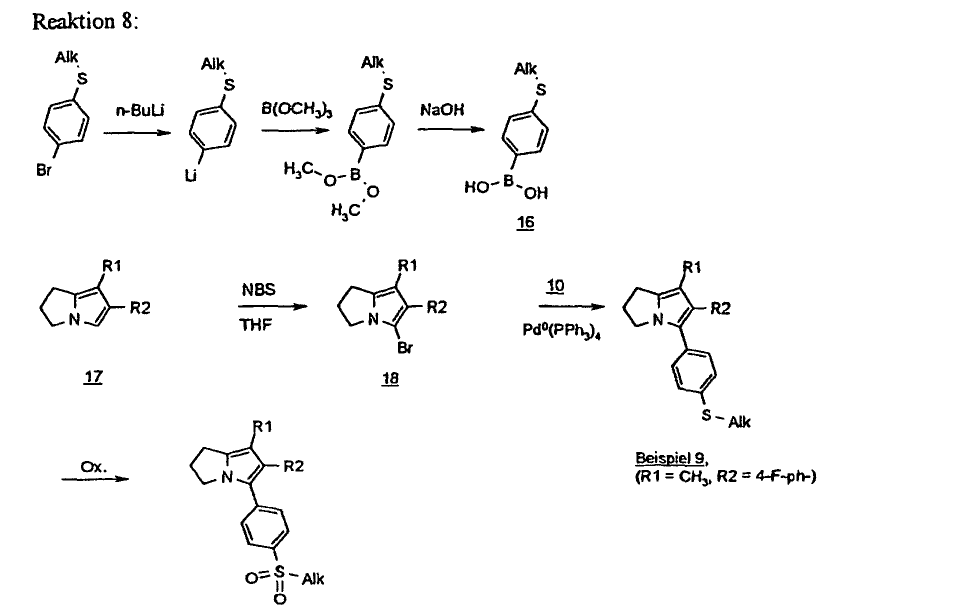

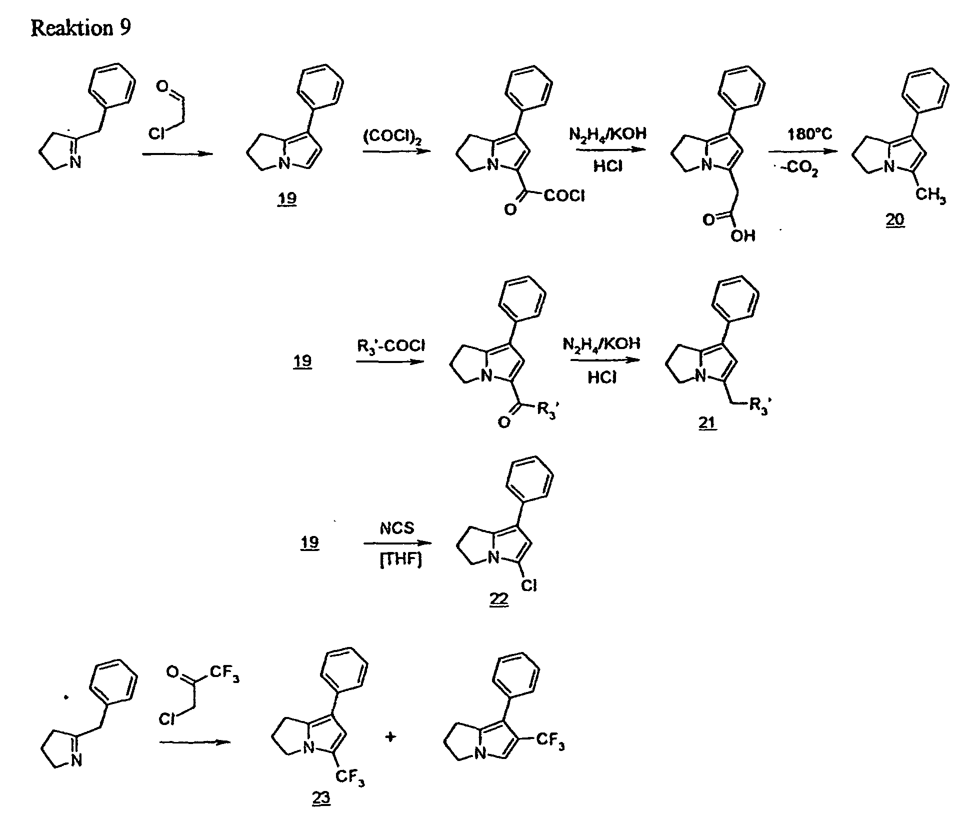

Anschließend kann, nach vorhergehender Modifikation dieser eingefügten Acylfunktion (vgl. Reaktion 9, 20, 21, Reduktion, Kondensation etc.), die verbleibende freie Pyrrolposition (Position 6) des Pyrrolizingerüstes erneut acyliert werden (Reaktion 10). In der Regel wird vor Einführung der Sulfochloridfunktion die Acylgruppe in eine Alkylgruppe überführt. Zu dieser Umwandlung eignet sich die Wolff-Kishner- oder Huang-Minion-Reaktion, oder die Umsetzung mit Natrium-cyanoborbydrid in Anwesenheit von Zinkjodid. Die erhaltenen Sulfochloride werden einerseits durch Ammoniak in die Sulfonamide oder durch Umsetzung mit Natriumsulfit und Methyljodid in die Sulfone umgewandelt (s.o. Reaktion 1).

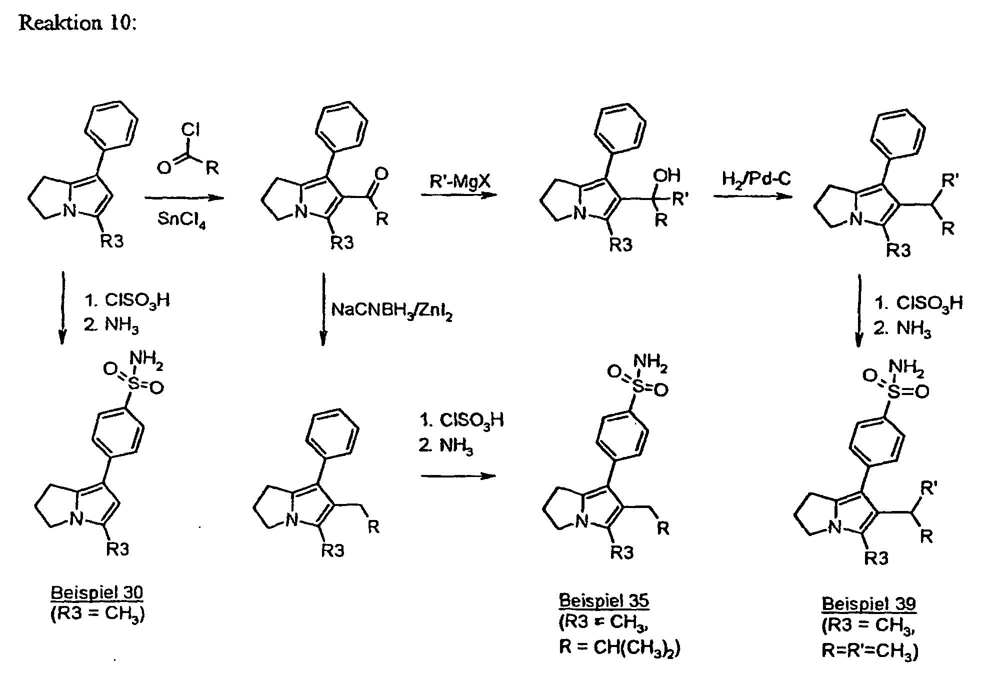

Als Ausgangspunkt für die Synthese von cycloaliphatisch substituierten Pyrrolizinen, z. B. des 6-Cyclohexyl-Derivates, können die acylierten Cycloaliphaten (z.B. Cyclohexyl-methyl-keton) verwendet werden. Ihre Bromierung führt auch zu den erwünschten Bromacetyl-Verbindungen. Vorteilhafter ist die Umsetzung des 1-Acetyl-1-ethoxycarbonyl-cycloalkyls, z.B. des 1-Acetyl-1-ethoxycarbonyl-cyclohexans mit Brom zu 1-Bromacetyl-1-ethoxycarbonyl-cyclohexan, das auch mit Pyrrolinen glatt zu einem Pyrrolizin cyclisiert. Vergleichbar der 1-Phenyl-6,7-dihydro-5H-pyrrolizin-3-yl-essigsäure in Reaktion 9 (Herstellung der Verbindung 20) ist auch die 6-yl-essigsäure instabil und decarboxyliert bei erhöhter Temperatur (Reaktion 11, 24-COOH).

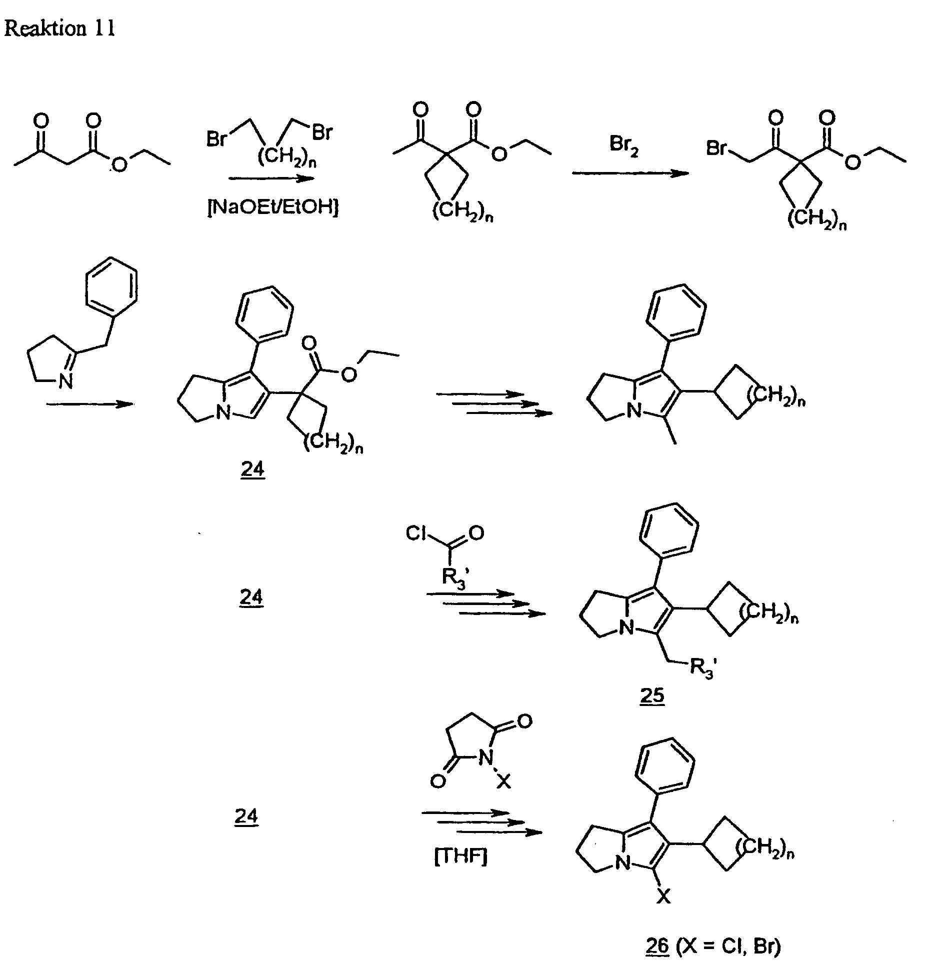

Die Ethoxycarbonylgruppe fungiert in diesem Fall als Schutzgruppe für die Verzweigungsposition im Cycloaliphaten. Schließlich werden auch hier mit Chlorsulfonsäure die Sulfochloride erhalten, die einerseits durch Ammoniak in die Sulfonamide (vgl. Beispiel 14) oder durch Umsetzung mit Natriumsulfit und Methyljodid in die Sulfone umgewandelt werden (Reaktion 12).

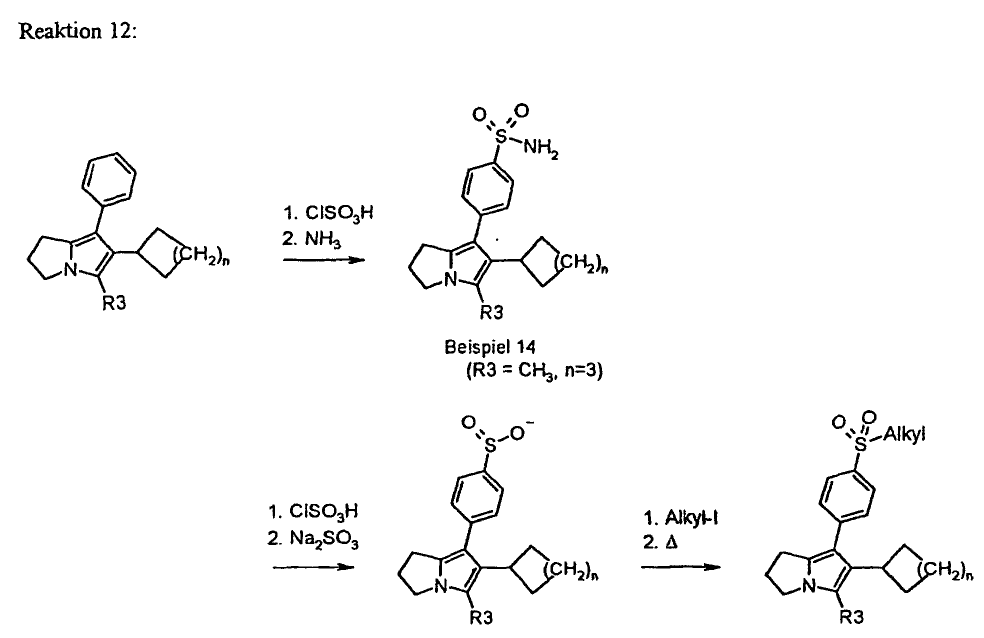

Die intermediär gewonnenen Pyrrolizine, sowie die Schwefelfunktionen tragenden Pyrrolizine können in verschiedener Weise weiter umgewandelt werden, zum Beispiel lassen sich in die aktivierten Positionen des Pyrrolizinkernes weitere funktionelle Gruppen einführen, wie dies in WO 95/23970 und WO 95/23971 ausgeführt wird. Die dem Pyrrolkern benachbarten α-Positionen des aliphatischen Anellanden (Position 1, bzw 7) können durch geeignete Methoden funktionalisiert werden: radikalische Bromierung mit N-Bromsuccinimid liefert in aprotischen Lösungsmitteln 1-Brom- (bzw. 7-Brom)-Derivate, in wässrigen Systemen und im Überschuß des Reagenzes die 1-Oxo-Derivate der Pyrrolizine.

Bei Verwendung von 1 Moläquivalent N-Bromsuccinimid erhält man die in 1-Position bromierte Verbindung der Formel 25 (Reaktion 13). Diese wiederum kann dann mit Acylaten, beispielsweise Natriumacetat oder Alkoholaten, wie Natriummethylat oder Natriumethylat, zu den entsprechenden Verbindungen der Formeln 26 und 27 (Reaktion 13) umgesetzt werden. Die Umsetzungen werden in bekannter Weise durchgeführt, die Umsetzung mit den Acylaten erfolgt in einem inerten Lösungsmittel, beispielsweise Dimethylformamid (DMF), bei erhöhter Temperatur, z.B. 70 bis 90 °C. Die Umsetzung der Verbindungen der Formel 25 mit Alkoholaten erfolgt zweckmäßigerweise in dem entsprechenden Alkohol. Alternativ können die Verbindungen der Formel 25 (Reaktion 13), gewünschtenfalls ohne aus der Reaktionsmischung isoliert zu werden, direkt mit einem Alkohol zu den Verbindungen der Formel 27 umgesetzt werden.

Alternativ kann man eine Verbindung der Formel IIa mit einem Moläquivalent NBS in einem chlorierten Lösungsmittel in Anwesenheit von Wasser zu einer Verbindung der Formel 28 umsetzen. Mit einem weiteren Moläquivalent NBS erhält man dann eine Verbindung der Formel 29.

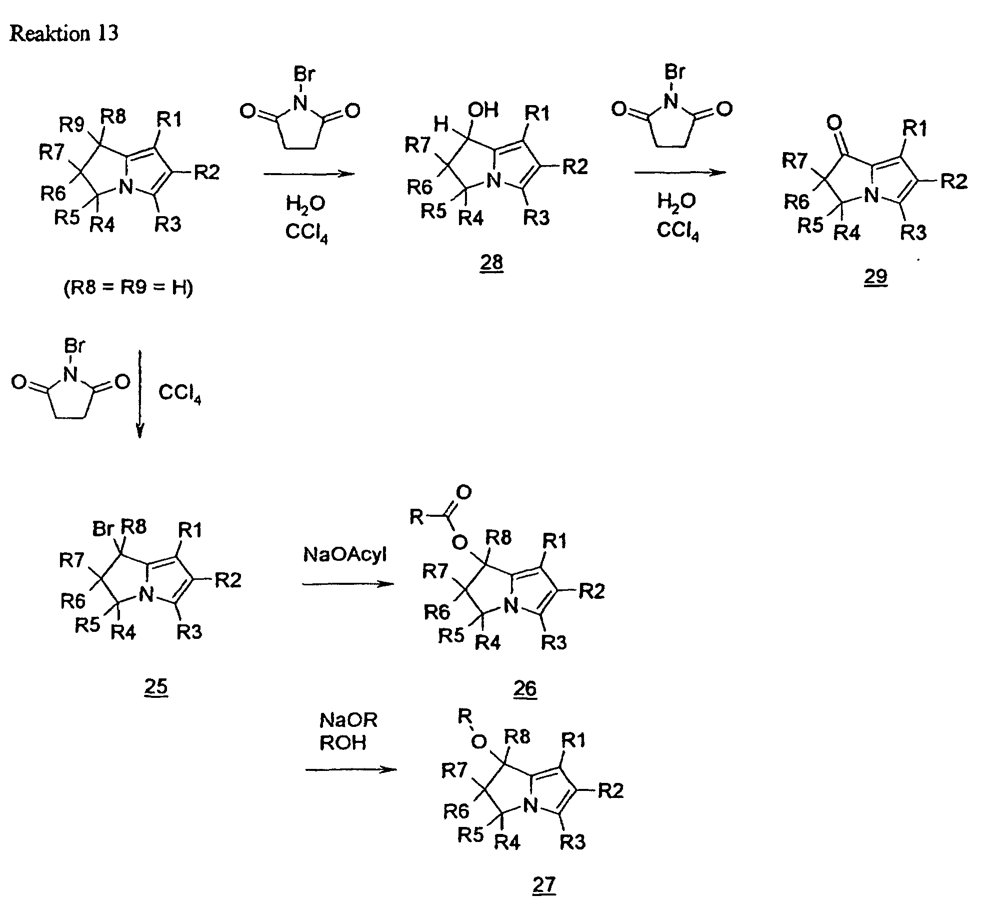

Die Einführung von polaren Gruppen am Kohlenwasserstoff-Gerüst des Anellanden, d.h. in die Position der Substituenten R4-R11, wie z.B. Hydroxylgruppen (Reaktion 14, 30), Aminogruppen (Reaktion 14, 31, Z = NR₂), Carbonyl- (Reaktion 14, 32) und Carboxylgruppen (Reaktion 14, 33) setzt eine abgewandelte Synthesestrategie voraus. Solche funktionellen Gruppen lassen sich am Kohlenwasserstoff-Gerüst des Anellanden nur indirekt nachträglich einführen. Die Herstellung gelingt über geeignete Vorstufen, die diese funktionellen Gruppen, geschützt durch sog. Schutzgruppen (=Pg protective groups), bereits enthalten oder in die gewünschte Gruppe aus einer geeigneten Vorläufergruppe (z.B. 30) nachträglich umgewandelt werden können.

Reaktion 14 zeigt ein Beispiel, wie, in Anwendung der in EP 0397175 beschriebenen Methodik, hier ausgehend von Tris-Hydroxymethylen-Derivaten (R4 - R6=H, R8 = R9 = H, R7=CH₂OH) durch selektive Einführung einer Schutzgruppe für eine der 3 Hydroxymethylengruppen, Verbindungen der Struktur 30, 31, 32 und 33 erhalten werden können, die das entsprechende Substitutionsmuster mit funktionellen Gruppen enthalten. Diese Verbindungen (30, 31, 32 und 33) treten in Form zweier optischer Antipoden auf, die bei Bedarf durch Anwendung chromatographischer Techniken getrennt werden oder auf der Stufe diastereomerer Syntheseintermediate (Acetate oder Ketale mit R=H, R'=Alkyl, Aryl etc, oder R=R'= Alkyl, Aryl etc.) nach deren Herstellung mit üblichen Reinigungsmethoden getrennt werden und die nach Rückspaltung die reinen Enantiomere der Vorstufen liefern.

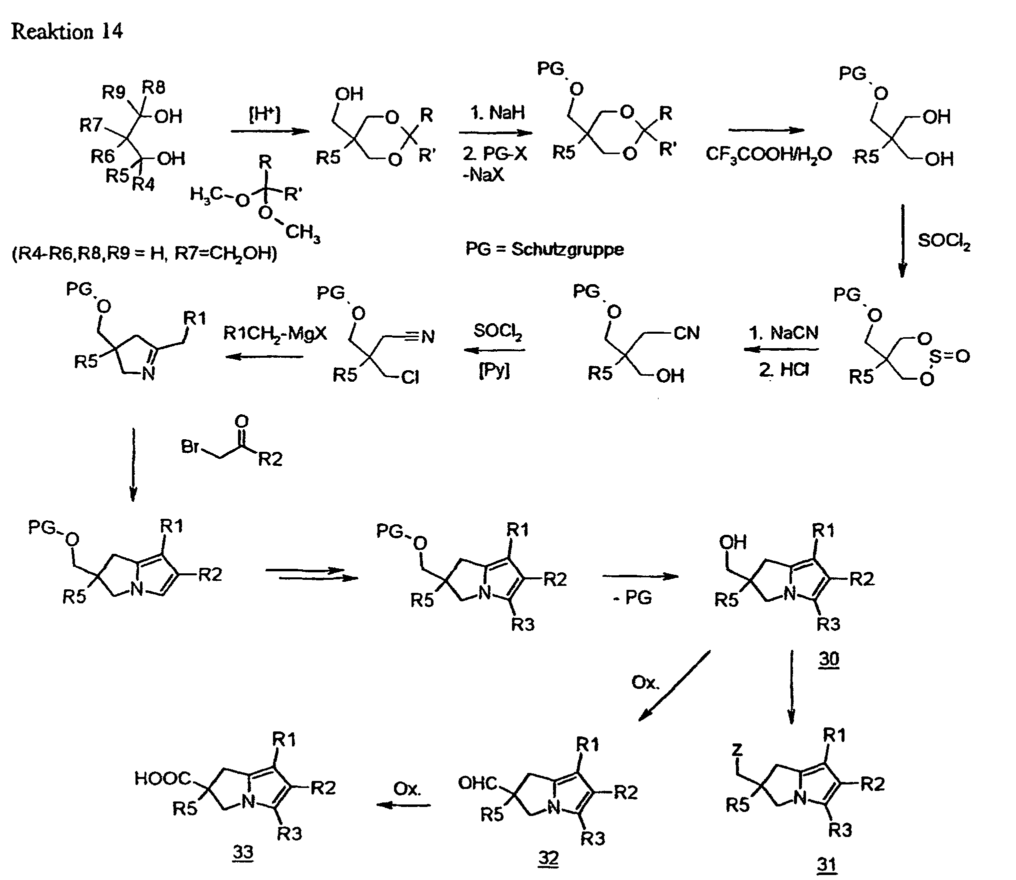

Die erfindungsgemäßen Verbindungen zeigen in vitro eine ausgeprägte Hemmung auf die Freisetzung proinflammatorischer Mediatoren der Arachidonsäurekaskade und damit in vivo antipyretische, analgetische und antientzündliche (antiinflammatorische) Wirkung in Enzündungsmodellen. Die erfindungsgemäßen Verbindungen hemmen die Prostaglandin-H-synthase, das Schlüsselenzym der Prostaglandinsynthese. Dabei zeigt dessen Isoform-2 eine erhöhte Empfindlichkeit gegenüber den erfindungsgemäßen Verbindungen, so daß diese als selektive Inhibitoren des Isoenzyms-2 wirken. Damit lassen die Verbindungen ein deutlich erniedrigtes Nebenwirkungsprofil, insbesondere an Niere und GI-Trakt, erwarten.

Schwache bis mittlere Hemmwirkung üben die Verbindungen auch auf die 5-Lipoxygenase, ein weiteres Enzym der Arachidonsäuremetabolisierung, aus. Mit der Hemmwirkung auf 5-LO lassen sich ähnlich wie bei dualen Inhibitoren der Cyclooxygenase-1/2 (COX-1/COX-2) und der 5-Lipoxygenase (5-LOX) die nachteiligen Effekte einer reinen COX-1 Inhibition auf Bronchien (Asthma) und GI-Trakt (Läsionen) kompensieren.

Die erfindungsgemäßen Verbindungen eignen sich somit zur Behandlung von Erkrankungen, bei denen erhöhte Freisetzungsraten der Eicosanoid-Mediatoren für die Entstehung bzw. den Progredientenverlauf dieser Erkrankungen verantwortlich sind. Insbesondere sind die erfindungsgemäßen Verbindungen zur Behandlung von Erkrankungen des rheumatischen Formenkreises und zur Prävention von allergisch induzierten Erkrankungen brauchbar. Die erfindungsgemäßen Verbindungen stellen somit wirksame Antiphlogistika, Analgetika, Antipyretika, Antiallergika und Broncholytika dar oder sind antibronchokonstriktorisch wirksam und daher zur Thromboseprophylaae und zur Prophylaxe des anaphylaktischen und septischen Schocks sowie zur Behandlung dermatologischer Erkrankungen, wie Psoriasis, Urtikaria, akuter und chronischer Exantheme, allergischer und nicht-allergischer Genese brauchbar.

Die erfindungsgemäßen Verbindungen besitzen erhöhte chemische Stabilität, parenterale Anwendbarkeit, enterale Bioverfügbarkeit und kurze Halbwertszeiten.

Die erfindungsgemäßen Verbindungen können entweder als einzelne therapeutische Wirkstoffe oder als Mischungen mit anderen therapeutischen Wirkstoffen verabreicht werden: sie können als solche verabreicht werden, im Allgemeinen werden sie jedoch in Form pharmazeutischer Mittel verabreicht, d.h. als Mischungen der Wirkstoffe mit pharmazeutisch akzeptablen Hilfsstoffen, insbesondere Trägerstoffen bzw. Verdünnungsmitteln und/oder Zusatzstoffen. Die Verbindungen oder Mittel können enteral, z.B. oral oder rektal, oder parenteral, z.B. subkutan, intravenös oder intramuskulär verabreicht werden, vorzugsweise werden sie jedoch in oralen Dosierungsformen gegeben.

Die Art des pharmazeutischen Mittels und des pharmazeutischen Trägers bzw. Verdünnungsmittels hängt von der gewünschten Verabreichungsart ab. Orale Mittel können beispielsweise als Tabletten oder Kapseln vorliegen und können übliche Exzipienzien enthalten, wie Bindemittel (z.B. Sirup, Akazia, Gelatine, Sorbit, Tragant oder Polyvinylpyrrolidon), Füllstoffe (z. B. Lactose, Zucker, Maisstärke, Calciumphosphat, Sorbit oder Glycin), Gleitmittel (z.B. Magnesiumstearat, Talcum, Polyethylenglykol oder Siliciumdioxid), disintegrierende Mittel (z.B. Stärke) oder Netzmittel (z.B. Natriumlaurylsulfat). Orale flüssige Präparate können in Form wässriger oder öliger Suspensionen, Lösungen,. Emulsionen, Sirupen, Elixieren oder Sprays usw. vorliegen oder können als Trockenpulver zur Rekonstitution mit Wasser oder einem anderen geeigneten Träger vorliegen. Derartige flüssige Präparate können übliche Additive, beispielsweise Suspendiermittel, Geschmacksstoffe, Verdünnungsmittel oder Emulgatoren, enthalten. Für die parenterale Verabreichung kann man Lösungen oder Suspensionen mit üblichen pharmazeutischen Trägern einsetzen.

Die Verwendung erfindungsgemäßer Verbindungen beinhaltet im Rahmen der Behandlung ein Verfahren. Dabei wird dem zu behandelnden Individuum, vorzugsweise einem Säuger, insbesondere einem Menschen, Nutz- oder Haustier, eine wirksame Menge einer oder mehrerer Verbindungen, in der Regel der pharmazeutischen und tierarzneilichen Praxis entsprechend formuliert, verabreicht. Ob eine solche Behandlung angezeigt ist und in welcher Form sie zu erfolgen hat, hängt vom Einzelfall ab und unterliegt einer medizinischen Beurteilung (Diagnose), die vorhandene Anzeichen, Symptome und/oder Fehlfunktionen, Risiken, bestimmte Anzeichen, Symptome und/oder Fehlfunktionen zu entwickeln, und weitere Faktoren miteinbezieht.

Die Behandlung erfolgt in der Regel durch einmalige oder mehrmalige tägliche Verabfolgung gegebenenfalls zusammen oder im Wechsel mit anderen Wirkstoffen oder wirkstoffhaltigen Präparaten, so daß einem zu behandelnden Individuum eine Tagesdosis von etwa 0,1 mg bis etwa 1000 mg und insbesondere 0,5 mg bis etwa 100 mg pro kg Körpergewicht zugeführt wird.

Die nachfolgenden Beispiele erläutern die Erfindung ohne sie zu begrenzen.

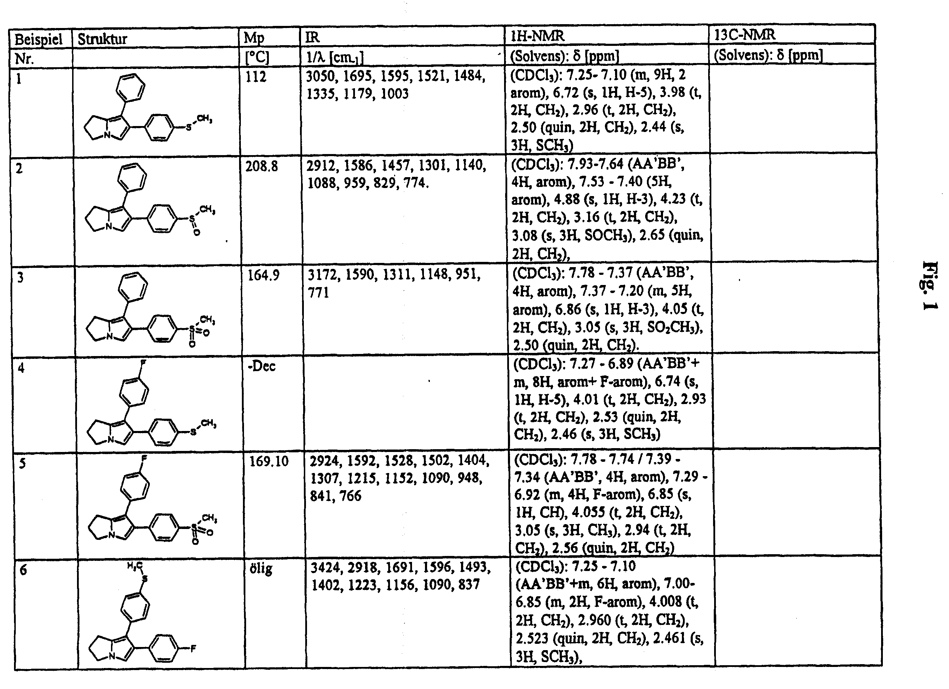

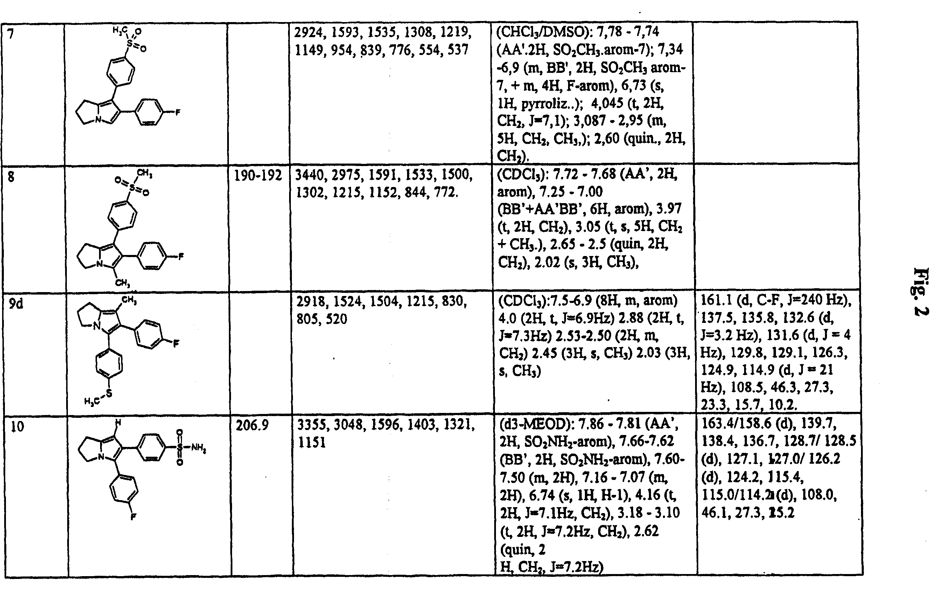

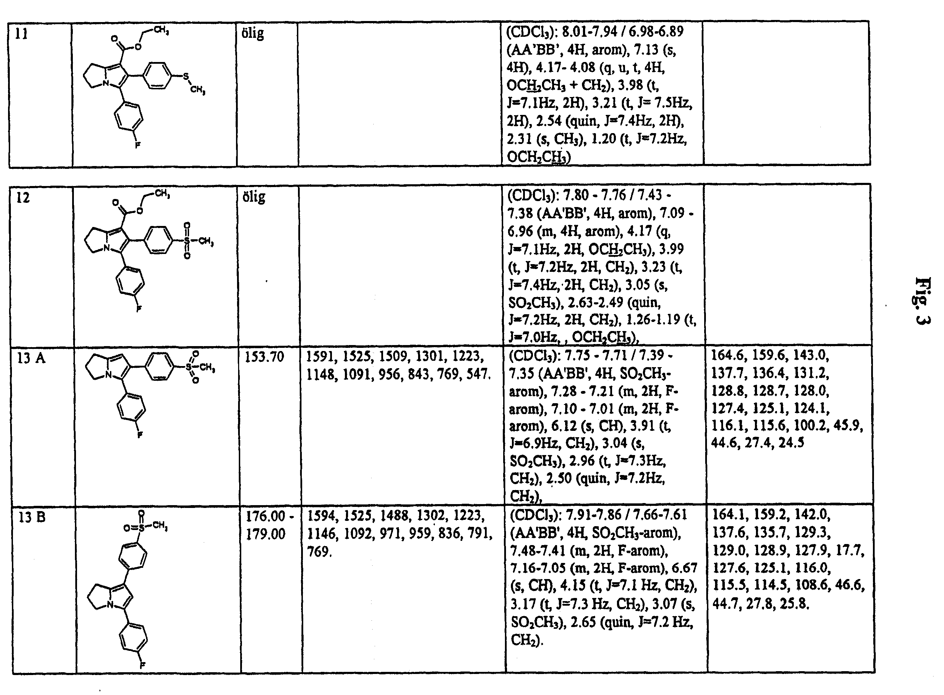

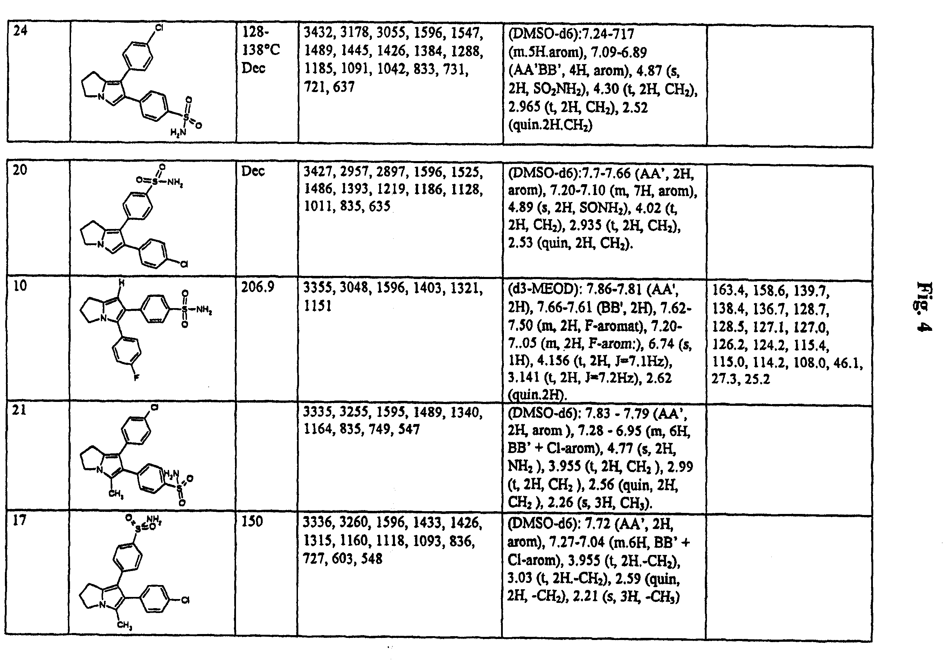

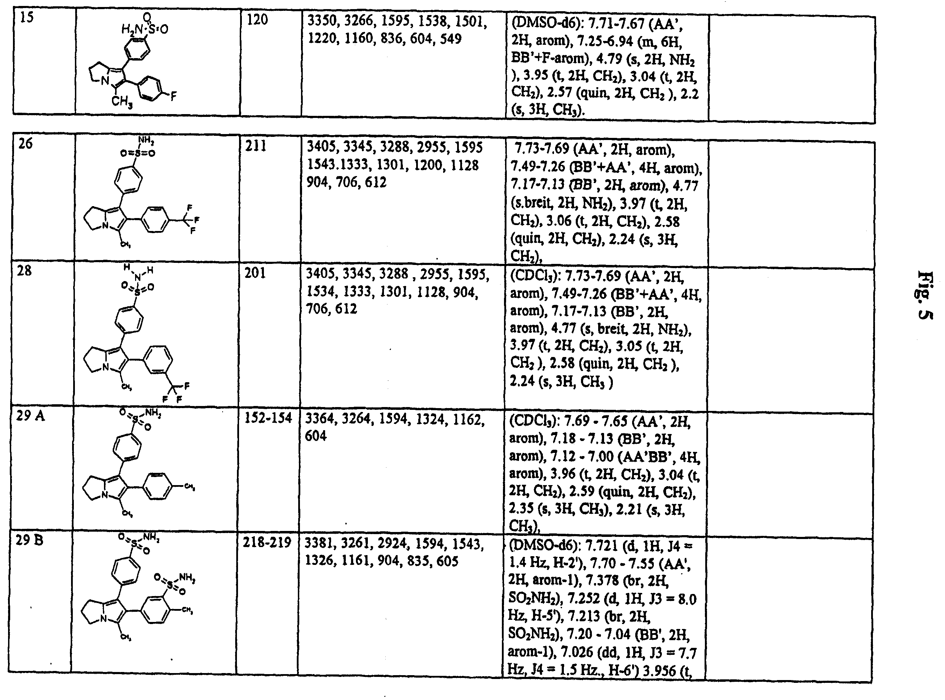

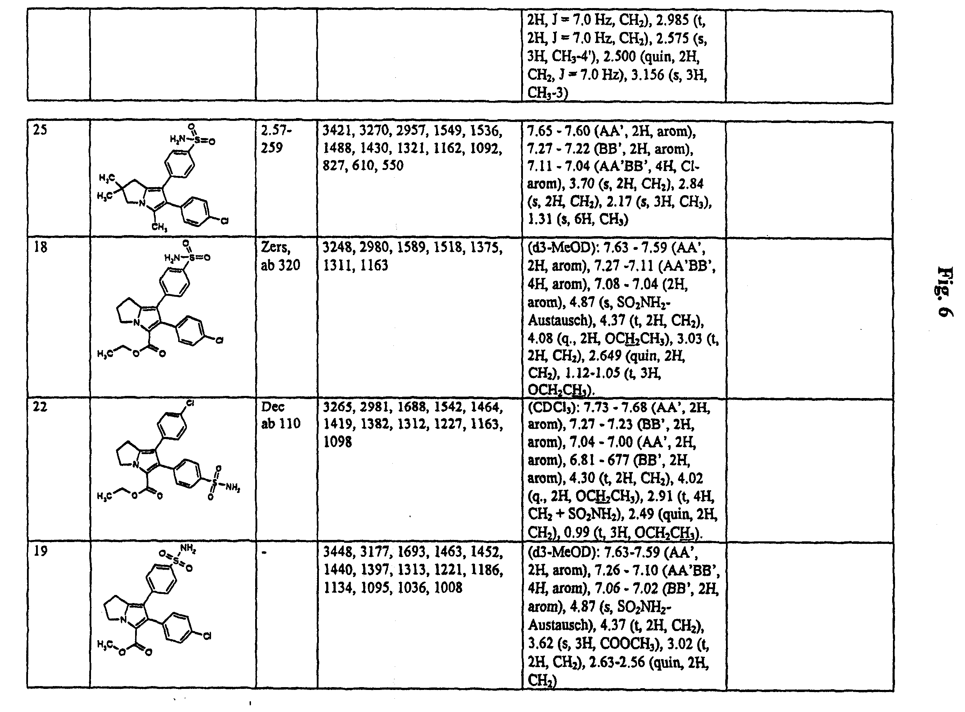

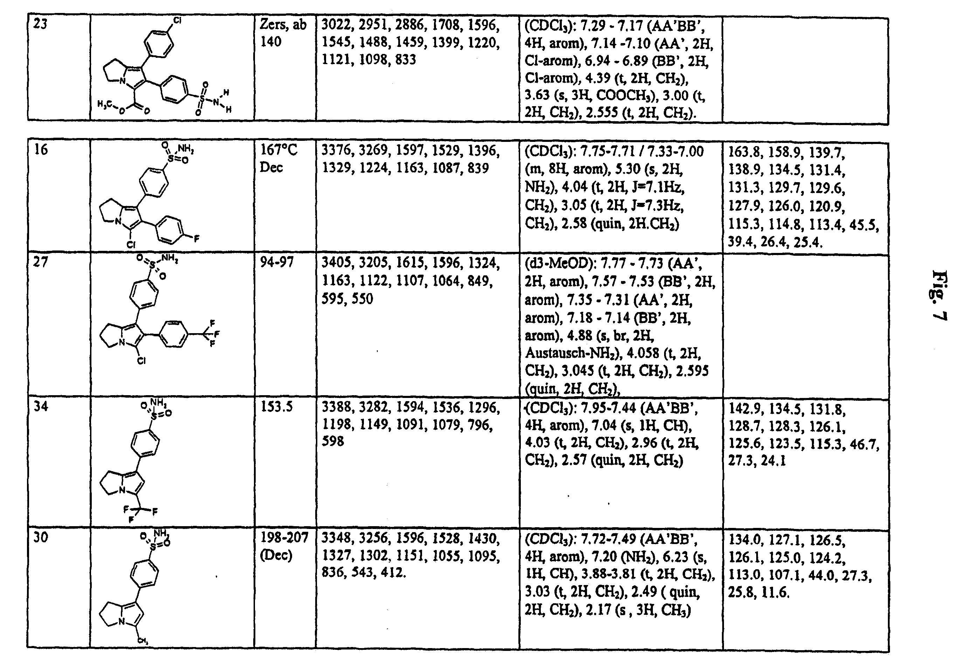

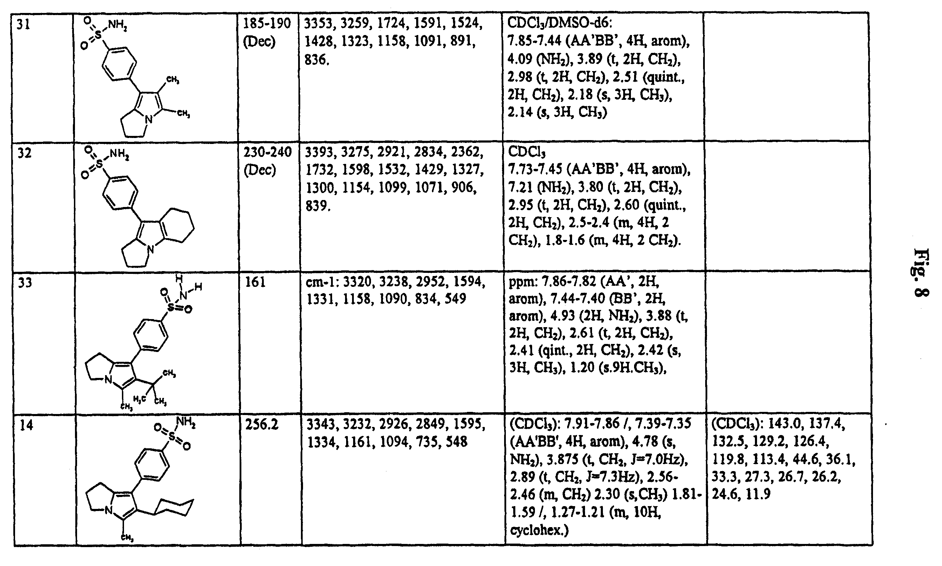

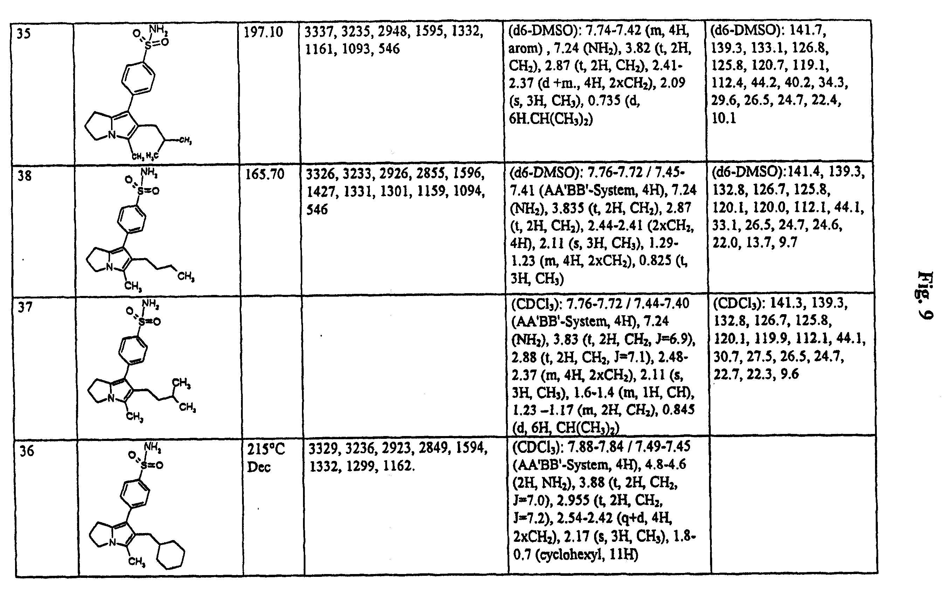

Es zeigen
die Figuren 1 bis 9 spektroskopische Daten der gemäß den Beispielen 1 bis 36 hergestellten Verbindungen;

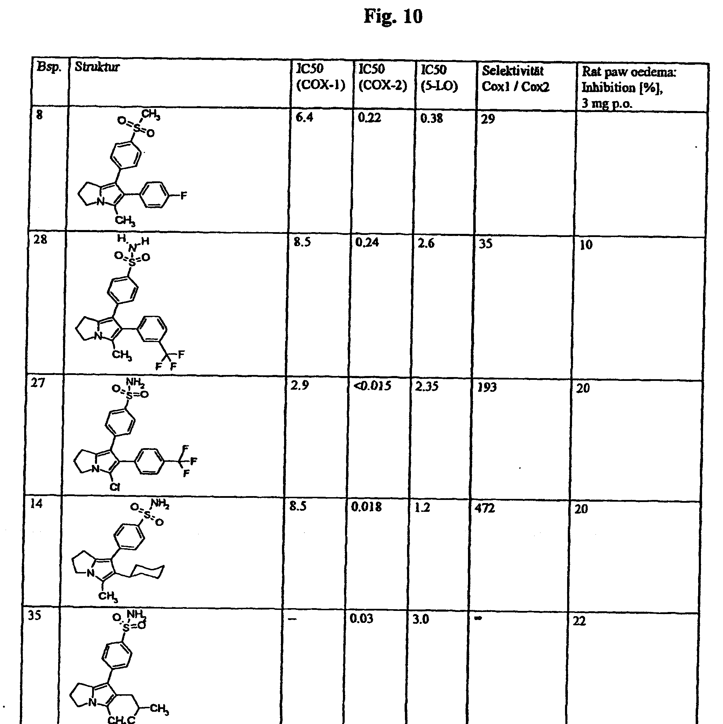

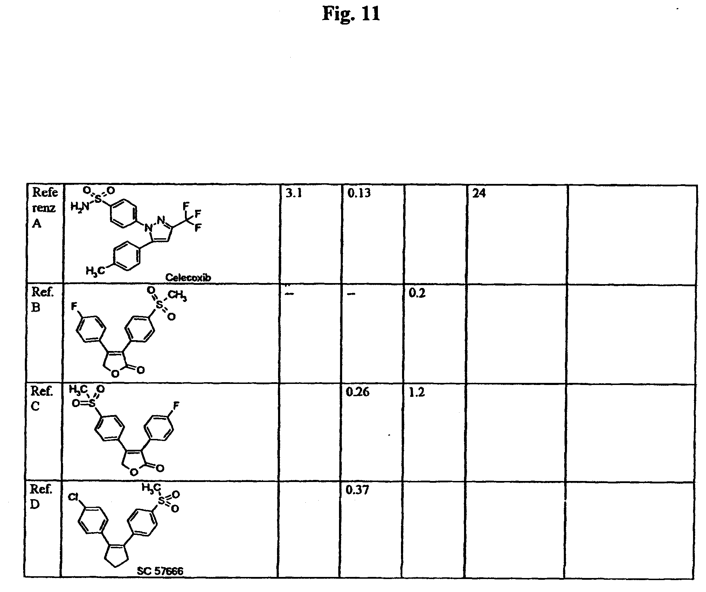

die Figuren 10 und 11 biologische Aktivitätsdaten von einigen erfindungsgemäßen Verbindungen sowie von bekannten Verbindungen zum Vergleich.

### Synthese

Es bedeuten im allgemeinen
- Mp:: Schmelzpunkt (Schmp.)
- Bp:: Siedepunkt
- Dec:: Zersetzung
- DC:: Dünnschichtchromatographie(isch)
- SC:: Säulenchromatographie(isch)
- GC:: Gaschromatographie(isch)
- Y:: Ausbeute
- TY:: Gesamtausbeute
- abs.: absolut

Im Rahmen der NMR bedeuten
- Arom:: aromatisch;
- quin:: Quintuplett;
- br:: breit;
- t:: Triplett;
- m:: Multiplett;
- s:: Singulett;
- q:: Quadruplett;
- d:: Duplett

Die Schmelzpunkte wurden an einem Mettler FP 51 bzw. an einem Büchi Melting Point B 545 bestimmt: Aufheizrate 2°C/min., Mittelwert (40 % Schwelle) aus 3 Messungen mit regulärem Schmelzvertauf (Schmelzverlaufs-Diagramm), Kalibrierung gegen Vanillin (83,0 °C), Phenacetin (136,0 °C) und Coffein (237 °C).
Die IR-Spektren wurden an einem Shimadzu IR 470; oder FT-IR Nicolet Impact 410 aufgenommen, an letzterem mit Omnic (Vers. 2.1) bearbeitet. Die Substanz wurde dazu in KBr-Tabletten verpreßt oder als Film zwischen NaCl-Platten im Durchlicht vermessen.
Die NMR-Spektren wurden an einem Bruker AC 200 aufgenommen (1H: 200 MHz, 13C: 50.3 MHz). Dazu wurden 20 - 50 mg Substanz, in ca. 0,5 - 1,0 mL eines deuterierten Lösungsmittels, das 0,1 % Tetramethylsilan als internen Standard enthielt, klar gelöst. Die Lösung wurde in einem 0.5 cm Probenröhrchen in den Probenraum verbracht. Unter D-Lock wurden für 1H-NMR-Spektren in der Regel 32 FIDs summiert und nach dem FT-Verfahren in Frequenzspektren umgerechnet, für die Aufnahme von 13C-NMR-Spektren wurden zwischen 500 und 10000 FIDs gesammelt.
UV-Spektren wurden am UV-Perkin-Euner 550 SE UV/VIS-Spektroskop aufgenommen. Die Messung erfolgte in 1cm Quarzküvetten in einem geeigneten Lösungsmittel.
Massenspektren wurden am GC-MS-System HP-MSD (HP 6890 GC - HP 5973 MSD) aufgenommen, die Anregung erfolgte nach GC-Trennung im Hochvakuum (10⁻⁶ Torr) durch EI, bei 70 eV Elektronenenergie.

Die Bestimmung flüchtiger und thermostabiler Verbindungen erfolgte mittels GC am GC-MSD. Die Trennung erfolgte, soweit keine anderen Angaben gemacht werden, an einer EP-5MS-Säule (30 m, 0,25 mm, 5% Phenylmethylsilikon, 0,25µm) mit Helium bei konstantem Fluß von 1,2 mL und einem Splitverhältnis von 50:1 nach der Flächenmethode des TIC.
Die Bestimmung schwerflüchtiger und thermolabiler Verbindungen erfolgte mittels HPLC an einem Merck-Hitachi-HPLC (L4000 Detector, L6200A Intelligent Pump, D 2500 Chromato-Integrator) nach der Flächenmethode, UV-Absorption gemessen am Absorptionsmaximum oder bei 256 nm. Die Säule (soweit nichts anderes angegeben LiChroCART 125-4; LiChrosphere 100 RP-18; 5µm; von Merck, Darmstadt) wurde mit einem WO-Electronics BFO-04 SV Säulenofen thermostatisiert (30 °C). Die Trennung erfolgte in der Regel mit einem Gradienten eines ternären Gemisches aus Methanol, Acetonitril und einem 5 mMol NaH₂PO₄-Puffer, der mit H₃PO₄ auf einen pH-Wert von 3,5 eingestellt ist, mit einer Flußrate von 1 mL/min nach Injektion von 20 µL einer Lösung von 30 - 40 mg der zu untersuchenden Substanz in 100 mL MeOH.

### Beispiel 1

### 2-(4-Methylmercapto-phenyl)-1-phenyl-6,7-dihydro-5H-pyrrolizin

### a) 3-Phenylacetyl-1-vinyl-pyrrolidin-2-on (nach Lit. Haslego M. L., Maryanoff C. A., Scott L., and Sorgi K. L., Heterocycles, 35, 643-647 (1993))

In einem 3L-Dreihalskolben wird unter Stickstoff in abs. THF (Tetrahydrofuran) (1400 mL) Natriumhydrid (300 g, 60 %ig in Weißöl, 7.5 Mol) suspendiert und die Suspension wird 30 min. erhitzt, während dessen die anfängliche Wasserstoffentwicklung abklingt. Man läßt auf RT (Raumtemperatur) abkühlen und tropft anschließend ein Gemisch von Phenylessigsäureethylester (457 g, 2,78 Mol) und 1-Vinyl-2-pyrrolidon (278 g, 2.5 Mol) in THF abs. (400 mL) über einen Tropftrichter zu.

Die Zugabegeschwindigkeit wird so gewählt, daß die Wasserstoffentwicklung unter Kontrolle gehalten werden kann (3.5 h). Nach vollständiger Zugabe wird der Rückfluss (66°C, IT (Innentemperatur)) weitere 3 h beibehalten; es wird auf RT abgekühlt und 16 h stehen gelassen. Der Natiumhydridüberschuß wird durch vorsichtige Zugabe einer gesättigten (37%ig) Ammoniumchloridlösung (2L) zur Ansatzlösung zerstört. Die organische Phase wird in einem Scheidetrichter abgetrennt, mit Na₂SO₄ getrocknet, und das Filtrat wird eingeengt. Das sich als eigenständige Phase abscheidende Weißöl wird abdekantiert. Es verbleibt ein öliger Rückstand von 569 g (99.4%) mit einer Reinheit von 90 % gc. (gaschromatographisch): 8 -9 % Phenylessigsäureethylester. Das erhaltene Material kann ohne weitere Reinigung im nächsten Schritt eingesetzt werden.

IR (NaCl): 1/λ (cm⁻¹) = 3307 (br), 3030 (CH), 2960, 2893 (CH), 1693 (CO), 1633, 1454, 1427, 1389, 1279, 700;
¹HNMR (CDCl₃): δ (ppm) = 7.37 - 7.15 (m, 5 H, arom), 7.09 - 6.97 (ABX, 1H, N-CH=), 4.52 - 4.40 (ABX, 2H, =CH₂), 4.19 - 4.00 (AB, 2 H, PhCH₂CO), 3.82 - 3.73 (m, 1 H, 3-H), 3.53 - 3.39 (m, 2 H, 5-CH₂), 2.80 - 2.40 (m, 1 H, 4-H), 2.20 - 1.90 (m, 1 H, 4-H);
¹³CNMR (CDCl₃): δ (ppm) = 203.0 (CO), 173.3 (CONR), 133.7 (C-quart.), 129.7, 128.6, 126.9 (CH), 52.7 (C-3), 49.3 (CH₂), 40.3 (CH₂), 22.4 (CH₂).

### b) 2-Benzyl-1-pyrrolin

### (nach Lit. Haslego M. L., Maryanoff C. A., Scott L., and Sorgi K. L., Heterocycles, 35, 643-647 (1993))

In einem 5L-Dreihalskolben werden mit Wasser (1.3 L) und HCl (36%, 1.3 L, 15 Mol) eine 6 N HCl zubereitet, und diese wird anschließend zum Rückfluß erhitzt. Über einen Tropftrichter wird die Lösung des rohen 3-Phenylacetyl-1-vinyl-pyrrolidin-2-on (552 g, 2.5 Mol) in THF (1L) zur heißen Säure getropft, und der unmittelbar freigesetzte flüchtige Acetaldehyd wird über eine Destillationsbrücke aus dem Ansatz destilliert (Zeitbedarf 2 h).
Die Mischung wird für weitere 16 h unter Rückfluß gehalten und nach dem Abkühlen in einem Eisbad bei RT mit Toluol (0.7L) versetzt. Die organische Phase wird in einem Scheidetrichter abgetrennt und die HCl-Phase mit Diethylether (0.7L) extrahiert. Die Extrakte werden verworfen. Die wäßrige Phase wird unter Kühlen mit zerkleinertem Eis (0.4 Kg) mit Natronlauge (32 %, 1L, 10 Mol) alkalisch eingestellt (pH 9 - 10). Das sich dabei abscheidende Öl wird in Diethylether (0.7L) aufgenommen, und die abgetrennte Wasserphase wird nochmals mit Diethylether (0.7L) extrahiert. Die vereinte Etherlösung wird mit Wasser gewaschen (1 L), über Na₂SO₄ sicc. (getrocknet) getrocknet, und das Filtrat wird eingeengt. Man erhält 304 g (75 %) 2-Benryl-1-pyrrolin mit 90 % Reinheit (gc).
Y: 75 % = 304 g (90%);
Mp: ölig;
IR (NaCl): 1/λ (cm⁻¹) = 3350 (br), 3028 (CH), 2958, 2868 (CH), 1641, 1603, 1495, 1454, 1429, 704;
¹HNMR (CDCl₃): δ (ppm) = 7.35 - 7.19 (m, 5H, CH, arom), 3.88 - 3.78 (t, 2H, 5-CH₂), 3.68 (br, 2H, PhCH₂), 2.44 - 2.34 (t, 2H, 3-CH₂), 1.91 - 1.75 (quin, 2H, 4-CH₂);
¹³CNMR (CDCl₃): δ (ppm) = 176.8 (C-2), 136.8 (C-quart.), 128.9, 128.5, 126.5 (CH), 60.7 (CH₂), 40.5 (CH₂), 36.4 (CH₂), 22.5 (CH₂).

### c) 2-(4-Methylmercapto-phenyl)-1-phenyl-6,7-dihydro-5H-pyrrolizin

2-Benzyl-1-pyrrolin (2.0 g, 80 %ig, 10 mMol), gelöst in MeOH (30 mL), wird mit einer Lösung von 2-Brom-1-(4-methylsutfanyl-phenyl)-1-ethanon (Beispiel 2, 2.0 g, 8 mMol) in CH₂Cl₂ (10 mL) und anschließend mit NaHCO₃ (1.5 g, 18 mMol) versetzt. Es wird bei RT gerührt bis eine DC-Probe (Al₂O₃, Hexan/Ether 9:1) die vollständige Umsetzung des Bromketons (Rf 0.75) und eine ausreichende Bildung von Produkt (Rf = 0.5) anzeigt (48 h). Nach Zugabe von Wasser (50 mL) wird mit Diethylether (3-mal 30 mL) extrahiert, die vereinigten Extrakte werden über Na₂SO₄ sicc. getrocknet und eingeengt. Der Rückstand wird sc (Al₂O₃, Hexan/Ether 9:1) gereinigt. Man isoliert 0.62 g (25.2%) der gesuchten Verbindung.
Mp: 112°C
IR (NaCl): 1/λ (cm⁻¹) = 3050, 1695, 1595, 1521, 1484, 1335, 1179, 1003;
¹HNMR (CDCl₃): δ (ppm) = 7.25-7.10 (m, 9H, arom), 6.72 (s, 1H, CH), 3.98 (t, 2H, CH₂), 2.96 (t, 2H, CH₂), 2.52-2.48 (quin, 2H, CH₂), 2.44 (s, 3H, SCH₃).

### Beispiel 2

### 2-(4-Methylsulfinyl-phenyl)-1-phenyl-6,7-dihydro-5H-pyrrolizin

### a) 1-(4-Methylsulfanyl-phenyl)-1-ethanon

In eine auf 0°C gekühlte Suspension von AlCl₃ (16 g, 0.12 Mol) in Dichlorethan (50 mL) gibt man Acetylchlorid (8.2 g, 0.105 Mol); man rührt 15 min. und tropft anschließend eine Lösung von Thioanisol (12.4 g, 0.1 Mol) in Dichlorethan (20 mL) zu; die Temperatur wird durch Kühlung unter 20 °C gehalten (30 min.). Es wird noch 1 h gerührt und über Nacht bei RT stehen gelassen. Unter Kühlung wird der Lewis-Säure-Komplex durch Eiswasserzugabe (100 mL) zerlegt, die organische Phase wird in einem Scheidetrichter abgetrennt, und die Wasserphase wird noch 2-mal mit Dichlorethan (100 mL) extrahiert. Die vereinigten Extrakte werden über Na₂SO₄ sicc. getrocknet und auf ¼ des Ausgangsvolumens eingeengt. Der rotbraune Rückstand (15.2 g) wird mit Diethylether (20 mL) und Hexan (20 mL) gewaschen: es ergeben sich 12 g hellrote (ziegelrote) Substanz.
Y: 72 % (12 g) C₉H₁₀OS, MW = 166.24;
IR (NaCl): 1/λ (cm⁻¹) = 1664;
¹HNMR (CDCl₃): δ (ppm) = 7.89-7.85 (m, 2H, AA', arom), 7.28-7.24 (m, 2H, BB', arom), 2.57 (s, 3H, COCH₃), 2.52 (s, 3H, SCH₃).

### b) 2- Brom-1-(4-methylsulfanyl-phenyl)-1-ethanon

Zu einer Lösung von 1-(4-Methylsulfanyl-phenyl)-1-ethanon (5 g, 30 mMol) in CH₂Cl₂ (10 mL) tropft man innerhalb einer Stunde eine Lösung von Brom (4,8 g, 30 mMol) in CH₂Cl₂ (20 mL) bei Raumtemperatur zu. Es wird eine Stunde weiter gerührt bis zum Verschwinden des Eduktes (dc-Kontrolle SiO₂, CH₂Cl₂/Hexan 1:1). Daraufhin wird auf Wasser (30 mL) gegossen, die Wasserphase abgetrennt und die organische Phase mit NaHCO₃-Lsg (20 mL, 8%-ig) neutral gewaschen, anschließend getrocknet (Na₂SO₄ sicc.) und eingeengt.
Y: 95% (7.0 g), C₉H₉BrOS, MW = 245.14;
Mp: 67-68 °C aus Hexan;
IR (NaCl): 1/λ (cm⁻¹) = 3005, 2945, 1687, 1581, 1551, 1402, 1313, 1281, 1202, 1189, 1091, 992, 969, 956, 816, 728, 660;
¹HNMR (CDCl₃): δ (ppm) = 8.20-8.05 (AA'BB', 4H, arom), 4.46 (s, 2H, CH₂Br), 3.10 (s, 3H, SCH₃).

### b') 2-Chlor-1-(4-methylsulfanyl-phenyl)-1-ethanon

In eine auf 0°C gekühlte Suspension von AlCl₃ (16 g, 0.12 Mol) in Dichlorethan (50 mL) gibt man Chloracetylchlorid (11.8 g, 0.105 Mol); man rührt 15 min. und tropft anschließend eine Lösung von Thioanisol (12.4 g, 0.1 Mol) in Dichlorethan (20 mL) zu; die Temperatur wird durch Kühlung unter 20 °C gehalten (30 min.). Es wird noch 1 h gerührt und dann unter Kühlung der Lewis-Säure-Komplex durch Eiswasserzugabe (100 mL) zerlegt, die organische Phase wird in einem Scheidetrichter abgetrennt, und die Wasserphase wird noch 2-mal mit CHCl₃ (100 mL) extrahiert. Die vereinigten Extrakte werden über Na₂SO₄ sicc. getrocknet und auf ¼ des Ausgangsvolumens eingeengt. Der rotbraune Rückstand wird mit Hexan (20 mL) versetzt, das kristallisierende Produkt wird abgesaugt und aus Hexan umkristallisiert: man erhält 4 g (20 %) lachsfarbene Substanz.
Mp: 76,5 °C aus Hexan;Y: 20 % (4 g) C₉H₉ClOS, MW = 200.69;
IR (NaCl): 1/λ (cm⁻¹) = 1687, 1577, 1546, 1394, 1213, 1086, 817;
¹HNMR (CDCl₃): δ (ppm) = 7.89-7.44 (m, 2H, AA', arom), 7.51-7.26 (m, 2H, BB', arom), 4.66 (s, 2H, COCH₂Cl), 2.53 (s, 3H, SCH₃).

### c) 1-Brom-2-(4-methylsulfinyl-phenyl)-2-ethanon

Eine Lösung von 2-Brom-1-(4-methylsulfanyl-phenyl)-1-ethanon (3.7 g, 15 mMol) in CHCl₃ (50 mL) wird auf -5 °C temperiert. Eine auf 0°C gekühlte Lösung von m-Chlor-perbenzoesäure (70 %, 3.7 g, 15 mMol) in CHCl₃ (30 mL) wird unter Beibehaltung einer IT von -5°C bis 0°C zugetropft. Nach ½stündigem Rühren in der Kälte wird die abgeschiedene m-Chlor-benzoesäure abfiltriert. Das Filtrat wird mit NaHCO₃-Lösung (8%ig, 50 mL) zunächst säurefrei gewaschen und anschließend mit Wasser (50 mL) nachgewaschen. Die über Na₂SO₄ sicc. getrocknete CHCl₃-Phase wird im Vakuum eingeengt. Es ergibt sich ein gelblicher Rückstand (1.3 g).
Y: 95 % (1.3 g) C₉H₉BrO₂S, MW = 261.14;
IR (NaCl): 1/λ (cm⁻¹) = 1763, 1706, 1569, 1396, 1290, 1202, 1149, 1103, 997, 956, 826, 758;
¹HNMR (CDCl₃): δ (ppm) = 8.2 - 8.07 (AA'BB', 4H, arom), 4.71 (s, 2H, CH₂Cl), 2.80 (s, 3H, SOCH₃).

### d) 2-(4-Methylsulfinyl-phenyl)-1-phenyl-6,7-dihydro-5H-pyrrolizin

2-Benzyl-1-pyrrolin (2.0 g, 80 %ig, 10 mMol), gelöst in MeOH (30 mL), wird mit einer Lösung von 4-Methylsulfinylphenacyl-bromid (2.1 g, 8 mMol) in CH₂Cl₂ (10 mL) und anschließend mit NaHCO₃ (1.5 g, 18 mMol) versetzt und bei RT gerührt, bis eine DC-Probe (Al₂O₃, Hexan/Ether 9:1) die vollständige Umsetzung des Bromketons (Rf 0.75) und eine ausreichende Bildung von Produkt (Rf = 0.2 ) anzeigt (48 h). Nach Zugabe von Wasser (50 mL) wird mit Diethylether (3-mal 30 mL) extrahiert, die vereinigten Extrakte werden über Na₂SO₄ sicc. getrocknet und eingeengt. Der Rückstand wird sc (Al₂O₃, Hexan/Ether 1:1) gereinigt. Man isoliert 0.3 g (12%) der gesuchten Verbindung.
Y: 12% (0.3 g), C₂₀H₁₉NOS, MW = 321.44
IR (NaCl): 1/λ (cm⁻¹) = 2912, 1586, 1457, 1301, 1140, 1088, 959, 829, 774;
¹HNMR (CDCl₃): δ (ppm) = 7.93 - 7.64 (4H, arom), 7.53 - 7.40 (4H, arom), 6.88 (s, 1H), 4.23 (t, CH₂), 3.16 (t, CH₂), 3.08 (s, CH₃), 2.65 (m, CH₂).

### Beispiel 3

### 2-(4-Methylsulfonyl-phenyl)-1-phenyl-6,7-dihydro-5H-pyrrolizin

### a) 2-Brom-1-(4-methylsulfonyl-phenyl)-1-ethanon

### (hergestellt nach J. Org. Chem. 35, 2106 (1970))

Eine Lösung von 1-Brom-2-(4-methylsulfanyl-phenyl)-2-ethanon (1.25 g, 5 mMol) in CHCl₃ (30 mL) wird auf -5 °C temperiert. Eine auf 0°C gekühlte Lösung von m-Chlor-perbenzoesäure (3.5 g, 10 mMol) in CHCl₃ (30 mL) wird unter Beibehaltung einer IT von -5°C bis 0°C zugetropft. Nach 3-stündigem Rühren in der Kälte wird die abgeschiedene m-Chlor-benzoesäure abfiltriert. Das Filtrat wird mit NaHCO₃-Lösung (8%ig, 50 mL) zunächst säurefrei gewaschen und anschließend mit Wasser (50 mL) nachgewaschen. Die über Na₂SO₄ sicc. getrocknete CHCl₃-Phase wird im Vakuum eingeengt, bis sich eine rötlich-braune Festsubstanz abscheidet. Man sammelt den aus der abgekühlten Mutterlauge abgeschiedenen Feststoff (1.3 g).
Y: 95 % (1.3 g) C₈H₈BrO₃S, MW = 184.22;
Mp: 117.3 °C;
IR (NaCl): 1/λ (cm⁻¹) = 3085, 2935, 1697, 1568, 1335, 1296, 1191, 1145, 1085, 1006, 997, 882, 784, 653;
¹HNMR (CDCl₃): δ (ppm) = 8.20-8.05 (AA'BB', 4H, arom), 4.46 (s, 2H, CH₂Br), 3.10 (s, 3H, SO₂CH₃).

### b) 2-(4-Methylsulfonyl-phenyl)-1-phenyl-6,7-dihydro-5H-pyrrolizin

2-Benzyl-1-pyrrolin (2.0 g, 80 %ig, 10 mMol), gelöst in MeOH (30 mL), wird mit einer Lösung von 4-Methylsulfonylphenacyl-bromid (2.3 g, 8 mMol) in CH₂Cl₂ (10 mL) und anschließend mit NaHCO₃ (1.5 g, 18 mMol) versetzt und bei RT gerührt, bis eine DC-Probe (Al₂O₃, Hexan/Ether 9:1) die vollständige Umsetzung des Bromketons (Rf 0.75) und eine ausreichende Bildung von Produkt (Rf = 0.5) anzeigt (48 h). Nach Zugabe von Wasser (50 mL) wird mit Diethylether (3-mal 30 mL) extrahiert, die vereinigten Extrakte werden über Na₂SO₄ sicc. getrocknet und eingeengt. Der Rückstand wird sc (Al₂O₃, Hexan/Ether 9:1) gereinigt. Man isoliert 0.6 g (25%) der gesuchten Verbindung.
MP: 165, Y 25 % (0.6 g), C₂₀H₁₉NO₂S, MW = 337.44;
IR (NaCl): 1/λ (cm⁻¹) = 3172, 1590, 1311, 1148, 951, 771, 546
¹HNMR (CDCl₃): δ (ppm) = 7.80-7.76 u. 7.43-7.38 (AA'BB', 4H, arom), 7.09-6.96 (m, 4H, arom), 4.23-4.12 (q, J=7.1Hz, 2H), 4.03-3.96 (t, J=7.2Hz, 2H), 3.27-3.19 (t, J=7.4Hz, 2H), 3.05 (s, CH₃), 2.63-2.49 (quin, J=7.2Hz, 2H), 1.26-1.19 (t, J=7.0Hz, CH₃).

### Beispiel 4

### 2-(4-Methylmercaptophenyl)-1-(4-fluorphenyl)-6,7-dihydro-5H-pyrrolizin

### a) 2-(4-Fluorbenzyl)-1-pyrrolin

4-Fluortoluol (5.5 g, 0.05 Mol, N-Bromsuccinimid (NBS, 8.9 g, 0.05 Mol) und Azaisobutyronitril (AIBN, 160 mg, 1 mMol) werden in Carbontetrachlorid (CCl₄, 40 mL) verteilt; es wird 3-4 h bei 75 °C gerührt. Nach dem Abkühlen wird vom abgeschiedenen Succinimid abfiltriert. Mit CCl₄ (10 mL) wird der Rückstand nachgewaschen. Aus den vereinigten CCl₄-Phasen werden beim Einengen im Vakuum 8.5 g 4-Fluorbenzylbromid (89%) als gelbes Öl erhalten.

In einem 500 mL-3-Hals-Kolben werden Magnesiumspäne (für Grignard, 6.4 g, 0.266 Mol) mit 20 mL einer Lösung von 4-Fluorbenzylbromid (50 g, 0.265 Mol) in abs. (absolut) Diethylether (250 mL) überschichtet und bis zum Anspringen der Reaktion kurz erhitzt. Das restliche Volumen an 4-Fluorbenrylbromid wird in 30 min. zugetropft, der Ansatz dabei im Ölbad noch 2 h auf 40 °C temperiert. In die noch warme, frisch bereitete Gringnard-Lösung tropft man innerhalb 30 min. eine Lösung von 4-Chlorbutyronitril (13.6 g, 0.132 Mol ) in Diethylether (50 mL). Der größte Teil des Ether (200 mL) wird über eine Brücke abdestilliert und durch abs. Toluol (250 mL) ersetzt. Man treibt Diethylether solange aus, bis im Boden eine Siedetemperatur von 95 °C erreicht wird. Man rührt noch 2 h bei dieser Temperatur und läßt dann über Nacht bei RT stehen. Die organische Phase wird in einen Scheidetrichter überführt und mit 10 %iger HCl (3-mal je 100 mL) hydrolysiert und extrahiert. Die Toluolphase wird verworfen, die HCl-saure Wasser-Phase nochmals mit Toluol (100 mL) gewaschen und dann mit konz. NaOH (32 %ig) auf pH 10 eingestellt. Im Scheidetrichter wird die sich abscheidende Pyrrolin-Base mit Diethylether (100 mL) aufgenommen. Nach Abtrennen der Etherphase wird nochmals mit Ether extrahiert (2-mal 50 mL), die vereinten Etherphasen werden über K₂CO₃ sicc. getrocknet und eingeengt. Es werden 8.8 g 2-(4-Fluorbenzyl)-1-pyrrolin (37,8% bezogen auf Chlornitril) erhalten.
Bp: 150-153 °C, gelboranges Öl, C₁₁H₁₂FN, MW = 177.22;
IR (NaCl): 1/λ (cm⁻¹) = 2957, 2868, 1641, 1603, 1509, 1430, 1222, 1157.5, 837, 825;
¹HNMR (CDCl₃): δ (ppm) = 7.26 - 6.94 (m, 4H, arom), 3.83 (t, 2H, CH₂), 3.64 (s 2H, CH₂), 2.39 (t, 2H, CH₂), 1.87 (quin 2H, CH₂).

### b) 1-(4-Fluorphenyl)-2-(4-methylsulfanylphenyl)-6,7-dihydro-5H-pyrrolizin

2-Brom-1-(4-methylsulfanyl-phenyl)-1-ethanon (0.83 g, 3.4 mMol), gelöst in Diethylether (10 mL) wird mit einer Lösung von 2-(4-Fluorbenzyl)-1-pyrrolin (0.6 g, 3.3 mMol) in Ethanol (10 mL) und NaHCO₃ (0.6 g, 7 mMol) versetzt. Man rührt die Mischung im Dunkeln bei RT 18 h. Die Umsetzug wird mit DC kontrolliert: SiO₂/Hexan-Ether 9:1. Der Ansatz wird mit H₂O (10 mL) versetzt, mit Diethylether 2-mal (100 mL) extrahiert und die vereinte Etherphase wird anschließend mit Wasser (30 mL) nachgewaschen. Die über Na₂SO₄ sicc. getrocknete organische Phase wird im Vakuum eingeengt. Der verbleibende Rückstand wird sc an Al₂O₃/Hexan/Diethylether 1:1 gereinigt. Fraktionen 1-3 ergeben 330 mg orangerotes Öl.
Y: 33 % (330 mg), C₂₀H₁₈FNS, MW = 323.44;
¹HNMR (CDCl₃): δ (ppm) = 7.27 - 6.89 (2 AA'BB', 8H, 2 arom), 6.74 (s, 1H, CH), 4.04-3.97 (t, 2H, CH₂), 2.97 - 2.90 (tr, 2H, CH₂), 2.59 -2.48 (m, 2H, CH₂), 2.46 (s, 3H, SCH₃).

### Beispiel 5

### 1-(4-Fluorphenyl)-2-(4-methylsulfonyl-phenyl)-6,7-dihydro-5H-pyrrolizin

2-Brom-1-(4-methylsulfonyl-phenyl)-1-ethanon (2.6 g, 9.5 mMol), gelöst in THF (30 mL) wird mit der Lösung von 2-(4-Fluorbenzyl)-1-pyrrolin (1.77 g, 10 mMol) in Methanol (50 mL) und NaHCO₃ (1.7 g, 20 mMol) versetzt. Man rührt die Mischung im Dunkeln bei RT 70 h, setzt Wasser (30 mL) hinzu und rührt weitere 5 h. Mit Diethylether wird 3-mal (100 mL) extrahiert, die vereinte Etherphase wird anschließend mit Wasser (50 mL) nachgewaschen und nach Trocknen über Na₂SO₄ sicc. im Vakuum eingeengt. Der verbleibende Rückstand wird sc an Al₂O₃ / Ethylacetat gereinigt. Fraktionen 3-4 ergibt
1.9 g mit einem Schmp. von 169.1 °C.
Y: 56 % (1.9 g), C₂₀H₁₈FNO₂S, MW = 355.43;
IR (NaCl): 1/λ (cm⁻¹) = 2924, 1592, 1528, 1502, 1404, 1307, 1215, 1152, 1090, 948, 841, 766;
¹HNMR (CDCl₃): δ (ppm) = 7.78 - 7.74 / 7.39 - 7.34 (AA'BB', 4H, arom), 7.29-6.92 (m, 4H, arom), 6.85 (s, 1H, CH), 4.09 - 4.02 (t, 2H, CH₂), 3.05 (s, 3H, CH₃), 2.97 - 2.90 (t, 2H, CH₂), 2.62 - 2.49 (quin, 2H, CH₂).

### Beispiel 6

### 2-(4-Fluorphenyl)-1-(4-methylsulfanyl-phenyl)-6,7-dihydro-5H-pyrrolizin

### a) 2-(4-Methylsulfanyl-benzyl)-1-pyrrolin

In abs. THF (150 mL) wird unter Stickstoff Natriumhydrid (23.9 g, 60 %ig in Weißöl, 0.59 Mol) suspendiert, und die Suspension wird zum Rückfluß erhitzt. Man läßt anschließend ein Gemisch von (4-Methylsulfanylphenyl)essigsäureethylester (38 g, 0.18 Mol) und 1-Vinyl-2-pyrrolidon (20 g, 0.18 Mol) in THF abs. (70 mL) über einen Tropftrichter zutropfen.
Die Zugabegeschwindigkeit wird so gewählt, daß die Wasserstoffentwicklung unter Kontrolle gehalten werden kann (1.5 h). Nach vollständiger Zugabe wird der Rückfluss (66 °C, IT) weitere 4 h beibehalten; dann wird auf RT abgekühlt. Der Natiumhydridüberschuß wird durch vorsichtige Zugabe einer gesättigten (37%ig) Ammoniumchloridlösung (150 mL) zur gekühlten Ansatzlösung zerstört. Die THF-Phase wird in einem Scheidetrichter abgetrennt, in der Wasserphase ausgefallener Feststoff wird durch Wasserzugabe in Lösung gebracht und mit THF (100 mL) extrahiert. Die THF-Phase wird mit Na₂SO₄ sicc. getrocknet und das Filtrat wird eingeengt. Das sich abscheidende Weißöl wird abdekantiert. Es verbleibt ein öliger Rückstand von 57 g (115 %) mit einer Reinheit von 80 % (gc: 4-Methylsulfanylphenylessigsäureethylester, THF, Paraffin)
Das erhaltene Material wird ohne weitere Reinigung im nächsten Schritt eingesetzt.

In einem 1L-Dreihalskolben mit Wasserabscheider wird 6N HCl (400 mL) zum Rückfluß erhitzt. Über einen Tropftrichter wird die Lösung des rohen 3-(4-Methylsulfanylphenyl-acetyl)-1-vinyl-pyrrolidin-2-on (58 g, 80%ig, 170 mMol) in THF (200 mL) über einen Tropftrichter zur heißen Säure getropft, und der unmittelbar freigesetzte flüchtige Acetaldehyd wird zusammen mit THF über den Wasserabscheider aus dem Ansatz destilliert (Zeitbedarf 2 h).
Die Mischung wird weitere 6 h unter Rückfluß gekocht und nach dem Abkühlen in einem Eisbad bei RT mit CHCl₃ (200 mL) versetzt. Die organische Phase wird in einem Scheidetrichter abgetrennt und verworfen, die HCl-Phase unter Kühlen mit zerkleinertem Eis (0.2 Kg) mit Natronlauge (32 %, 400 mL) alkalisch eingestellt (pH 11 - 12). Das sich dabei abscheidende Öl wird in CHCl₃ (100 mL) aufgenommen und die abgetrennte Wasserphase wird 2-mal mit CHCl₃ (200 mL) extrahiert. Die vereinte CHCl₃ -Lösung wird mit Wasser gewaschen (1 L), über Na₂SO₄ sicc. getrocknet und das Filtrat wird eingeengt. Man erhält 22.3 g (64.3 %) 2-(4-Methylsulfanyl-henzyl)-1-pyrrolin mit 80 % Reinheit (gc).
Y: 64.3 % = 22.3 g (80%),
Mp: ölig, C₁₂H₁₅NS, MW = 205.32;
IR (NaCl): 1/λ (cm⁻¹) = 3238, 2920, 2866, 1690, 1640, 1493, 1424, 1092;
¹HNMR (CDCl₃): δ (ppm) = 7.23 - 7.12 (AA'BB', 4H, arom), 3.9 -3.75 (m, 2H, CH₂), 3.63 (br, 2H, CH₂), 2.465 (s, 3H, SCH₃), 2.45 - 2.35 (m, 2H, CH₂), 1.90 - 1.79 (quin, 2H, CH₂).

### b) 2-(4-Fluorphenyl)-1-(4-methylsulfanyl-phenyl)-6,7-dihydro-5H-pyrrolizin

2-(4-Methylsulfanyl-benzyl)-1-pyrrolin (19.3 g, 80 %ig, 0.1 Mol), gelöst in MeOH (100 mL), wird mit einer Suspension von 2-Brom-1-(4-fluorphenyl)-1-ethanon (21.7 g, 0.1 Mol) in MeOH (50 mL) und anschließend mit NaHCO₃ (8.4 g, 0.1 Mol) versetzt und bei RT 72 h gerührt (DC-Probe (Al₂O₃, Hexan/Ether 9:1)).
Der aus der MeOH-Phase abgeschiedene Feststoff wird abfiltriert, in Aceton gelöst, nicht gelöste Salze werden abfiltriert und Aceton wird im Vakuum entfernt. Der Rückstand wird mit heißem Diisopropylether digeriert und daraus in der Kälte kristallisiert: es ergeben sich 3 g braungefärbte Kristalle.
Die MeOH-Filtrat-Lösung wird eingeengt und mit Diethylether digeriert. Der erhaltene kristalline braune Bodenkörper (9 g) wird verworfen.
Die Diethylether-Extrakte werden vereinigt und eingeengt. Der Rückstand (10 g), ein rotbraunes zähes Öl, wird sc auf SiO₂ / CHCl₃ gereinigt. 3g (9.3%) der gesuchten Verbindung werden erhalten.
Y: 9.3 % (3 g); C₂₀H₁₈FNS, MW = 323.44;
Mp: ölig;
IR (NaCl): 1/λ (cm⁻¹) = 3424, 2918, 1691, 1596, 1493, 1402, 1223, 1156, 1090, 837;
¹HNMR (CDCl₃): δ (ppm) = 7.23-7.10 (m, 6H, arom), 7.0-6.88 (m, 2H, F-arom), 4.01 (t, 2H, CH₂), 2.96 (t, 2H, CH₂), 2.522 (quin, 2H, CH₂), 2.460 (s, 3H, SCH₃); GC-MS : m/e (rel.Int.[%]) = 323 (M^{+•}, 100%), 308 ((M-CH₃)^{+•}, 10%).

### Beispiel 7

### Methyl-4-[2-(4-fluorphenyl)-6,7-dihydro-5H-pyrrolizin-1-yl]-phenyl-sulfon (synonym 2-(4-Fluorphenyl)-1-(4-methansulfonyl-phenyl)-6,7-dihydro-5H-pyrrolizin)

### a) 2-(4-Fluorphenyl)-1-phenyl-6,7-dihydro-5H-pyrrolizin-3-carbonsäuremethylester

Eine Lösung von 6-(4-Fluorphenyl)-7-phenyl-2,3-dihydro-1H-pyrrolizin (hergestellt nach Laufer et al., 1.11 g, 4 mMol) in CHCl₃ (20 mL) wird in einem Eisbad auf 0°C gekühlt, mit Triethylamin (0,44 g, 4,4 mMol) versetzt, und Diphosgen (0.43 g, 2,2 mMol), gelöst in CHCl₃ (5 mL) wird zugetropft. Nach beendeter Zugabe in der Kälte wird noch 4h bei RT gerührt. Nach Zugabe von MeOH (4 mL) wird 16 h bei RT weitergerührt. Die Reaktionslösung wird mit Wasser, und dann mit Na₂CO₃-Lösung (10 %) säurefrei gewaschen und schließlich nochmals mit Wasser gewaschen. Die abgetrennte CHCl₃-Phase wird mit Na₂SO₄ sicc. getrocknet und eingeengt. Als Rückstand werden 1.3 g (100%) 2-(4-Fluorphenyl)-1-phenyl-6,7-dihydro-5H-pyrrolizin-3-carbonsäuremethylester erhalten (gc. 93% ).
Die Substanz wird ohne weitere Reinigung im nächsten Umsetzungsschritt verwendet.
Y: 100 % = 1.3 g (93 %), C₂₁H₁₈FNO₂, MW = 335.38.
Mp: 160.0 °C
IR (NaCl): 1/λ (cm⁻¹) = 2997, 2955, 2900, 1701(Ester), 1600, 1550, 1525, 1469, 1401, 1313, 1217, 1121, 1095, 847, 783, 698;
¹HNMR (CDCl₃): δ (ppm) = 7.24 - 6.95 (m, 9H, arom.); 4.38 (t, 2H, CH₂, J=7,2Hz); 3.64 (s, 3H, CH₃,); 3.02 (t, 3H, CH₂); 2.54 (quin, 2H, CH₂);
GC-MS : m/e (rel.Int.[%]) = 335 (M^{+•}, 100%), 304 ((M-OCH₃^{•})⁺, 22%), 276 ((M-COOCH₃^{•})⁺, 22%), 248 (10%).

### b) 4-[2-(4-Fluorphenyl)-3-methoxycarbonyl-6,7-dihydro-5H-pyrrolizin-1-yl]-benzolsulfonsäurechlorid

In getrocknetem (CaCl₂ sicc.) CHCl₃ (10 mL) wird unter Stickstoff der 2-(4-Fluorphenyl)-1-phenyl-6,7-dihydro-5H-pyrrolizin-3-carbonsäuremethylester (1.3 g, 3.8 mmol) gelöst. Anschließend läßt man Chlorsulfonsäure (2,66 g, 22,8 mMol) in CHCl₃ (5 mL) über einen Tropftrichter zutropfen. Nach vollständiger Zugabe wird 16 h bei RT gerührt.
Die Ansatzlösung wird auf Eis/Wasser (80 mL) gegossen und die abgeschiedene CHCl₃/Ölphase wird in weiterem CHCl₃ (20 mL) aufgenommen. Die CHCl₃-Phase wird in einem Scheidetrichter abgetrennt, die Wasserphase wird mit NaCl gesättigt und erneut mit CHCl₃ (30 mL) extrahiert. Die gesammelte CHCl₃-Phase wird mit Na₂SO₄ sicc. getrocknet, und das Filtrat wird eingeengt. Das beim Einengen sich abscheidende Produkt wird abgesaugt. Es verbleiben 1.7 g (106 %), die ohne weitere Reinigung im nächsten Schritt eingesetzt werden.
Y: 106 % = 1.7 g (85% gc), C₂₁H₁₇ClFNO₄S, MW = 433.89;
IR (NaCl): 1/λ (cm⁻¹) = 1693, 1587, 1525, 1371, 1225, 1177, 1094, 837, 584, 561;
¹HNMR (CDCl₃): δ (ppm) = 7.83 - 7.78 (AA', 2H, arom), 7.22 - 6.97 (m, 6H, BB' + F-arom), 4.428 (t, 2H, J=7Hz, CH₂), 3.657 (s, 3H, OCH₃), 3.082 (t, 2H, J=7Hz, CH₂), 2.609 (quin, 2H, J=7Hz);

### c) 2-(4-Fluorphenyl)-1-(4-methylsulfonyl-phenyl)-6,7-dihydro-5H-pyrrolizin-3-carbonsäuremethylester

4-[2-(4-Fluorphenyl)-3-methoxycarbonyl-6,7-Mydro-5H-pyrrolizin-1-yl]-benzolsulfonsäurechlorid (1.7 g, 3.8 mMol), suspendiert in Wasser (10 mL), wird mit Na₂SO₃ (0.56 g, 4.4 mMol) und anschließend mit Na₂CO₃ (0.72 g, 6.8 mMol) versetzt und 2 h bei 70 °C gerührt. Die Lösung wird mit einer Lösung von CH₃J (0.88 g, 6.2 mMol) in Ethanol (20 mL) versetzt und 2 h unter Rückfluß erhitzt. Nach dem Abkühlen wird der Ethanol im Vakuum entfernt, der Rückstand mit Wasser (25 mL) versetzt und das Produkt der Umsetzung in Ethylacetat (2-mal 30 mL) aufgenommen. Die Ethylacetatphase wird mit konz. NaCl-Lösung und Wasser gewaschen, mit Na₂SO₄ sicc. getrocknet und das Filtrat wird eingeengt. Nach dem Entfernen des Lösemittels im Vakuum verbleiben 0.4 g (25%) 2-(4-Fluorphenyl)-1-(4-methylsulfonyl-phenyl)-6,7-dihydro-5H-pyrrolizin-3-carbonsäuremethylester als Rückstand mit einem Gehalt von 77 %.
Y: 25 % = 0.4 g (gc. 77 %ig), C₂₂H₂₀FNO₄S, MW = 413.47;
Mp.: 191.0 °C
IR (NaCl): 1/λ (cm⁻¹) = 1716, 1693, 1595, 1527, 1311, 1221, 1150, 1097, 778, 556,
¹HNMR (CDCl₃): δ (ppm) = 7.74 - 7,69 (AA', 2H, atom), 7,22 - 6,95 (m, 6H, BB' + F-arom), 4.42 (t, 2H, J=7Hz, CH₂), 3.646 (s, 3H, OCH₃), 3.055 (t, 2H, J=7Hz, CH₂), 3.03 (s, 3H, SO₂CH₃), 2.594 (quin, 2H, J=7Hz);
GC-MS : m/e (rel.Int.[%]) = 413(M^{+•}, 100%), 382 ((M-OCH₃^{•})⁺, 14%), 355 (10%), 274(12%).

### d) Methyl-4-[2-(4-Fluorphenyl)-6,7-dihydro-5H-pyrrolizin-1-yl]-phenyl-sulfon

2-(4-Fluorphenyl)-1-(4-methylsulfonyl-phenyl)-6,7-dihydro-5H-pyrrolizin-3-carbonsäuremethylester (0.4 g, 0.96 mMol) wird in methanolischer KOH (2 n, 9 mL, 1.01 g KOH, 18 mMol) 1 h unter Rückfluß erhitzt. Nach dem Abkühlen wird Wasser (20 mL) zugesetzt, und die alkalische Phase wird mit HCl (konz. 25 %) schwach sauer (pH 3) eingestellt. Die abgeschiedene Substanz wird mit Ethylacetat extrahiert, der Ethylacetatextrakt mit Na₂SO₄ sicc. getrocknet und nach dem Filtrieren eingeengt. Nach dem Entfernen des Lösemittels im Vakuum verbleiben 0.4 g (75% gc.) Rückstand. Die sc Reinigung erfolgt über SiO₂ mit Ethylacetat als Elutionsmittel. Die Hauptfraktionen werden im Vakuum eingeengt und der Rückstand wird aus Diisopropylether umkristallisiert:
Y: 17 % = 0.06 g (gc. 95 %), C₂₀H₁₈FNO₂S, MW = 355.43;
IR (NaCl): 1/λ (cm⁻¹) = 2924, 1593, 1535, 1308, 1219, 1149, 954, 839, 776, 554, 537;
¹HNMR (CDCl₃/DMSO-d6): δ (ppm) = 7.78 - 7.74 (AA'.2H, SO₂CH₃.arom-7); 7.34 -6.9 (m, BB', 2H, SO₂CH₃ arom-7, + m, 4H, F-arom), 6.73 (s, 1H, pyrroliz..); 4.045 (t, 2H, CH₂, J=7,1); 3.087 - 2.95 (m, 5H, CH₂, CH₃,); 2.60 (quin., 2H, CH₂);
GC-MS : m/e (rel.Int.[%]) = 355 (M^{+•}, 100%), 276, 248, 220.

### Beispiel 8

### 2-(4-Fluorphenyl)-1-(4-methansulfonyl-phenyl)-3-methyl-6,7-dihydro-5H-pyrrolizin

### a) 6-(4-Fluorphenyl)-7-phenyl-2,3-dihydro-1H-pyrrolizin

Nach der Vorschrift von Laufer et al. (J. Med. Chem. 1994, 37, 1894-1897) wird in einem Rundkolben einer Lösung von 2-Benzylpyrrolin (Beispiel 1 a, 15 g, 80 %, 0.075 Mol) in Ethanol (150 mL) zunächst 2-Brom-1-(4-Fluorphenyl)-ethanon (10.8 g, 0.05 Mol) in Anteilen und anschließend NaHCO₃ (5.0 g, 0.06 Mol) zugesetzt. Die Reaktionsmischung wird unter Lichtausschluß 24 - 60 h gerührt; während dieser Zeit wird die Temperatur der Lösung zwischen 30 - 40 °C gehalten.
Die sich abscheidenden Kristalle werden abfiltriert, mehrmals mit kaltem EtOH gewaschen und getrocknet. Es werden 11 g Rohprodukt erhalten, das noch anorganische Salze enthält.
Y: < 79%, NaBr, NaHCO₃, C₁₉H₁₆FN, MW = 277.34;
IR (NaCl): 1/λ (cm⁻¹) = 3447, 3040, 2949, 2888, 1596, 1534, 1496, 1396, 1213, 1155, 839, 766, 701; ¹HNMR (CDCl₃): δ (ppm) =7.26-7.13 (m, 9Harom), 6.74 (s,1H, CH=C), 4.05-3.98 (t, 2H, CH₂), 3.01-2.93 (t, 2H, CH₂ ), 2.56-2.49 (quin, 2H, CH₂ );
¹³CNMR (CDCl₃): δ (ppm) = 161.18 (d, C-F, J=242 Hz), 136.19 (d, J=2.0 Hz), 132.63 (d, J=3.2 Hz), 129.73 (d, J= 7.7 Hz), 128.52, 128.11, 126.34, 124.946, 114.88 (d, J=21.0 Hz), 113.74, 112.96 (Pyrrol-C-H), 46.48, 27.34, 24.51.

### b) 2-[2-(4-Fluorphenyl)-1-phenyl-6,7-dihydro-5H-pyrrolizin-3-yl]-essigsäure

Laufer et al. (J. Med. Chem. 1994, 37, 1894-1897)

Oxalsäuredichlorid (11.4 g, 0.09 Mol) wird zur eiskalten Lösung von 6-(4-Fluorphenyl) -7-phenyl-2,3-dihydro-1H-pyrrolizin (s.o. 16.6 g, 0.06 Mol) in THF (100 mL) bei 0-10 °C zugetropft. Nach vollständiger Zugabe wird eine weitere Stunde gerührt und anschließend vorsichtig Eiswasser (30 mL) eingetropft, was zu einer starken Gasentwicklung und zum Aufschäumen des Ansatzes führt. Die Gase (HCl, CO, CO₂) werden durch einen Absorber geführt. Hydrazin (36.5 mL, 37.5 g, 0.6 Mol) wird zugetropft und das Diethylenglykol (72 mL) wird zugegossen.

Man erhitzt die Reaktionsmischung auf 100 °C, unter Atmosphärendruck gehen an einer Destillationsbrücke THF und Wasser über. Nach Erhöhen der Temperatur im Sumpf auf 105 °C setzt Schäumen ein. Man kühlt ab und gibt bei 30 - 40 °C Kaliumhydroxid-Lösung (50 % in Wasser (44g), 62.8 g KOH 85%, 1.32 Mol) in 5 Portionen zu und steigert die Temperatur in Schritten. Bei 50 -60 °C setzt die Gasentwicklung (N₂) wieder ein (Achtung starkes Schäumen). Bei 135 °C (IT) gehen Wasser und restliche Lösemittel über (H₂O). Man hält diese Temperatur im Sumpf etwa 2 h, dann läßt die Gasentwicklung nach und die Farbe des Ansatzes hellt sich auf. Hat die Mischung eine hellbeige Färbung erreicht, wird der Ansatz auf Eis gegossen. Mit HCl 10 %ig (175 g, 150 mL HCl 32 %, Wasser 300 mL) wird die Reaktionsmischung auf pH 2-3 angesäuert, während durch Kühlen mit Eis die Temperatur unter 10 °C gehalten wird, um die Zersetzung des Produktes zurückzudrängen.
Das Produkt, das sich in Form feiner Kristalle aus der sauren Lösung abscheidet, wird abfiltriert, und mit Wasser gewaschen (pH-Wert der Waschlösungen > 5.0).
Scheidet sich das Produkt Öl ab, wird mit Ethylacetat (3-mal 100 mL) extrahiert, mit Na₂SO₄ sicc. getrocknet, und das Filtrat wird eingeengt. Man erhält 13 g (65%) öligen Rückstand, der aus Diisopropylether kristallisiert.
Y: 65 % = 13 g (2%), C₂₁H₁₈FNO₂, MW = 335.38;
IR (NaCl): 1/λ (cm⁻¹) = 3436, 1721, 1600, 1530, 1501, 1393, 1222, 1181, 1156, 839, 699;
¹HNMR (CDCl₃): δ (ppm) = 7.25-6.93 (m, 9H, arom), 4.06-3.99 (t, 2H, -CH₂), 3.61 (s, 3H, -CH₃), 3.08-3.01 (t, 2H, -CH₂), 2.59-2.52 (quin, 2H, -CH₂).

### c) 6-(4-Fluorphenyl)-5-methyl-7-phenyl-2,3-dihydro-1H-pyrrolizin

Ein Kolben, gefüllt mit kristalliner Festsubstanz der 2-[2-(4-Fluorphenyl)-1-phenyl-6,7-dihydro-5Hpyrrolizin-3-yl]-2-essigsäure (61 g, 0.182 Mol), wird in einem Ölbad auf 180 °C erhitzt. Durch einen Gasblasenzähler wird die Decarboxylierung über die CO₂-Gasfreisetzung kontrolliert, sie ist nach 10 - 15 min beendet. Das Bad wird entfernt, der Inhalt wird auf 50 °C gekühlt und es wird Diisopropylether (100 mL) hinzugegeben. Die Suspension wird bis zum Abkühlen auf Raumtemperatur gerührt, anschließend werden die Kristalle abgesaugt und das Rohprodukt bei Bedarf nochmals aus Diisopropylether kristallisiert.
Man erhält 46 g (86.7%) kristalline Verbindung.
Y: 86.7 % = 46 g (2%), C₂₀H₁₈FN, MW = 291.37;
IR (NaCl): 1/λ (cm⁻¹) = 2880, 1599, 1538, 1500, 1218, 829, 700;
¹HNMR (CDCl₃): δ (ppm) = 7.25-6.90 (m, 9H, arom), 3.94 (t, 2H, J= 7.0 Hz, CH₂), 3.036 (t, 2H, J= 7.0 Hz, CH₂), 2.54 (quin, 2H, J= 7.0 Hz, CH₂), 2.227 (s, 3H, CH₃).

### d) 2-(4-Fluorphenyl)-1-(4-methansulfonyl-phenyl)-3-methyl-6,7-dihydro-5H-pyrrolizin

Man tropft zu einer auf 0°C gekühlten Lösung von 6-(4-Fluorphenyl)-5-methyl-7-phenyl-2,3-dihydro-1H-pyrrolizin (2.91 g, 10 mMol) in CHCl₃ (12 mL) unter weiterer Kühlung Chlorsulfonsäure (7 g, 60 mMol) und erhitzt nach vollständiger Zugabe 3 h unter Rückfluß (66 °C). Danach wird die Reaktionsmischung auf Eiswasser (0.4 kg) gegossen und das sich abscheidende Sulfonsäurechlorid in Chloroform (150 mL) aufgenommen. Die Eiswasserphase wird mit Chloroform (100 mL) extrahiert. Diese CHCl₃-Extrakte werden mit Na₂SO₄ sicc. getrocknet und eingeengt. Der Rückstand wird mit einer wäßrigen Lösung (25 mL) von Na₂SO₃ (1.42 g, 11.2 mMol) und NaHCO₃ (1.89 g, 22.5 mMol) versetzt und 2 h auf 70 °C (IT) erwärmt. Nach Zugabe von Ethanol (25 mL) und Methyljodid (1.0 mL, 2.27 g, 16 mMol) wird die Temperatur auf 100 °C (IT) erhöht und weitere 2 h gehalten (Rückfluss). Nach dem Abkühlen des Ansatzes wird ausgeschiedener Feststoff abgesaugt, mit Wasser (10 mL) gewaschen und im Vakuum über P₂O₅ getrocknet: es ergeben sich 0.4 g (11 %) des Produktes mit einem Schmp. von 191 °C (97% Reinheit). Die wäßrig-ethanolische Phase wird mit Ethylacetat (50mL) extrahiert und nach dem Trocknen mit Na₂SO₄ sicc. wird das Filtrat eingeengt. Der Rückstand kristallisiert aus EtOH (5 mL): es ergeben sich 0.5 g (14 % d. Th, 94 % Reinheit.)
Y: 25 % = 0.9 g, C₂₁H₂₀FNO₂S, MW = 369.46;
Mp: 190- 192 °C;
IR (NaCl): 1/λ (cm⁻¹) = 3440, 2975, 2925, 1591, 1533, 1500, 1302, 1215, 1152, 844, 772;
¹HNMR (CDCl₃): δ (ppm) = 7.72-7.68 (AA', 2H, arom), 7.25-7.00 (m, 6H, arom), 3.97 (t, 2H, CH₂), 3.07 (t, 2H, CH₂), 3.00 (s, 3H, SO₂CH₃,), 2.65-2.5 (quin, 2H, CH₂), 2.02 (s, 3H, CH₃).

### Beispiel 9

### 2-(4-Fluorphenyl)-3-(4-methansulfanyl-phenyl)-1-methyl-6,7-dihydro-5H-pyrrolizin

### a) 2-Ethyl-1-pyrrolin

### (analog Beispiel 1, Schritte a und b)

In einem 1L-3-Halskolben mit Tropftrichter und Rückflußkühler wird Natriumhydrid (60 % in Paraffin, 36 g, 0.9 Mol) in abs. THF (180 mL) suspendiert, und die Suspension wird 10 min. unter schwachem Reflux erwärmt. Die Mischung von Propionsäureethylester (33.7 g, 0.33 Mol) und 1-Vinyl-2-pyrrolidon (33.34 g, 0.3 Mol) in abs. THF (35 mL) werden zur siedenden Suspension getropft (10 min.) und unter Rühren durch Erwärmen 3,5 h unter Rückfluß gehalten.
Nach Abkühlung auf 10 °C (Eisbad) wird mit gesättigter Ammoniumchlorid-Lösung (300 mL) der Natriumhydridüberschuß vernichtet (Cave! H₂) und neutralisiert, und zum Austreiben des freiwerdenden Ammoniaks wird die inzwischen 30 °C warme Mischung noch 10 min. intensiv gerührt. Die abgeschiedene THF-Phase wird abgetrennt, über Na₂SO₄ sicc. getrocknet und eingeengt. Die sich auf der Ölphase abscheidende Paraffinschicht wird dekantiert. Die erhaltene rohe ölige Produktfraktion (3-Propionyl-1-vinyl-2-pyrrolidon, 51,2 g, 102 % d. Th.) wird ohne weitere Reinigung zur Herstellung des 2-Ethyl-1-pyrrolin herangezogen:
Y: 102 % = 51.2 g (ca 92 %), C₉H₁₃NO₂, MW = 167.21;
Bp: 140.20 °C (760 Torr);
IR (NaCl): 1/λ (cm⁻¹) = 2955, 2925, 2854, 698, 1633, 1456, 1427, 1387, 1327, 1273, 1114, 979;
¹HNMR (CDCl₃): δ (ppm) = 7.08 - 6.95 (CH), 4.52 - 4.42 (CH₂), 3.74 - 3.67 (CH), 3.60 - 3.41 (CH₂), 3.13 - 2.96 (CH), 2.69 - 2.51 (CH₂), 2.23 - 2.09 (CH), 1.12 - 1.04 (t, CH₃);
¹³CNMR (CDCl₃): δ (ppm) = 205.3 (C=O), 168.4 (C=O), 129.1 (C-H), 95.6 (CH₂), 55.2 (C-H), 43.1 (CH₂), 35.9 (CH₂), 19.3 (CH₂), 7.3 (CH₃).

In einem 1L-3-Halskolben mit Tropftrichter sowie Wasserabscheider mit Rückflußkühler wird HCl (20 %, 300 mL) zum schwachen Sieden erhitzt. Aus dem Tropftrichter wird eine Lösung des rohen 3-Propionyl-1-vinyl-2-pyrrolidon (40.4g, 240 mMol) in THF (60 mL) zugetropft (10 min.), und die Ansatzmischung wird bei 100 °C (IT) gehalten. Das am Wasserabscheider gesammelte Acetaldehyd/THF-Gemisch (47 mL) wird verworfen. Die Mischung wird 6 h bei dieser Temperatur gehalten, abgekühlt und mit Ether (200 mL) extrahiert. In der Kälte (5-10 °C) wird aus der HCl-sauren Wasserphase das 2-Ethyl-1-pyrrolin durch Alkalisieren auf pH 9-10 abgeschieden. Das abgeschiedene Öl wird in Diethylether (150 mL) aufgenommen, und die Wasserphase wird mit Diethylether (300 mL) extrahiert. Die Etherphasen werden vereint, getrocknet (K₂CO₃) und bei schwachem Vakuum (240 mmHg, 45 °C) eingeengt. Man erhält 18.4 g (79 %) Ethyl-1-pyrrolin als gelbgefärbtes Öl
Y: 79 % = 18.4 g (ca 94 %), C₆H₁₁N, MW = 97.16;
Bp: 109.5 (760 mmHg);
IR (NaCl): 1/λ (cm⁻¹) = 3378, 2969, 2937, 2870, 1644, 1462, 1454, 1431, 1371, 1300, 1144, 1093, 1019, 961;
¹HNMR (CDCl₃): δ (ppm) = 3.38 - 3.76 (m, CH₂), 2.52 - 2.34 (m, 2 CH₂), 1.95 -1.83 (quin, CH₂, J=7,8 Hz), 1.19 - 1.12 (t, CH₃, J=7,6Hz);
¹³CNMR (CDCl₃): δ (ppm) = 179.8, 60.5, 36.9, 26.8, 22.4, 10.6.

### b) 6-(4-Fluorphenyl)-7-methyl-2,3-dihydro-1H-pyrrolizin

In einem 500 mL-Kolben gibt man zum öligen Ethyl-1-pyrrolin (17.55 g, 180 mMol) in Anteilen 2-Brom-1-(4-fluorphenyl)-1-ethanon (19.53 g, 90 mMol), wobei man zwischen den Zugaben das exoterm reagierende Gemisch abkühlt. Die Mischung der Reaktionskomponenten wird im Ölbad (100 °C) unter Rühren erhitzt (30 min). Der Reaktionsverlauf wird dc überwacht.
Die abgekühlte Mischung wird mit CH₂Cl₂ (250 mL) versetzt, und abgeschiedene Salze werden im Scheidetrichter mit zwei Portionen HCl (3 %, 40 mL) ausgewaschen. Die CH₂Cl₂-Phase wird mit Wasser (50 mL) gewaschen, getrocknet (Na₂SO₄ sicc.) und eingeengt. Als Rückstand bleiben 14.04 g (72 % d. Th.) 6-(4-Fluorphenyl)-7-methyl-2,3-dihydro-1H-pyrrolizin als braunes zähes Öl.
Y: 72 % = 14.04 g (ca. 90 %), C₁₄H₁₄FN, MW = 215.27;
IR (NaCl): 1/λ (cm⁻¹) = 2958, 1703, 1601, 1509, 1223, 1158, 839, 755;
¹HNMR (CDCl₃): δ (ppm) = 7.39 - 7.30 (m, 2H, F-arom), 7.08 - 6.98 (m, 2H, F-arom), 6.67 (s, 1H, 5-H), 3.954 (t, 2H, CH₂, J=7 Hz), 2.801 (t, 2H, CH₂, J=7 Hz), 2.487 (quin, CH₂, J=7 Hz), 2.125 (s, 3H, CH₃);
¹³CNMR (CDCl₃): δ (ppm) = 161.04 (d, C-F, J=242 Hz), 135.58 (d, J=2.0 Hz), 133.51 (d, J=2 Hz), 128.87 (d, J = 7.5 Hz), 127.65, 115.025 (d, J = 20.9 Hz), 111.11 (Pyrrol-C-H), 106.71, 46.47, 27.44, 23.14, 10.86;
GC-MS : m/e (rel.Int.[%]) = 216 (14), 215 (M^{+•}, 100%), 214 (99), 201 (7),200 (40), 199 (7), 198 (10), 172 (10) 146 (10).

### c) 5-Brom-6-(4-fluorphenyl)-7-methyl-2,3-dihydro-1H-pyrrolizin

Die Lösung von 6-(4-Fluorphenyl)-7-methyl-2,3-dihydro-1H-pyrrolizin (9.0 g, 42 mMol) in abs. THF (50 mL) wird auf -15 °C gekühlt. In Anteilen wird N-Bromsuccinimid (3.46 g, 19,4 mMol) zugegeben; es wird gerührt, bis kein Edukt mehr nachweisbar ist (DC SiO₂, Diisopropylether-Hexan 2:1).
Die schwarz-lila gefärbte Reaktionsmischung wird über eine mit Al₂O₃ (ICI, f. Trockenbefüllung) befüllte Säule (20cmx3cm) chromatographiert. Die Säule wird mit n-Hexan (80 mL) nachgewaschen, und die Filtrate werden eingeengt. Man erhält 3.55 g (28.9 %) eines schwarz-braunen Öls, das sich rasch zersetzt.
Y: 28.9 % = 3.55 g (ca. 90 %), C₁₄H₁₄FN, MW = 215.27;
¹HNMR (CDCl₃): δ (ppm) = 7.37 - 7.29 (m, 2H, F-arom), 7.15 - 7.00 (m, 2H, F-arom), 3.923 (t, 2H, CH₂, J=7 Hz), 2.861 (t, 2H, CH₂, J=7 Hz), 2.482 (quin, CH₂, J=7 Hz), 2.006 (s, 3H, CH₃).

### d) 2-(4-Fluorphenyl)-3-(4-methansulfanyl-phenyl)-1-methyl-6,7-dihydro-5H-pyrrolizin

### (nach Suzuki et al., J. Heterocyclic Chem. 31, 1637 (1994))

5-Brom-6-(4-fluorphenyl)-7-methyl-2,3-dihydro-1H-pyrrolizin (2.66 g, 8.83 mMol) und Tetrakis-(triphenyl-phosphin)-palladium (330 mg, 0.29 mMol, 1:30) werden in Toluol (12 mL) verteilt, die Lösung von 4-Methylthiophenyl-boronsäure (1.0 g, 6 mMol) in EtOH (7.5 mL) wird zugegeben und schließlich wird Na₂CO₃-Lösung (12 mL, 2 M, 2,8 g) zugesetzt. Die Mischung der Komponenten wird in ein auf 100 °C temperiertes Ölbad getaucht und bis zum Verschwinden der Brom-Verbindung refluxiert (16 h). Die schwarzgefärbte Mischung wird mit gesättigter NaCl-Lösung (50 mL) und CH₂Cl₂ (80 mL) versetzt. Die organische Phase wird abgetrennt und im Vakuum eingeengt, und der Rückstand wird sc (Al₂O₃, Diisopropylether, n-Hexan 1:3) gereinigt. Der aus den Fraktionen 7-18 erhaltene Rückstand wird aus n-Hexan kristallisiert: 0.58 g (28 %) 2-(4-Fluorphenyl)-3-(4-methansulfanylphenyl)-1-methyl-6,7-dihydro-5H-pyrrolizin mit einem Schmelzpunkt von 113.7 °C.
Y: 28 % = 0.58 g, C₂₁H₂₀FNS, MW = 337.46;
IR (NaCl): 1/λ (cm⁻¹) = 2985, 2918, 2842, (1596) 1524, 1504, 1479, 1424, 1404, 1367, 1305, 1215, 830, 805;
¹HNMR (CDCl₃): δ (ppm) = 7.2 - 6.9 (8H, m, arom), 4.0 (2H, t, J=6.9Hz), 2.88 (2H, t, J=7.3Hz), 2.501 (2H, quin, CH₂), 2.45 (3H, s, CH₃), 2.03 (3H, s, CH₃);
¹³CNMR (CDCl₃): δ (ppm) = 161.1 (d, C-F, J=240 Hz), 137.52, 135.76, 132.59 (d, J=3.2 Hz), 131.60 (d, J = 4 Hz, CH), 129.83, 129.06 (CH), 126.32 (CH), 124.87, 114.94 (d, J = 21 Hz, CH), 108.50 (C-5), 46.32 (CH₂), 27.26 (CH₂), 23.29 (CH₂), 15.69 (SCH₃), 10.23 (7-CH₃).
GC-MS (70 eV): m/z (rel. Int. [%]) = 339 (5), 338 (27), 337 (100, M+.), 336 (25), 322 (40), 290 (10).

### e) 2-(4-Fluorphenyl)-3-(4-methansulfinyl-phenyl)-1-methyl-6,7-dihydro-5H-pyrrolizin

SC (SiO₂, EE/n-Hexan 4:1):
Fraktion 1-2:
   2-(4-Fluorphenyl)-3-(4-methansulfonyl-phenyl)-1-methyl-6,7-dihydro-5H-pyrrolizin
Fraktion 3-5
   2-(4-Fluorphenyl)-3-(4-methansulfinyl-phenyl)-1-methyl-6,7-dihydro-5H-pyrrolizin
   ¹HNMR (CDCl₃): δ (ppm) = 7.52 - 7.47 (AA', 2H, Ar.), 7.29 - 7.24 (BB', 2H, Ar.), 7.16 - 7.10 (m, 2H, F-arom), 7.09 - 6.92 (m, 2H, F-arom), 4.04 (2H, t, J=6.9Hz), 2.89 (2H, t, J=7.3Hz), 2.73 (s, 3H, SOCH₃), 2.53 (2H, quin, CH₂), 2.02 (3H, s, CH₃).

### f) 2-(4-Fluorphenyl)-3-(4-methansulfonyl-phenyl)-1-methyl-6,7-dihydro-5H-pyrrolizin

SC (SiO₂, EE/n-Hexan 4:1):
¹HNMR (CDCl₃): δ (ppm) = 7.77 - 7.72 (AA', 2H, Ar.), 7.29 - 7.24 (BB', 2H, Ar.), 7.16 - 7.10 (m, 2H, F-arom), 7.09 - 6.92 (m, 2H, F-arom), 4.06 (2H, t, J=6.9Hz), 3.05 (s, 3H, SO₂CH₃), 2.90 (2H, t, J=7.3Hz), 2.54 (2H, quin, CH₂), 2.01 (3H, s, CH₃).

### Beispiel 10

### 4-[3-(4-Fluorphenyl) -6,7-dihydro-5H-pyrrolizin-2-yl]- benzolsulfonsäureamid

### a) 1-(4-Fluorbenzoyl)-pyrrolidin-2-carbonsäure

L-Prolin (2.5 g, 22 mmol) wird in NaOH (5%, 25 mL, 31 mMol) in Lösung gebracht und nach Abkühlen tropfenweise, unter starkem Rühren, mit 4-Fluorhenzoesäurechlorid (3.17 g, 20 mMol) versetzt. Die entstehende Suspension wird nochmals mit NAOH (5%, 15 mL, 19 mmol) versetzt und 1 h bei RT gerührt. Unter Eiskühlung wird HCl (20 %ig, 10 mL) zugesetzt und bis zur Bildung eines absaugbaren Kristallisates gerührt. Das anschließend abgesaugte Kristallisat wird mit Wasser HCl-frei gewaschen und im Exsiccator über P₂O₅ getrocknet.
Nach dem Trocknen verbleiben 4,21 g (88,8 % d.Th.) Kristallisat.
Y: 89 % (4,21 g), C₁₂H₁₂FNO₃, MW = 237.23;
Mp: 174.0 °C;
IR (NaCl): 1/λ (cm⁻¹) = 1735, 1605, 1585, 1514, 1440, 1230, 1180, 1161, 856, 762, 513;
¹HNMR (CDCl₃): δ (ppm) = 7.64-7.61 (d, 2H, J=5.4Hz), 7.59-7.57 (d, 2H, J=5.4Hz), 7.16-7.07 (t, 2H, J=8.6Hz).

### b) 3-(4-Fluorphenyl)-2-phenyl-6,7-dihydro-5H-pyrrolizin-1-carbonsäure-methylester

Phenylpropiolsäure-methylester (Dehydrozimtsäuremethylester (0.96 g, 6 mMol)) wird in Acetanhydrid (14 mL) gelöst und im heißen Ölbad (140 °C) auf Siedetemperatur des Acetanhydrid gebracht. Die gesamte Menge der 1-(4-Fluorbenzoyl)-pyrrolidin-2-carbonsäure (1.42 g, 6 mMol) wird zugegeben, und die gut durchmischte Lösung wird unter Rühren 20 h unter Rückfluß (128 °C) gekocht. Nach 4-stündigem Stehen und Abkühlen auf RT setzt sich ein reiner kristalliner Festkörper ab (DC:Al₂O₃, Diisopropylether, Rf, 0.7). Die Kristalle werden abgesaugt, mit Wasser (20 mL) und schließlich mit Isopropylether (10 mL) gewaschen. Die getrockneten Kristalle wiegen 650 mg (32.5 % d. Th.)
Y: 89 % (4,21 g), C₂₁H₁₈FNO₂, MW = 335.38;
IR (NaCl): 1/λ (cm⁻¹) = 2497, 1791, 1493, 1428, 1157, 846;
¹HNMR (CDCl₃): δ (ppm) = 7.50-7.37 (m, 6H), 7.17-7.01 (m, 2H, F-arom), 3.93-3.86 (t, 2H), 3.53 (s, CH₃), 3.01-2.94 (t, 2H), 2.55-2.41 (m, 2H).

### c) 4-[3-(4-Fluorphenyl)-6,7-dihydro-5H-pyrrolizin-2-yl]-benzolsulfonsäureamid

In einem getrockneten Kolben, der mit Argon gespült und unter Feuchtigkeitsausschluß im Kältebad auf -15 °C gekühlt wurde, wird Chlorsulfonsäure (2,6 mL, 4,537 g, 39 mMol) in 2 Portionen mit 3-(4-Fluorphenyl)-2-phenyl-6,7-dihydro-5H-pyrrolizin-1-carbonsäureethylester (436 mg, 1.3 mMol) versetzt. Die Mischung wird 16 h gerührt, es wird die Kühlung entfernt und auf RT erwärmt. Die Umsetzung wird dc verfolgt (SiO₂/Isoether-EE 1:1, Edukt Rf 0.8, Sulfochlorid Rf 0.7). Chlorsulfonsäureüberschuß und entstandene H₂SO₄ werden nach Kühlung auf -15 bis -20 °C durch tropfenweise Zugabe von Wasser (20 mL) abgelöscht und die schwefelsaure Wasserphase wird mit Ethylacetat (60 mL) extrahiert. Die vereinten, getrockneten (Na₂SO₄ sicc.) EE-Phasen werden im Kältebad (-15°C) mit NH₃ gesättigt (10 min.) und noch 2 h bei RT nachgerührt (DC-Kontrolle: SiO₂/Isoether-EE 1:1, Edukt Rf 0.8, Sulfochlorid Rf 0.7, Sulfonamid Rf 0,0; RP 18 / Aceton - H₂O 7:3, Edukt Rf 0.2, Sulfochlorid Rf 0.1, Sulfonamid Rf 0,4). Der sich absetzende halbfeste Rückstand wird mit MeOH (10 mL) digeriert, die unlöslichen Ammoniumsalze werden abfiltriert und die klare Lösung wird im Vakuum eingeengt. Der erhaltene Rückstand (0.55 g) wird mit Ether digeriert, und die aus Ether im Vakuum erhaltene Festsubstanz wird aus Ethanol umkristallisiert: es verbleiben 0.45 g (97 % d. Th.).
Y: 89 % (4,21 g), C₁₉H₁₇FN₂O₂S, MW = 356.42;
Mp: 206.9 °C;
IR (NaCl): 1/λ (cm⁻¹) = 3355, 3048, 1596, 1403, 1321, 1151;
¹HNMR (CD₃OD): δ (ppm) = 7.58-7.81 (d, 1H, J=8.4Hz), 7.66-7.62 (d, 1H, J=8.5Hz), 7.59-7.56 (d, 1H, J=5.3Hz), 7.54-7.52 (d, 1H, J=5.3Hz), 7.16-7.07 (t, 2H, J=8.8Hz), 6.74 (s, 1H), 4.19-4.12 (t, 2H, J=7.1Hz), 3.18-3.10 (t, 2H, J=7.2Hz), 2.68-2.55 (m, 2H);
¹³CNMR (CD₃OD): δ (ppm) = 163.4, 158.6, 139.7, 138.4, 136.7, 128.7, 128.5, 127.1, 127.0, 126.2, 124.2, 115.4, 115.0, 114.2, 108.0, 46.1, 27.3, 25.2.

### Beispiel 11

### 3-(4-Fluorphenyl)-2-(4-methylsulfanylphenyl)-6,7-dihydro-5H-pyrrolizin-1-carbonsäureethylester

### a) 2-Brom-3-(4-methylsulfanyl-phenyl)-acrylsäure-ethylester

Zu einer Lösung von Brom-ethoxycarbonyl-triphenylphosphoranyliden (4.48 g, 10.5 mMol) in Toluol (30 mL) wird unter Lichtausschluß (Alufolienumhüllung) 4-Methylsulfanyl-benzaldehyd (1.52 g, 10 mMol), gelöst in Toluol (8 mL), rasch zugetropft. Die Mischung wird bei RT über Nacht gerührt (16 h, dc Kontrolle: SiO₂/Ether-Hexan 1:1). Toluol wird im Vakuum entfernt, der Rückstand wird mit Diethylether digeriert (50 mL), und der verbleibende Kristall-Niederschlag (Triphenylphosphinoxid) wird 2-mal mit Ether (15 mL) gewaschen. Die Etherfiltrate werden im Vakuum eingeengt und das Rohprodukt (5.7 g) säulenchromatographisch (SiO₂/Ether-Hexan 7:3) gereinigt: es ergeben sich 2.72 g
Y: 90%, C₁₂H₁₃BrO₂S MW = 301.20;
¹HNMR (CDCl₃): δ (ppm) = 8.16 (s, 1H), 7.85-7.80 u. 7.28-7.24 (AA'BB', 4H, arom), 4.40-4.29 (q, J=7.1Hz, 2H), 2.51 (s, CH₃), 1.42-1.35 (t, J=7.1Hz, CH₃).

### b) 3-(4-Fluorphenyl)-2-(4-methylsulfanylphenyl)-6,7-dihydro-5H-pyrrolizin-1-carbonsäureethylester

Eine Lösung von 2-Brom-3-(4-methylsulfanyl-phenyl)-acrylsäure-ethylester (0.75 g, 3 mMol) in Acetanhydrid (7 mL) wird mit N-4-Fluorbenzoyl-prolin (0.59 g, 2,5 mMol) versetzt, und das Reaktionsgefäß wird in ein auf 150 °C temperiertes Ölbad getaucht. Die Mischung wird unter Rühren 18 h unter Rückfluß gekocht; anschließend läßt man abkühlen. Nach ca. 48 h wird die Ansatzlösung mit Eiswasser versetzt und die essigsaure Wasserphase wird mit Ethylacetat extrahiert. Die mit Wasser gewaschene Essigesterphase wird mit Na₂SO₄ sicc. getrocknet und einrotiert. Der erhaltene Rückstand (1.0 g) wird sc gereinigt (SiO₂/Ether-Hexan 1:1): es werden zwei Produkte isoliert: Fraktion 11-13 (Rf 0.5) ergibt 20 mg, Fraktion 15-25 (Rf 0.3) 15 mg. In Fraktion 6-8 werden 2/3 (0.49 g) des eingesetzten 2-Brom-3-(4-methylsulfanyl-phenyl)-acrylsäure-ethylester zurückerhalten.

### Produkt A:

3-(4-Fluorphenyl)-2-(4-methylsulfanylphenyl)-6,7-dihydro-5H-pyrrolizin-1-carbonsäureethylester
Y: 20 mg, C₂₃H₂₂FNO₂S, MW = 395.50; ;
¹HNMR (CDCl₃): δ (ppm) =: 8.01-7.94 u. 6.98-6.89 (m, 4H, arom), 7.13 (s, 5H), 4.17-4.08 (q u. t, 4H), 4.02-3.95 (t, J=7.1Hz, 2H), 3.25-3.17 (t, J= 7.5Hz, 2H), 2.61-2.47 (quin, J=7.4Hz, 2H), 2.31 (s, CH₃), 1.24-1.16 (t, J=7.2Hz, CH₃).

### Produkt B:

3-(4-Fluorphenyl)-1-(4-methylsulfanylphenyl)-6,7-dihydro-5H-pyrrolizin-2-carbonsäureethylester
Y: 15 mg, C₂₃H₂₂FNO₂S, MW = 395.50;

### Beispiel 12

### 3-(4-Fluorphenyl)-2-(4-methylsulfonylphenyl)-6,7-dihydro-5H-pyrrolizin-1-carbonsäureethylester

### a) 4-Methylsulfonyl-benzaldehyd

Eine Lösung von 4-Methylmercaptobenzaldehyd (6,08 g, 40 mMol) in CH₂Cl₂ (40 mL) wird im Eisbad auf 5 °C gekühlt. Tropfenweise wird eine Lösung von m-Chlorperbenzoesäure (mCPBA, 19.72 g, 70 %ig, 80 mMol) in CHCl₃ (220 mL) zugegeben, wobei die Reaktionstemperatur unter 10 °C gehalten wird (1h). Die Mischung rührt man 4 h im Eisbad. Man saugt die auskristallisierte m-Chlorbenzoesäure (14 g) ab. Das Filtrat wird mit NaHCO₃-Lösung (8%ig, 50 mL) zunächst säurefrei gewaschen (m-Chlorbenzoesäure 5 g) und anschließend mit Wasser (100 mL) nachgewaschen. Die über Na₂SO₄ sicc. getrocknete CHCl₃-Phase wird im Vakuum eingeengt, bis sich eine weiße Festsubstanz abscheidet. Man sammelt den aus der abgekühlten Mutterlauge abgeschiedenen Feststoff (5.26 g).
Y: 75 % (5.26 g), C₈H₈O₃S, MW = 184.22;
Mp: 160.5 - 162.1 °C;
IR (NaCl): 1/λ (cm⁻¹) = 3000, 2925, 1702, 1294, 1148, 961, 766, 529;
¹HNMR (CDCl₃): δ (ppm) = 10.15 (s, CHO), 8.17-8.13 u. 8.11-8.07 (AA'BB', 4H, arom), 3.11 (s, CH₃);
¹³CNMR (CDCl₃): δ (ppm) = 190.6, 145.3, 139.6, 130.3, 128.2, 44.3.

### b) 2-Brom-3-(4-methylsulfonyl-phenyl)-acrylsäure-ethylester

Zu einer Lösung von Brom-ethoxycarbonyl-triphenylphosphoranyliden (8.55 g, 20 mMol) in Toluol (60 mL) wird unter Lichtausschluß die Suspension von 4-Methylsulfonyl-benzaldehyd (3.68 g, 20 mMol) in Toluol (30 mL) zugegeben. Die Mischung wird zunächst bei RT über Nacht gerührt (16 h, dc Kontrolle: SiO₂/Ether-Hexan 1:1), dann 6 h auf 60 °C erwärmt, nach Zugabe von Brom-ethoxycarbonyl-triphenylphosphoranyliden (2,0 g, 5 mMol) weitere 24 h gerührt, dies nochmals wiederholt (2,0 g, 5 mMol) und schließlich nach weiteren 24 h aufgearbeitet. Toluol wird im Vakuum entfernt, der Rückstand wird mit Diethylether (40 mL) digeriert, und der verbleibende Kristall-Niederschlag (Triphenylphosphinoxid) 2-mal mit Ether (10 mL) aufgeschlämmt und abgesaugt. Die Etherfiltrate werden im Vakuum eingeengt, und das Rohprodukt wird aus Diisopropylether (5 mL) kristallisiert: es ergeben sich 2.3 g weiße Festsubstanz, die jedoch 4-Methylsulfonyl-benzaldehyd (25%) enthalten.
Y : 25,9% (2.3 g, 75%ig), C₁₂H₁₃BrO₄S, MW = 333.20;
¹HNMR (CDCl₃): δ (ppm) = 8.24 (s, 1H), 7.99 (s, 5H), 4.44-4.33 (q, J=7.1Hz, 2H), 3.09 (s, CH₃), 1.44-1.37 (t, J=7.1Hz, CH₃).
oder: 2-Brom-3-(4-methylsulfanyl-phenyl)-acrylsäure-ethylester (Beispiel 11a, 0.9 g, 3 mMol) wird in CHCl₃ gelöst, die Lösung wird auf 5 °C gekühlt und danach tropfenweise mit einer CHCl₃-Lösung (20 mL) von mCPBA (1.65 g, 70%, 6.6 mMol) versetzt. Die Mischung wird 3 h in der Kälte und anschließend 16 h bei RT gerührt, dann wird der abgeschiedene Feststoff abgesaugt und mit CHCl₃ gewaschen (20 mL). Die CHCl₃-Filtrate werden mit NaHCO₃-Lösung (8%ig, 50 mL) zunächst säurefrei gewaschen (m-Chlorbenzoesäure 5 g) und anschließend mit Wasser (100 mL) nachgewaschen. Die über Na₂SO₄ sicc. getrocknete CHCl₃-Phase wird im Vakuum vollständig eingeengt: es verbleibt eine weiße Festsubstanz von 1.03 g (103 %), die geringe Mengen Begleitsubstanz enthält.

### c) 3-(4-Fluorphenyl)-2-(4-methylsulfanylphenyl)-6,7-dihydro-5H-pyrrolizin-1-carbonsäureethylester

Eine Lösung von 2-Brom-3-(4-methylsulfonyl-phenyl)-acrylsäure-ethylester (0.67 g, 2 mMol) in Acetanhydrid (4 mL) wird mit N-4-Fluorbenzoyl-prolin (0.47 g, 2 mMol) versetzt, und das Reaktionsgefäß wird in ein auf 150 °C temperiertes Ölbad getaucht. Die Mischung wird unter Rühren 72 h unterRückfluß gekocht; anschließend läßt man abkühlen. Die schwarz verfärbte Ansatzlösung wird im Vakuum eingeengt, der Rückstand in Ethylacetat (20 mL) aufgenommen und die essigsäurehaltige Ethylacetat-Lösung wird mit NaHCO₃-Lösung (10%ig, 20 mL) zunächst säurefrei gewaschen. Die anschließend mit Wasser (20 mL) gewaschene Essigesterphase wird mit Na₂SO₄ sicc. getrocknet und einrotiert. Der erhaltene Rückstand (1.0 g) wird sc gereinigt (Al₂O₃/Ether-THF 9:1): Fraktion 11/12 (Rf 0.75) ergibt 20 mg Produkt A. In Fraktion 2-8 (Rf 0.8) wird der eingesetzte 2-Brom-3-(4-methylsulfonyl-phenyl)-acrylsäure-ethylester (0.5 g), in Fraktion 15-31 (Rf 0.5) 170 mg des N-4-Fluorbenzoyl-prolin zurückerhalten.
Y: 20 mg, C₂₃H₂₂FNO₄S MW = 427.50;
¹HNMR (CDCl₃): δ (ppm) = 7.80-7.76 u. 7.43-7.38 (AA'BB', 4H, arom), 7.09-6.96 (m, 4H, arom), 4.23-4.12 (q, J=7.1Hz, 2H), 4.03-3.96 (t, J=7.2Hz, 2H), 3.27-3.19 (t, J=7.4Hz, 2H), 3.05 (s, CH₃), 2.63-2.49 (quin, J=7.2Hz, 2H), 1.26-1.19 (t, J=7.0Hz, CH₃).

### Beispiel 13

### {4-[3-(4-Fluorphenyl)-6,7-dihydro-5H-pyrrolizin-2-yl]-phenyl}-methyl-sulfon

### a) Methyl-[4-(2-nitrovinyl)-phenyl]-sulfon

Eine Mischung aus 4-Methylsulfonyl-benzaldehyd (1,84 g, 10 mMol), Nitromethan (1,0 mL, 1.134 g, 18,5 mMol), wasserfreies Ammoniumacetat (1.5 g, 19 mMol) und Eisessig (7,0 mL) wird für 3h bei 120 °C refluxiert. Das Gemisch wird mit Wasser (20 mL) versetzt und mit Ether (30 mL) dreimal extrahiert. Die gesammelte Etherphase wird mit NaHCO₃-Lösung (10%ig, 20 mL) zunächst säurefrei gewaschen. Die anschließend mit Wasser (20 mL) gewaschene Etherphase wird mit Na₂SO₄ sicc. getrocknet und einrotiert. Ausbeute: 54.0% (1.23 g).
oder

Eine Mischung aus 4-Methylsulfonyl-benzaldehyd (22.08 g, 120 mMol) und Nitromethan (12,5 mL, 14.2 g, 233 mMol) in MeOH (1L) wird im Eisbad auf 3°C gekühlt; dann wird Natronlauge (32%, 420 mL, 567 g, 135 g NaOH, 3.36 Mol) zugetropft, wobei nach Zugabe von 30 mL eine klare Lösung entsteht (Zeitbedarf 1 h). Die Mischung wird 2 h bei 10°C gerüht und anschließend bei 10 °C in eine vorgelegte HCl (20 %, 1080 mL, 216 g HCl, 5.9 Mol) getropft (Zeitbedarf 40 min.). Die HCl-saure Mischung wird bis zur vollständigen Ausbildung des Niederschlages (30 min) gerührt. Das Nitrostyrol-Kristallisat wird abgesaugt, mit Eiswasser bis zur neutralen Reaktion der Waschphase gewaschen und im evakuierten Exsiccator über P₂O₅ getrocknet. Die Ausbeute an Kristallisat beträgt nach Trocknen 16.03 g.
Y: 58.8% (16.03 g), C₉H₉NO₄S, MW = 227.24;
IR (NaCl): 1/λ (cm⁻¹) = 3105, 1640, 1520, 1341, 1308, 1148, 956, 773, 664, 547;
¹HNMR (CDCl₃): δ (ppm) = 8.07-8.02 u. 7.77-7.73 (AA'BB', 4H, arom), 8.035 / 7.645 (AB, J=14 Hz, 2H, CH=CH), 3.10 (s, CH₃).

### b) {4-[3-(4-Fluorphenyl)-6,7-dihydro-5H-pyrrolizin-2-yl]-phenyl}-methyl-sulfon

Eine Lösung von N-4-Fluorbenzoyl-prolin ( 11.08 g, 46.7 mMol) in Acetanhydrid (4 mL) wird im Ölbad auf 80 °C IT temperiert. Methyl-[4-(2-nitrovinyl)-phenyl]-sulfon (9.66 g, 42.5 mMol) wird zugesetzt, und das Reaktionsgefäß wird in ein auf 150 °C temperiertes Ölbad getaucht. Die Mischung wird unter Rühren 4 h unter Rückfluß gekocht; nach Abklingen der CO₂-Entwicklung läßt man abkühlen. Die schwarz verfärbte Ansatzlösung wird in Ethylacetat (200 mL) aufgenommen, und die essigsäurehaltige Ethylacetat-Lösung wird mit Na₂CO₃-Lösung (10%ig, 20 mL) säurefrei gewaschen. Die anschließend mit gesättigter NaCl-Lösung (200 mL) gewaschene Essigesterphase wird mit Na₂SO₄ sicc. getrocknet und einrotiert. Der erhaltene Rückstand (22,6 g) wird in Ethylacetat (30 mL) aufgenommen und sc gereinigt (Al₂O₃/Ether): Fraktion 4-7 (Rf 0.75) ergibt 1.45 g Produkt A, Fraktion 8/9 (Rf 0.75 + Rf 0.6) ist eine Mischfraktion und ergibt Produkt A+B. In Fraktion 10-14 (Rf 0.6) wird das Strukturisomer B erhalten (0.88 g).

Produkt A: {4-[3-(4-Fluorphenyl)-6,7-dihydro-5H-pyrrolizin-2-yl]-phenyl}-methyl-sulfon
Y: 8.7 % (1.45 g), C₂₀H₁₈FNO₂S, MW = 355.43;
Mp: 153.7 °C;
IR (NaCl): 1/λ (cm⁻¹) = 1591, 1525, 1509, 1301, 1223, 1148, 1091, 956, 843, 769, 547;
¹HNMR (CDCl₃): δ (ppm) = 7.75-7.71 u. 7.39-7.35 (AA'BB', 4H, -pyridyl), 7.28-7.21 (m, 2H, arom), 7.10-7.01 (m, 2H, arom), 6.12 (s, CH), 3.94-3.88 (t, J=6.9Hz, CH₂), 3.04 (s, CH₃), 2.99-2.92 (t, J=7.3Hz, CH₂), 2.59-2.44 (quin, J=7.2Hz, CH₂);
¹³CNMR (CD₃OD): δ (ppm) = 164.6, 159.6, 143.0, 137.7, 136.4, 131.2, 131.0, 128.8, 128.7, 128.0, 127.4, 125.1, 124.1, 116.1, 115.6, 100.2, 45.9, 44.6, 27.4, 24.5.

Produkt B: {4-[3-(4-Fluorphenyl)-6,7-dihydro-5H-pyrrolizin-1-yl]-phenyl}-methyl-sulfon
Y: 5.3 % (0.88 g), C₂₀H₁₈FNO₂S, MW = 355.43;
Mp: 176.00 - 179.00 °C,
IR (NaCl): 1/λ (cm⁻¹) = 1594, 1525, 1488, 1302, 1223, 1146, 1092, 971, 959, 836, 791, 769;
¹HNMR (CDCl₃): δ (ppm) = 7.91-7.86 u. 7.66-7.61 (AA'BB', 4H, -pyridyl), 7.48-7.41 (m, 2H, arom), 7.16-7.05 (m, 2H, arom), 6.67 (s, CH), 4.18-4.11 (t, J=7.1 Hz, CH₂), 3.20-3.13 (t, J=7.3 Hz, CH₂), 3.07 (s, CH₃), 2.72-2.57 (quin, J=7.2 Hz, CH₂);
¹³CNMR (CD₃OD): δ (ppm) = 164.1, 159.2, 142.0, 137.6, 135.7, 129.3, 129.0, 128.9, 127.9, 127.7, 127.6, 125.1, 116.0, 115.5, 114.5, 108.9, 46.6, 44.7, 27.8, 25.8.

### Beispiel 14

### 4-[2-Cyclohexyl-3-methyl-6,7-dihydro-5H-pyrrolizin-1-yl]-benzolsulfonsäureamid

### a) 1-Acetyl-cyclohexancarbonsäure-ethylester

In einem 200 mL-3-Hals-Kolben werden Acetessigsäureethylester (52.06 g, 0.40 Mol) und 1,5-Dibrompentan (92.0 g, 0.40 Mol) gemischt und im Ölbad auf 80 °C temperiert. Aus einem Tropftrichter wird eine Na-Ethanolat-Lösung (21 % 245 g, 0.75 Mol) über 45 min zugetropft, wobei die Temperatur anschließend 1 h zwischen 70 und 80 °C gehalten wird. Eine GC-Probe zeigt ein Edukt-Nebenprodukt-Produkt-Verhältnis von 1:4:13, insgesamt 18 % Anteil an dem Säurespaltungsprodukt Cyclohexancarbonsäureethylester.
Man setzt Wasser (100 mL) zu und entfernt im Vakuum ein Wasser/Ethanol-Gemisch (200 mL), kühlt auf RT ab und setzt nochmals Wasser (100 mL) zu. Man extrahiert mit Diethylether (250 mL) und trennt die Phasen. Die Wasserphase wird mit Diethylether 2-mal extrahiert (150 mL), und die mit den Nachextrakten vereinigte Etherphase wird mit ges. NaCl-Lösung gewaschen (100 mL). Die etherische Produktlösung wird über Na₂SO₄ sicc. getrocknet und im Vakuum eingeengt.
Die erhaltene Ölfraktion (92 g, 116%) enthält das gewünschte Produkt in einer Konzentration von 65 % (gc).
Die Isolierung erfolgt mit fraktionierter Desillation: die zwischen 83 - 90 °C / 0.3 bar erhaltene Fraktion (33 g) enthält die gesuchte Verbindung in 90 %iger Reinheit (Ausbeute: 38 % )
Y: 41,5 % = 33 g (90 %ig), C₁₁H₁₈O₃, MW = 198.26;
Bp: 52.00 (0.075 mmHg);
IR (NaCl): 1/λ (cm⁻¹) = 2938, 2858, 1735, 1712, 1452, 1361, 1303, 1214, 1133;
¹HNMR (CDCl₃): δ (ppm) = 4.25-4.15 (q, CH₂, J=7.1Hz), 2.15 (s, CH₃), 2.11-1.40 (m, 10H, cyclohex.), 1.30-1.23 (t, CH₃, J=7.1Hz);
¹³CNMR (CDCl₃): δ (ppm) = 205.6, 172.2, 61.1, 30.5, 25.8, 25.2, 22.7, 14.0.

### b) 1-Bromacetyl-cyclohexancarbonsäure-ethylester

1-Acetyl-cyclohexancarbonsäure-ethylester (2.2 g, 90%ig, 10 mMol) wird in abs. CH₂Cl₂ (10 mL) aufgelöst. Dazu wird im Verlauf von 45 min. eine Lösung von Brom (1.6 g, 10 mMol) in CH₂Cl₂ (3 mL) zugetropft. Durch 3-stündiges intensives Rühren wird die Mischung von freiwerdendem HBr befreit (GC-Probe). Die Reaktionslösung wird mit 5 mL halbgesättigter NaHCO₃-Lösung neutralisiert und die, im Scheidetrichter abgetrennte, CH₂Cl₂-Phase wird über Na₂SO₄ sicc. getrocknet und im Vakuum eingeengt. Es verbleibt ein Rückstand von 3.08 g (111%), der nach GC-Analyse 78.8 % Produkt (86.8 % Ausbeute) und als Begleitsubstanz 1,5-Dibrompentan aus der vorhergehenden Umsetzung enthält.
Y: 111% = 3.1 g (78.8%ig), C₁₁H₁₇BrO₃, MW = 277.16;
Bp: 98.00 (0.0005 mmHg);
IR (NaCl): 1/λ (cm⁻¹) = 2939, 2858, 1735, 1720, 1451, 1304, 1221, 1137, 1022;
¹HNMR (CDCl₃): δ (ppm) = 4.24-4.13 (q, CH₂, J=7.2Hz), 4.11 (s, CH₂), 2.11-1.37 (m, 10H, cyclohex.), 1.28-1.21 (t, CH₃, J= 7.2Hz);
¹³CNMR (CDCl₃): δ (ppm) = 198.6, 171.5, 61.6, 60.4, 31.8, 30.9, 25.0, 22.6, 14.0.

### c) 1-(1-Phenyl-6,7-dihydro-5H-pyrrolizin-2yl)-cyclohexancarbonsäure-ethylester

Eine Mischung aus 2-Benzyl-1-pyrrolin (11.65 g, 60 mMol) und 1-Bromacetyl-cyclohexancarbonsäure-ethylester (15.2 g 78,8 %ig, 45 mMol) wird ohne Lösemittel 1,5 h auf 100 °C erhitzt; danach wird nochmals 2-Benzyl-1-pyrrolin (5.82 g, 30 mMol) zugesetzt und die Temperatur wird eine weitere Stunde bei 100 °C gehalten. Man kühlt ab und nimmt den Schmelzrückstand in CH₂Cl₂ (200 mL) auf und extrahiert zweimal mit verdünnter HCl (3%ig, 200 mL). Die HCl-saure Phase wird mit CH₂Cl₂ (100 mL) zurückgewaschen und die vereinten CH₂Cl₂ -Lösungen werden über Na₂SO₄ sicc. getrocknet und im Vakuum auf 50 mL eingeengt. Dieses Konzentrat wird auf eine kurze Säule von Al₂O₃ (neutral, für TSC, ICI) übertragen, und die Säule wird anschließend mit einem Gemisch aus CH₂Cl₂ und n-Hexan-(1:2, 250 mL) eluiert. Das Eluat wird im Vakuum bis zur Trockne eingeengt, der Rückstand enthält 12.6 g des Produktes (86.9 % der Theorie) in 90 %iger Reinheit.
Y: 87% = 12.6 (90 %ig), C₂₂H₂₇NO₂, MW = 337.47;
IR (NaCl): 1/λ (cm⁻¹) = 2935, 2857, 1720, 1450, 1298, 1214, 1129, 702;
¹HNMR (CDCl₃): δ (ppm) = 7.35-7.16 (5H, arom), 6.58 (s, 1H), 4.01-3.85 (m, 2xCH₂), 2.71-2.63 (t, CH₂), 2.49-2.32 (q, CH₂), 2.25-2.14 (CH₂), 1.69-1.22 (m), 1.19-1.12 (t, CH₃);
¹³CNMR (CDCl₃): δ (ppm) = 176.3, 138.1, 135.9, 130.6, 129.7, 127.4, 125.8, 114.8, 111.4, 60.2, 47.6, 46.7, 35.6, 27.0, 25.8, 23.8, 23.3, 14.0.

### d) 1-(3-Carboxymethyl-1-phenyl-6,7-dihydro-5H-pyrrolizin-2-yl)-cyclohexancarbonsäure

Eine Lösung von 1-(1-Phenyl-6,7-dihydro-5H-pyrrolizin-2-yl)-cyclohexancarbonsäure-ethylester (2.03 g, 5.5 mMol) in abs. THF (10 mL) wird unter Argon mit einem Tropfen Triethylamin versetzt; anschließend wird unter Kühlung in einem Eisbad eine Lösung von Oxalsäuredichlorid (1.12 g, 8.8 mMol) in THF abs. (2 mL) in kleinen Anteilen zugetropft und nach der Zugabe wird noch 30 min nachgerührt (DC: SiO₂, Ether-n-Hexan (1:1): Edukt Rf 0.8, Produkt 0.4) Ohne Isolierung des Oxalsäurechlorid-Zwischenproduktes wird anschließend Hydrazin-Hydrat (80 %ig, 3.8 mL, 60 mMol) über 30 min zugetropft. Die Reaktionsmischung wird in einem Ölbad zunächst auf eine IT von 85 °C erwärmt und gleichzeitig wird über eine Destillationsbrücke THF (11 mL) überdestilliert. Nach dem Abkühlen auf ca. 50 °C wird Diethylenglycol (7.0 mL) und in kleinen Anteilen KOH (6.3 g, 112 mMol) zugesetzt. Die Mischung wird langsam in Schritten auf 140 °C erwärmt (Ölbad 180 °C). Während anfänglich restliches THF, Wasser (3 mL) und überschüssiges Hydrazin überdestillieren, wird nach ca. 30 min eine Reaktionstemperatur von 140-150 °C erreicht, die 3 h gehalten wird.
Die Mischung wird gekühlt, bei 80 °C wird Wasser (20 mL) zugesetzt und mit HCl 20 %ig sauer eingestellt (pH 2.0, Eisbadkühlung). Der sich abscheidende hellgelbe Feststoff wird abgesaugt und mit 10 mL Eiswasser bis zur neutralen Reaktion des Waschwassers gewaschen. Nach dem Trocknen im Exsiccator (P₂O₅) verbleiben 2.14 g (87 % d. Th.) Dicarbonsäure.
Y: 87% = 2.1g (92 %ig), C₂₂H₂₅NO₄, MW = 367.45;
Mp: 147.00 - 157.00°C;
IR (NaCl): 1/λ (cm⁻¹) = 2930, 2856, 1703, 1602, 1450, 1079, 702;
¹HNMR (CDCl₃): δ (ppm) = 9.14 (COOH), 7.27-7.23 (m, arom 5H), 3.90-3.83 (t, CH₂, J=6.8Hz), 3.79 (s, CH₂), 2.64-2.57 (t, CH₂, J=7.1Hz), 2.43-2.33 (m, CH₂), 2.19- 2.12 (m, 2H), 1.62-1.10 (m, 10H, cyclohex.);
¹³CNMR (CDCl₃): δ (ppm) = 181.8, 177.5, 139.7, 134.9, 131.0, 127.7, 126.1, 124.8, 117.0, 115.3, 61.8, 49.5, 35.8, 33.4, 29.7, 26.4, 25.8, 24.2, 23.8, 19.9.

### e) 6-Cyclohexyl-5-methyl-7-phenyl-2,3-dihydro-1H-pyrrolizin

In einem 25 mL-Kölbchen wird 1-(3-Carboxymethyl-1-phenyl-6,7-dihydro-5H-pyrrolizin-2yl)-cyclohexancarbonsäure (1.5 g, 3.7 mMol) 30 min in ein Ölbad von 150 °C eingetaucht. Über einen Blasenzähler kontrolliert man die Freisetzung von CO₂. Die noch warme Schmelze wird mit Diisopropylether (15 mL) digeriert. Der unlösliche kristalline Rückstand wird abgesaugt und getrocknet. Man erhält 0.84 g (82% d. Th. ) Decarboxylierungsprodukt 6-Cyclohexyl-5-methyl-7-phenyl-2,3-dihydro-1H-pyrrolizin (DC: SiO₂, Ether-n-Hexan (1:1): Edukt Rf 0-0.2, Produkt 0.9).
Y: 82% = 0.84g (97%ig), C₂₀H₂₅N, MW = 279.43;
Mp: 107.30°C;
IR (NaCl): 1/λ (cm⁻¹) = 2926, 2849, 1602, 1446, 1423, 1378, 1301, 1087, 1071, 767, 689;
¹HNMR (CDCl₃): δ (ppm) = CDCl₃: 7.39-7.15 (m, arom 5H), 3.90-3.83 (t, CH₂, J=7.0Hz), 2.91-2.84 (t, CH₂, J=7.3Hz), 2.49-2.42 (t, CH₂, J=7.2Hz), 2.30 (s, CH₃), 1.84-1.60 u. 1.29-1.19 (m, 10H, cyclohex.);
¹³CNMR (CDCl₃): δ (ppm) = CDCl₃: 137.7, 131.4, 129.2, 128.0, 126.4, 124.8, 118.9, 114.8, 44.4, 36.0, 33.4, 27.3, 126.7, 26.3, 24.4, 22.9, 11.9.

### f) 4-(2-Cyclohexyl-3-methyl-6,7-dihydro-5H-pyrrolizin-1-yl)-benzolsulfonsäureamid

In Chlorsulfonsäure (1.0 mL, 1.745 g, 15 mMol) wird 6-Cyclohexyl-5-methyl-7-phenyl-2,3-dihydro-1Hpyrrolizin (0.56 g, 2 mMol) bei RT eingerührt. Nach 3 h Reaktionszeit (DC: SiO₂, Ether-n-Hexan (1:1): Edukt Rf 0.9, Zwischen-Produkt Rf 0.7) wird kurzzeitig (30 min) auf 80 °C erwärmt und wieder abgekühlt. CHCl₃ (15 mL) wird zugesetzt und vorsichtig Eiswasser (15 mL) eingetropft. Die Phasen werden getrennt und die wäßrige Schwefelsäurephase wird noch 5-mal mit CHCl₃ (10 mL) extrahiert. Die CHCl₃-Phase wird über Na₂SO₄ sicc. getrocknet und aus einem Gaszylinder wird über 30 min ein trockener NH₃-Strom in die Lösung geleitet (DC: SiO₂, Ether-n-Hexan (1:1): Zwischen-Produkt Rf 0.7, Endprodukt Rf 0.3). Man rührt noch weitere 60 min, filtriert und engt im Vakuum ein (Rohausbeute 210 mg, 29% d. Th., hellbeige Substanz). Der Rückstand wird in heißem EtOH (9 mL) gelöst und nach Zusatz von Wasser (4 mL) in der Kälte wieder auskristallisiert. Es kristallisieren 106 mg (14.9 %) 98.7 %iges Produkt mit einem Schmelzpunkt von 256.2°C.
Y: 15% = 0.1g (99 %ig), C₂₀H₂₆N₂O₂S, MW = 358.51
Mp: 256.20;
IR (NaCl): 1/λ (cm⁻¹) = 3343, 3232, 2926, 2849, 1595, 1334, 1161, 1094, 735, 548;
¹HNMR (CDCl₃): δ (ppm) = 7.91-7.86 u. 7.39-7.35 (AA'BB', 4H, arom), 4.78 (s, NH₂), 3.91-3.84 (t, CH₂, J=7.0Hz), 2.93-2.85 (t, CH₂, J=7.3Hz), 2.56-2.46 (m, CH₂), 2.30 (s, CH₃), 1.81-1.59 u. 1.27-1.21 (m, 10H, cyclohex.);
¹³CNMR (CDCl₃): δ (ppm) = 143.0, 137.4, 132.5, 129.2, 126.4, 119.8, 113.4, 44.6, 36.1, 33.3, 27.3, 26.7, 26.2, 24.6, 11.9.

### Beispiel 15

### 4-[2-(4-Fluorphenyl)-3-methyl-6,7-dihydro-5H-pyrrolizin-1-yl]-benzolsulfonsäureamid

Ein Kolben mit Chlorsulfonsäure (84.2 g, 0.72 Mol) wird auf 0-5 °C gekühlt. Unter einer trockenen Argon-Atmosphäre wird 2-(4-Fluorphenyl)-3-methyl-1-phenyl-6,7-dihydro-5H-pyrrolizin (Beispiel 8 d, 35 g, 0.12 Mol) in 7 Portionen innerhalb von 1.5 h untergerührt . HCl wird unverzüglich frei. Es wird noch 30 h bei RT weiter gerührt. Dann wird die Reaktionsmischung auf Eiswasser (0.4 kg) abgelöscht. Das sich abscheidende Sulfonsäurechlorid wird in CHCl₃ (150 mL) aufgenommen, und die Eiswasserphase wird mit CHCl₃ (2-mal 50 mL) extrahiert. Diese CHCl₃-Extrakte werden mit Na₂SO₄ sicc. getrocknet.
Die getrocknete CHCl₃-Lösung des Sulfonsäurechlorides wird mit trockenem CHCl₃ (CaCl₂ sicc.) aufgefüllt (800 mL) und in einen Kolben überfuhrt. In die verdünnte Lösung, die in einem Eiswasserbad auf eine Innentemperatur von 0-10°C gekühlt ist, wird aus einem Druckbehälter Ammoniak (getrocknet über NaOH) eingeleitet. Nach vollständiger Sättigung der Lösung mit NH₃ wird die sich bildende weiße Suspension bei niedriger Temperatur (5-10 °C) noch weitere 3 h gerührt. Die Mischung wird anschließend in Eiswasser (300 mL) eingegossen und gerührt. Anschließend wird die organische Phase im Scheidetrichter abgetrennt und zweimal mit Wasser (200 mL) gewaschen, über Na₂SO₄ sicc. getrocknet und nach Filtration im Vakuum eingeengt. Der Rückstand wird aus Methanol kristallisiert. Die Kristalle werden gesammelt, mit Diisopropylether gewaschen und getrocknet. Es bleiben 12.5 g (27 %) Produkt.
Y: 27 % =12.5 g (94%), C₂₀H₁₉FN₂O₂S, MW = 370.45;
Mp: 120.0 °C;
IR (NaCl): 1/λ (cm⁻¹) = 3350, 3266, 1595, 1538, 1501, 1220, 1160, 836, 604, 549;
¹HNMR (CDCl₃): δ (ppm) = 7.71-7.67 (AA',2H, arom), 7.25-6.94 (BB'+AA'BB', 6H, arom), 4.79 (s, 2H, CH₂ ), 3.99-3.92 (t, 2H, CH₂), 3.08-3.0 (t, 2H, CH₂), 2.64-2.5 (quin 2H, CH₂), 2.2 (s, 3H, CH₃).

### Beispiel 16

### 4-[3-Chlor-2-(4-fluorphenyl)-6,7-dihydro-5H-pyrrolizin-1-yl]-benzolsulfonsäureamid

### a) 5-Chlor-6-(4-fluorphenyl)-7-phenyl-2,3-dihydro-1H-pyrrolizin

Die klare Lösung von 6-(4-Fluorphenyl)-7-phenyl-2,3-dihydro-1H-pyrrolizin (Beispiel 8 d, 2.77 g, 10 mMol) in THF (20 mL) wird bei RT mit N-Chlorsuccinimid (NCS, 1.34 g, 10 mMol) versetzt. Die sich unter Selbsterwärmung sofort von gelb nach schwarz färbende Lösung wird noch 10 min gerührt, dann auf eine Al₂O₃-Säule (100 g, TSC-ICI) adsorbiert, und die gesuchte Substanz wird mit Ethylacetat-n-Hexan 1:9 (Rf = 0.8, Edukt Rf = 0.6) eluiert. Die Eluatfraktionen werden im Vakuum eingeengt und der Rückstand wird aus Diisopropylether kristallisiert: 1.63 g (52%).
Y : 52 % (1.63 g), C₁₉H₁₅ClFN, MW = 311.79;
Mp: 127 °C;
IR (NaCl): 1/λ (cm⁻¹) = 1601, 1530, 1490, 1400, 1294, 1219, 836, 783, 736, 705;
¹HNMR (CDCl₃): δ (ppm) = 7.25-6.93 (m, 9H, arom), 4.01 (t, 2H, J=7.1Hz, CH₂), 3.04 (t, 2H, J=7.3Hz, CH₂), 2.54 (quin, 2H, J=7.2Hz, CH₂);
¹³CNMR (CDCl₃): δ (ppm) = 163.9, 159.0, 135.4, 133.5, 131.6, 131.4, 130.3, 130.2, 128.3, 128.2, 125.2, 121.0, 115.2, 114.7, 45.5, 26.7, 25.3.

### b) 4-[3-Chlor-2-(4-fluorphenyl)-6,7-dihydro-5H-pyrrolizin-1-yl]-benzolsulfonsäureamid

5-Chlor-6-(4-fluorphenyl)-7-phenyl-2,3-dihydro-1H-pyrrolizin (1.39 g, 4.5 mMol) wird in Portionen (5) zu der im Kolben auf 0°C gekühlten Chlorsulfonsäure (3.75 g, 32.2 mMol) gegeben und 16 h bei RT gerührt. Der schwarz gefärbte Ansatz wird mit CHCl₃ (25 mL) verdünnt und vorsichtig auf Eis (40 mL) gegossen. Im Scheidetrichter wird die CHCl₃-Phase abgetrennt; Wasserphase und Emulsionsschicht werden mit CHCl₃ zweimal extrahiert (80 mL). H₂O-Phase und Emulsionsschicht werden mit NaCl gesättigt und mit Ethylacetat extrahiert.
Der CHCl₃-Extrakt wird über Na₂SO₄ sicc. getrocknet, im Eisbad auf 2 °C gekühlt und mit NH₃ aus einem Druckbehälter gesättigt (30 min.). Die gesättigte Lösung wird 1 h gerührt, das aus der Reaktionsmischung kristallisierte Ammoniumchlorid wird abgesaugt, und das Filtrat wird eingeengt. Der Rückstand (0.52 g, 89 % Reinheit) aus Ethanol kristallisiert: 0.27 g (15%).
Y 15% (0.27 g), C₁₉H₁₆ClFN₂O₂S, MW = 390.87;
Mp: (162°C Dec;
IR (NaCl): 1/λ (cm⁻¹) = 3376, 3269, 1597, 1529, 1396, 1329, 1224, 1163, 1087, 839;
¹HNMR (CDCl₃+DMSO-d6): δ (ppm) = 7.75-7.71 u. 7.33-7.00 (m, 8H, arom), 5.30 (s, 2H, NH₂), 4.03 (t, 2H, J=7.1Hz, CH₂), 3.05 (t, 2H, J=7.3Hz, CH₂), 2.58 (quin, 2H, J=7.1Hz, CH₂);
¹³CNMR (CDCl₃/DMSO-d6): δ (ppm) = 163.8, 158.9, 139.7, 138.9, 134.5, 131.4, 131.3, 129.7, 129.6, 127.9, 126.0, 120.9, 115.3, 114.8, 113.4, 45.5, 39.4, 26.4, 25.4.

### Beispiel 17

### 4-[2-(4-Chlorphenyl)-3-methyl-6,7-dihydro-5H-pyrrolizin-1-yl]-benzolsulfonsäureamid

### a) 6-(4-Chlorphenyl)-7-phenyl-2,3-dihydro-1H-pyrrolizin

### (nach Laufer et al. J. Med. Chem. 1994, 37, 1894-1897)

2-Benzyl-1-pyrrolin (Beispiel 1 a, 19,8 g, 80 %ig, 0.1 Mol), gelöst in MeOH (300 mL) wird mit 4-Chlorphenacyl-bromid (23,4 g, 0.1 Mol) und anschließend mit NaHCO₃ (10 g, 0.12 Mol) versetzt und unter Lichtausschluß 16 h bei RT gerührt. Ausgefallenes Kristallisat wird abgesaugt und mit MeOH gewaschen. Man erhält nach dem Trocknen 16.4 g (56 %) Produkt.
Y: 56 % (16.4 g), C₁₉H₁₆ClN, MW = 293.80;
Mp: 142.0°C; C₁₉H₁₆ClN, MW = 293.80;
IR (NaCl): 1/λ (cm⁻¹) = 3447, 2983, 2879, 1600, 1524, 1485, 1401, 1187, 1088, 1010, 837, 765, 701;
¹HNMR (CDCl₃): δ (ppm) = 7.26-7.13 (m, 9H, arom), 6.748 (s, 1H, -CH=C), 4.026 (t, sH, J=7.2Hz, CH₂ ), 2.980 (t, 2H, J=7.4Hz, CH₂), 2.536 (quin, 2H, CH₂, J=7.2Hz).

### b) 2-[2-(4-Chlorphenyl)-1-phenyl-6,7-dihydro-5H-pyrrolizin-3-yl]-essigsäure nach Laufer et al. J. Med. Chem. 1994, 37, 1894-1897

Oxalsäuredichlorid (2.8 g, 0,0225 Mol) wird zur eiskalten Lösung von 6-(4-Chlorphenyl) -7-phenyl-2,3-dihydro-1H-pyrrolizin (s.o. 4.35 g, 0.015 Mol) in THF (50 mL) bei 0-10 °C zugetropft. Nach vollständiger Zugabe wird 15 min. gerührt und anschließend vorsichtig mit Eiswasser (30 mL) der Überschuß an Säurechlorid zersetzt (Gas- und Schaumbildung!). Hydrazin (9.5 mL, 80 %, 0.15 Mol) wird zugetropft, es wird 30 min gerührt und das Diethylenglykol (18 mL) werden zugegossen.
Bei einer Badtemperatur von 100 - 110 °C werden unter Atmosphärendruck THF und Wasser abdestilliert (ab 105 °C Schaumbildung!). Man kühlt auf 60 °C ab und gibt Kaliumhydroxid-Lösung (50 % in Wasser, 15.7 g KOH 85%, 0.238 Mol) in 5 Portionen zu, steigert die Temperatur in Schritten auf 140 °C (Schaumbildung!) und treibt Wasser und restliche Lösemittel ab. Man hält diese Temperatur im Sumpf, bis die Gasentwicklung nachläßt (1 h) und die Farbe des Ansatzes sich aufhellt. Der auf 60 °C gekühlte Ansatz wird auf Eis (300 g) gegossen und unter Eiskühlung mit konz. HCl auf pH 2-3 angesäuert (Temp. < 10 °C).
Das sich abscheidende Produkt wird mit Ethylacetat (150 mL) extrahiert, mit Wasser neutral gewaschen, mit Na₂SO₄ sicc. getrocknet und bis zur beginnenden Kristallisation in der Wärme bei 45 °C eingeengt. Die bei 5°C abgeschiedene Kristallmasse wird abgesaugt und mit kaltem Ethylacetat gewaschen und getrocknet.
Y: 46 % = 2.4 g (%), C₂₁H₁₈ClNO₂, MW = 351.84;
Mp: 170 °C;
IR (NaCl): 1/λ (cm⁻¹) = 2950, 1710, 1596, 1418, 1232, 830, 705;
¹HNMR (CDCl₃): δ (ppm) = 7.27-7.05 (m, 9H, arom), 6.74 (s, 1H, -CH=C), 4.06-3.99 (t, 2H, J= 7.0 Hz, CH₂ ), 3.60 (s, 2H, CH₂), 3.03 (t, 2H, J= 7.0 Hz, CH₂ ), 2.61 (quin, 2H, J= 7.0 Hz, CH₂).

### c) 6-(4-Chlorphenyl)-5-methyl-7-phenyl-2,3-dihydro-1H-pyrrolizin

Ein Kolben, gefüllt mit kristalliner Festsubstanz der 2-[2-(4-Chlorphenyl)-1-phenyl-6,7-dihydro-5Hpyrrolizin-3-yl]-2-essigsäure (1.8 g, 0.005 Mol), wird in einem Ölbad auf 180 °C erhitzt bis die, durch einen Gasblasenzähler kontrollierte, CO₂-Freisetzung abklingt (10 - 15 min). Der auf 50 °C gekühlte Rückstand wird mit Diisopropylether (50 mL) bis zum Abkühlen auf Raumtemperatur gerührt, anschließend werden die ungelösten Kristalle abgesaugt und das Rohprodukt bei Bedarf nochmals aus Diisopropylether kristallisiert.
Man erhält 1.0 g (64%) weiße, kristalline Verbindung.
Y: 64 % = 1.0 g (99%), C₂₀H₁₈ClN, MW = 307.83;
IR (NaCl): 1/λ (cm⁻¹) = 3447, 2943, 1600, 1532, 1415, 1116, 708;
¹HNMR (CDCl₃): δ (ppm) = 7.255 - 7.04 (m, 9H, arom), 3.94 (t, 2H, J=7 Hz, CH₂), 3.03 (t, 2H, CH₂), 2.54 (quin, 2H, J=7 Hz, CH₂), 2.236 (s, 3H, CH₃).

### d) 4-[2-(4-Chlorphenyl)-3-methyl-6,7-dihydro-5H-pyrrolizin-1-yl]-benzolsulfonsäureamid

6-(4-Chlorphenyl)-5-methyl-7-phenyl-2,3-dihydro-1H-pyrrolizin (0.8 g, 0.0026 Mol) wird zu der im Kolben auf 0°C gekühlten Chlorsulfonsäure (1.87 g, 0.0167 Mol) gegeben und 5 h bei RT gerührt. Mit Wasser (10 mL) wird unter Kühlung im Eisbad vorsichtig die Chlorsulfonsäure zerstört, und entstandene Schwefelsäure wird verdünnt. Mit Ethylacetat (50 mL) wird entstandenes Sulfochlorid extrahiert, der Extrakt wird über Na₂SO₄ sicc. getrocknet und eingeengt. Der nach dem Abdampfen des Lösungsmittels im Vakuum verbliebene Rückstand wird in trockenem CHCl₃ (CaCl₂) aufgenommen, und die CHCl₃-Lösung wird mit NH₃ aus einem Druckbehälter gesättigt. Die gesättigte Lösung wird 2 h gerührt, nochmals mit NH₃ gesättigt und nach weiterem 2-stündigen Rühren wird das aus der Reaktionsmischung kristallisierte Ammoniumchlorid abgesaugt. Das Filtrat wird eingeengt und der Rückstand aus Diethylether kristallisiert: 0.4 g (40% ).
Y: 40 % = 0.4 g (97%), C₂₀H₁₉ClN₂O₂S, MW = 386.90;
IR (NaCl): 1/λ (cm⁻¹) = 3336, 3260, 1596, 1433, 1426, 1315, 1160, 1118, 1093, 836, 727, 603, 548;
¹HNMR (CDCl₃): δ (ppm) = 7.72 (d, 2H, arom), 7.27-7.04 (m, 6H, arom), 3.99-3.92 (t, 2H, CH₂), 3.06-2.99 (t, 2H, CH₂), 2.64-2.54 (quin, 2H, CH₂), 2.21 (s, 3H, CH₃).

### Beispiel 18

### 2-(4-Chlorphenyl)-1-(4-sulfamoyl-phenyl)-6,7-dihydro-5H-pyrrolizin-3-carbonsäure-ethylester

### a) 2-(4-Chlorphenyl)-1-phenyl-6,7-dihydro-5H-pyrrolizin-3-carbonsäure-ethylester

6-(4-Chlorphenyl)-7-phenyl-2,3-dihydro-1H-pyrrolizin (Beispiel 17 a, 1.5 g, 5.1 mMol), gelöst in THF (10 mL) wird mit 1 Tropfen Triethylamin (ca. 50 mg) versetzt. Zu dieser Lösung wird bei RT die Lösung von Diphosgen (0.5 g, 2,5 mMol), gelöst in THF (5 mL) zugetropft; es wird 8 h gerührt. Danach wird Ethanol (abs., 3 mL) zugegeben, und es wird weitere 12 h bei RT gerührt. Das Lösemittel wird im Vakuum (45 °C) vollständig entfernt, der Rückstand mit Wasser (5 mL) ausgewaschen und schließlich aus wenig Diisopropylether umkristallisiert.
Y (nach Trocknung): 87.1% (1.62 g), C₂₂H₂₀ClNO₂, MW = 365.86;
Mp: 143.7 °C;
IR (NaCl): 1/λ (cm⁻¹) = 2985, 2969, 1694, 1545, 1473, 1415, 1385, 1311, 1221, 1126, 1097;
¹HNMR (CDCl₃): δ (ppm) = 7.28-7.17 (m, 5H, arom), 7.14-6.91 (AA'BB', 4H, arom), 4.40 (t, CH₂), 4.10 (q, OCH₂), 3.00 (t, CH₂), 2.55 (quin, CH₂), 1.15 (t, CH₃).

### b) 2-(4-Chlorphenyl)-1-(4-sulfamoyl-phenyl)-6,7-dihydro-5H-pyrrolizin-3-carbonsäure-ethylester

In die auf-10 °C gekühlte Chlorsulfonsäure (8.0 mL, 14.0 g, 120 mMol) wird in 4 Anteilen 2-(4-Chlorphenyl)-1-phenyl-6,7-dihydro-5H-pyrrolizin-3-carbonsäure-ethylester (1.6 g, 4.4 mMol) unter Schutzgas eingerührt. Nach 15 h Rühren bei RT wird mit trockenem CH₂Cl₂ (100 mL) verdünnt, und aus einem Druckbehälter wird ein Strom NH₃-Gas bis zur Sättigung eingeleitet. Die Suspension aus Ammoniumsalzen wird noch 2 h nachgerührt und dann mit Wasser (200 mL) versetzt. Mit verd. HCl (10%) wird ein schwach saurer pH-Wert eingestellt (pH 5.0), und die Phasen werden getrennt. Die Wasserphase wird mit Ethylacetat (100 mL) extrahiert. Die vereinigten organischen Phasen werden über Na₂SO₄ sicc. getrocknet, und das Solvensgemisch wird im Vakuum entfernt. Der erhaltene Rückstand wird aus Ethanol kristallisiert (1.8 g, 92.3 %).
Y: 92.3 % (1.8 g), C₂₂H₂₁ClN₂O₄S, MW = 444.94;
Mp: Dec 320°C;
IR (NaCl): 1/λ (cm⁻¹) = 3248, 2980, 1589, 1518, 1375, 1311, 1163;
¹HNMR (CD₃OD): δ (ppm) = 7.63-7.59 (AA', 2H, arom), 7.27-7.11 (BB'+AA', 4H, arom), 7.08-7.04 (BB', 2H, arom), 4.86 (s, SONH₂), 4.37 (t, 2H, CH₂), 4.08 (q, 2H, OCH₂), 3.03 (t, 2H, CH₂), 2.65 (quin, 2H, CH₂), 1.09 (t, 3H, CH₃).

### Beispiel 19

### 2-(4-Chlorphenyl)-1-(4-sulfamoyl-phenyl)-6,7-dihydro-5H-pyrrolizin-3-carbonsäure-methylester

### a) 2-(4-Chlorphenyl)-1-phenyl-6,7-dihydro-5H-pyrrolizin-3-carbonsäure-methylester

6-(4-Chlorphenyl)-7-phenyl-2,3-dihydro-1H-pyrrolizin (Beispiel 17 a, 1.5 g, 5.1 mMol), gelöst in THF (10 mL) wird mit 1 Tropfen Triethylamin (ca. 50 mg) versetzt. Zu dieser Lösung wird bei RT die Lösung von Diphosgen (0.5 g, 2,5 mMol), gelöst in THF (5 mL) zugetropft, und es wird 8 h gerührt. Danach wird Methanol (abs., 3 mL) zugegeben, und es wird weitere 12 h bei RT gerührt. Das Lösemittel wird im Vakuum (45 °C) vollständig entfernt. Der harzige Rückstand wird in CHCl₃ (20 mL) gelöst und mit Wasser (20 mL) zweimal ausgewaschen. Aus der mit Na₂SO₄ sicc. getrockneten CHCl₃-Phase wird nach dem Entfernen des Lösemittels im Vakuum ein Rückstand erhalten, der aus wenig Diisopropylether umkristallisiert wird.
Y 99.5 % (1.98 g), C₂₁H₁₈ClNO₂, MW = 351.84;
IR (NaCl): 1/λ (cm⁻¹) = 2947, 1630, 1463, 1396, 1227, 1013, 708;
¹HNMR (CDCl₃): δ (ppm) = 7.25-7.11 (m, 7H, arom), 7.01-6.91 (BB', 2H, arom), 4.39 (t, 2H, CH₂), 3.65 (s, 3H, CH₃), 3.02 (t, 2H, CH₂), 2.56 (quin, 2H, CH₂).

### b) 2-(4-Chlorphenyl)-1-(4-sulfamoyl-phenyl)-6,7-dihydro-5H-pyrrolizin-3-carbonsäure-methylester

In Dichlorethan (10 mL) wird 2-(4-Chlorphenyl)-1-phenyl-6,7-dihydro-5H-pyrrolizin-3-carbonsäuremethylester (0.87 g, 2.5 mMol) gelöst und die Lösung wird im Eisbad auf 0-5°C gekühlt. Hierzu tropft man langsam (IT < 10°C) eine Lösung von Chlorsulfonsäure (0.5 g, 5.7 mMol) in Dichlorethan (5 mL), und man rührt anschließend bei RT 12 h. Eine in der Kälte gesättigte Lösung von NH₃ in Dichlorethan wird zugegeben, und es wird weitere 12 h bei RT gerührt. Danach wird mit Wasser (20 mL) versetzt, und die organische Phase wird im Scheidetrichter abgetrennt. In Wasser unlöslicher Feststoff wird aus der Wasserphase abgesaugt und im Vakuum getrocknet. Die getrocknete Festsubstanz wird aus MeOH kristallisiert: 0.5 g (47 %); in der Kälte kristallisiert eine 2. Kristallfraktion aus der Mutterlauge: 23 % (0.25 g).
Y: 70.5 % (0.75 g) +, C₂₁H₁₉ClN₂O₄S, MW = 430.91;
IR (NaCl): 1/λ (cm⁻¹) = 3448, 3177, 1693, 1463, 1452, 1440, 1397, 1313, 1221, 1186, 1134, 1095, 1036, 1008;
¹HNMR (CD₃OD): δ (ppm) = 7.63-7.59 (AA', 2H, arom), 7.26-7.10 (AA'BB', 4H, arom), 7.06-7.02
(BB', 2H, arom), 4.87 (s, SONH₂), 4.37 (t, 2H, CH₂), 3.62 (s, 3H, COOCH₃), 3.02 (t, 2H, CH₂), 2.56 (quin, 2H, CH₂).

### Beispiel 20

### 4-[2-(4-Chlorphenyl)-6,7-dihydro-5H-pyrrolizin-1-yl]-benzolsulfonsäureamid

### a) 2-(4-Chlorphenyl)-1-phenyl-6,7-dihydro-5H-pyrrolizin-3-carbonsäure-benzylester

6-(4-Chlorphenyl)-7-phenyl-2,3-dihydro-1H-pyrrolizin (Beispiel 17 a, 1.5 g, 5.1 mMol), gelöst in THF (10 mL), wird mit Triethylamin (0.7 mL, 0.51 g, 5 mMol) versetzt. Zu dieser Lösung wird bei RT eine Lösung von Diphosgen (0.5 g, 2,5 mMol), gelöst in THF (5 mL), zugetropft, und es wird 8 h gerührt. Danach wird Benzylalkohol (abs., 0.52 mL, 0.54 g) und Triethylamin (0.7 mL, 0.51 g, 5 mMol) zugegeben, und es wird weitere 48 h bei RT gerührt. Die Reaktionsmischung wird zwischen Wasser (30 mL) und Diethylether (60 mL) verteilt, und die Etherphase wird mit NaOH (5%, 20 mL) gewaschen. Aus der mit Na₂SO₄ sicc. getrockneten THF/Ether Phase wird nach dem Entfernen des Lösemittels im Vakuum ein öliger Rückstand erhalten.
Y: 55% (1.2 g), C₂₇H₂₂CINO₂, MW = 427.93;
IR (NaCl): 1/λ (cm⁻¹) = 2956, 1685, 1600, 1515, 1465, 1305, 1224, 1133;
¹HNMR (CDCl₃): δ (ppm) = 7.24-6.57 (m, 14H arom), 5.11 (s, CH₂), 4.443-4.36 (t, CH₂), 3.05-2.98 (t, CH₂), 2.61-2.50 (qu, CH₂).

### b) 4-[2-(4-Chlorphenyl)-6,7-dihydro-5H-pyrrolizin-1-yl]-benzolsulfonsäureamid

2-(4-Chlorphenyl)-1-phenyl-6,7-dihydro-5H-pyrrolizin-3-carbonsäure-benzylester (0.48 g, 1.1 mMol) wird in eine Vorlage von Chlorsulfonsäure (2.5 mL, 4.4 g, 38 mMol) gegeben, die auf -48 °C temperiert ist (> Erstarrungspunkt ). Nach dem Entfernen des Kältebades läßt man unter Rühren auf RT erwärmen; hier rührt man weitere 24 h. Danach wird mit einer gesättigten Lösung von NH₃ in CHCl₃ (30 mL) versetzt, und es wird wiederum 16 h gerührt. Man nimmt die ausgeschiedenen Salze in Wasser (30 mL) auf und extrahiert die Phasenmischung mit Ethylacetat (50 mL), trocknet (Na₂SO₄ sicc.) und engt auf 10% des Ausgangsvolumens ein. Aus EE kristallisieren 110 mg (27%) Festsubstanz.
C₁₉H₁₇CIN₂O₂S, MW = 372.88;
IR (NaCl): 1/λ (cm⁻¹) = 3427, 2957, 2897, 1596, 1525, 1486, 1393, 1219, 1186, 1128, 1011, 835, 635;
¹HNMR (CDCl₃): δ (ppm) = 7.7 - 7.66 (AA', 2H, arom), 7.20-7.10 (m, 7H, arom), 4.89 (s, 2H, SONH₂), 4.01 (t, 2H, CH₂), 2.935 (t, 2H, CH₂), 2.54 (quin, 2H, CH₂).

### Beispiel 21

### 4-[1-(4-Chlorphenyl)-3-methyl-6,7-dihydro-5H-pyrrolizin-2-yl]-benzolsulfonsäureamid

### a) 2-(4-Chlorbenzyl)-1-pyrrolin

In Diethylether abs. (10 mL) werden Magnesiumspähne (4.04 g, 168 mmol) vorgelegt, und mit einem Jodkristall und 4-Chlorbenzylchlorid (2.2 mL, 2.7 g, 16.8 mMol) wird die Bildung des Grignard-Reagenz gestartet. Man tropft die übrige Menge an 4-Chlorbenzylchlorid (19 mL, 24 g, 148.7 mMol), gelöst in Diethylether abs. (150 mL), so zu (45 min), daß die Mischung lebhaft siedet. Nach vollständiger Zugabe wird durch Erwärmen noch weitere 1.5 h unter Rückfluß gekocht. In die so bereitete Lösung des 4-Chlorbenzyl-Grignard-Reagenzes tropft man die Lösung von 4-Chlorbuttersäurenitril (16.6 g, 160 mMol) in Diethylether abs.(150 mL) (45 min), rührt nach der Zugabe noch weitere 45 min, setzt Toluol abs. (200 mL) zu und destilliert über eine Brücke Diethylether ab, bis im Sumpf eine Temperatur von 95 °C erreicht wird (2.5 h). Man kühlt im Eisbad ab und gibt bei 25 -30 °C verd. HCl (10%) zu, bis ein pH von 2-3 in der sich absetzenden Wasserphase erreicht ist. Die Toluol/Ether-phase wird abgetrennt und 3-mal mit HCl (10%, 150 mL) extrahiert. Die gesammelte HCl-Phase wird nochmals mit Toluol (100 mL) gewaschen und anschließend mit NaOH (32%) alkalisiert (pH 9-10). Die sich abscheidende ölige Pyrrolinphase wird in Diethylether (300 mL) aufgenommen, über K₂CO₃ getrocknet und eingeengt. Die konzentrierte Etherphase (auf 50%-55% Produktgehalt) wird ohne weiter Reinigung verwendet.
Y: 14.1 g (21.6 %);
¹HNMR (CDCl₃): δ (ppm) = 7.25-7.09 (AA'BB', 4H, arom), (s, 2H, CH₂), 4.035 (t, CH₂), 2.965 (t, CH₂), 2.545 (quin, CH₂).

### b) 7-(4-Chlorphenyl)-6-phenyl-2,3-dihydro-1H-pyrrolizin

2-(4-Chlorbenzyl)-1-pyrrolin (9 g, 50 %ig, 25 mMol), gelöst in MeOH (75 mL) wird mit Phenacylbromid (4.97 g, 25 mMol) und anschließend mit NaHCO₃ (2.5 g, 30 mMol) versetzt und unter Lichtausschluß 16 h bei RT gerührt. Ausgefallenes Kristallisat wird abgesaugt und mit MeOH gewaschen. Man erhält nach dem Trocknen 3.3 g (45 %) Produkt.
Y: 45 % (3.3 g), C₁₉H₁₆ClN, MW = 293.80;
IR (NaCl): 1/λ (cm⁻¹) = 3029, 2970, 2894, 1598, 1524, 1486, 1443, 1392, 1295, 1189, 1090;
¹HNMR (CDCl₃): δ (ppm) = 7.25-7.09 (m, 9H, arom), 6.75 (s, 1H), 4.035 (t, CH₂), 2.965 (t, CH₂), 2.545 (quin, CH₂).

### c) 2-[1-(4-Chlorphenyl)-2-phenyl-6,7-dihydro-5H-pyrrolizin-3-yl]-essigsäure

Oxalsäuredichlorid (5.6 g, 0,044 Mol) wird zur eiskalten Lösung von 7-(4-Chlorphenyl) -6-phenyl-2,3-dihydro-1H-pyrrolizin (9.2 g, 0.031 Mol) in THF (100 mL) bei 0-10 °C zugetropft. Nach vollständiger Zugabe wird 15 min gerührt und anschließend vorsichtig mit Eiswasser (12 mL) der Überschuß an Säurechlorid zersetzt (Gas- und Schaumbildung!). Hydrazin (18 mL, 80 %, ca. 0.3 Mol) wird zugetropft, es wird 30 min gerührt und Diethylenglykol (36 mL) zugegossen.
Bei einer Badtemperatur von 100 - 110 °C, wird unter Atmosphärendruck THF und Wasser abdestilliert (an 105 °C Schaumbildung!). Man kühlt auf 60 °C ab und gibt Kaliumhydroxid (15.7 g KOH 85%, 0.238 Mol) in Portionen zu und steigert die Temperatur im Sumpf auf 140 °C (Schaumbildung!), Wasser, Hydrazin und restliche Lösemittel werden abdestilliert. Man hält diese Temperatur im Sumpf 1 h und läßt abkühlen. Der auf 60 °C gekühlte Ansatz wird mit Wasser (200 mL) versetzt und im Eisbad mit konz. HCl auf pH 2-3 angesäuert (Temp. < 10 °C).
Die sich abscheidende Carbonsäure wird mit Ethylacetat (300 mL) extrahiert, der EE-Extrakt mit Wasser neutral gewaschen, mit Na₂SO₄ sicc. getrocknet und bis zur beginnenden Kristallisation in der Wärme (45 °C) eingeengt und kaltgestellt. Die bei 5°C abgeschiedene Kristallmasse wird abgesaugt und mit kaltem Ethylacetat gewaschen und getrocknet.
Y: 6 g (55 %), C₂₁H₁₈ClNO₂, MW = 351.84;
IR (NaCl): 1/λ (cm⁻¹) = 3434, 2959, 2903, 1703, 1531, 1489, 1425, 1303, 1091, 834, 702, 515;
¹HNMR (CDCl₃): δ (ppm) = 7.33-6.96 (m, 9H, arom), 4.02 (t, 2H, CH₂), 3.62 (s, 2H, CH₂ ), 3.02 (t, 2H, CH₂), 2.56 (quin, 2H, CH₂).

### d) 7-(4-Chlorphenyl)-5-methyl-6-phenyl-2,3-dihydro-1H-pyrrolizin

Ein Kolben, gefüllt mit kristalliner Festsubstanz der 2-[2-(4-Chlorphenyl)-1-phenyl-6,7-dihydro-5Hpyrrolizin-3-yl]-2-essigsäure (1.5 g, 4.3 mMol), wird in einem Ölbad auf 180 °C erhitzt bis die, durch einen Gasblasenzähler kontrollierte, CO₂-Freisetzung abklingt (10 - 15 min). Der auf 50 °C gekühlte Rückstand wird mit Diisopropylether (50 mL) bis zum Abkühlen auf Raumtemperatur gerührt, anschließend werden die ungelösten Kristalle abgesaugt und das Rohprodukt wird aus Diisopropylether kristallisiert.
Man erhält 1.1 g cremefarbene, kristalline Verbindung.
Y: 73 % (1.1 g), C₂₀H₁₈ClN, MW = 307.83;
Mp.: 120.0 °C;
IR (KBr): 1/λ (cm⁻¹) = 3050, 2970, 2870, 1606, 1535, 1487, 1424, 1088, 1010, 829, 766, 701;
¹HNMR (CDCl₃): δ (ppm) = 7.3-6.98 (m, 9H, arom), 3.94 (t, 2H, CH₂), 3.02 (t, 2H, CH₂), 2.54 (quin, 2H, CH₂), 2.24 (s, 3H, CH₃).

### e) 4-[1-(4-Chlorphenyl)-3-methyl-6,7-dihydro-5H-pyrrolizin-2-yl]-benzolsulfonsäureamid

7-(4-Chlorphenyl)-5-methyl-6-phenyl-2,3-dihydro-1H-pyrrolizin (5.4 g, 0.0175 Mol) wird zu der im Kolben auf 0°C gekühlten Chlorsulfonsäure (13 g, 0.112 Mol) portionsweise (0.5 g) gegeben, und es wird 18 h bei RT gerührt. Mit Wasser (10 mL) wird unter Kühlung im Eisbad vorsichtig die Chlorsulfonsäure zerstört und entstandene Schwefelsäure verdünnt. Mit Ethylacetat (50 mL) wird entstandenes Sulfochlorid extrahiert, der Extrakt wird über Na₂SO₄ sicc. getrocknet und eingeengt. Der nach dem Abdampfen des Lösungsmittels im Vakuum verbliebene Rückstand wird mit einer Lösung von NH₃ in CHCl₃ (150 mL) versetzt, die durch Einleiten von NH₃ bei 0°C bis zur Sättigung erhalten wurde. Die Mischung wird 1 h gerührt, daraufhin werden die aus der Reaktionsmischung kristallisierten Ammoniumsalze abgesaugt. Das Filtrat wird eingeengt und der Rückstand (5,4 g) sc gereinigt (SiO₂/EE-n-Hexan 6:4). Aus den Produktfraktionen erhält man nach dem Entfernen des Lösungsmittels 1.0 g (15% ) der Reinsubstanz.
Y: 15 % = 1.0 g (%), C₂₀H₁₉ClN₂O₂S, MW = 386.90;
IR (KBr): 1/λ (cm⁻¹) = 3335, 3255, 1595, 1489, 1340, 1164, 835, 749, 547;
¹HNMR (CDCl₃): δ (ppm) = 7.83-7.79 (d, 2H, arom ), 7.28-6.95 (m, 6H, atom), 4.77 (s, 2H, NH₂ ), 3.99-3.92 (t, 2H, CH₂ ), 3.03-2.96 (t, 2H, CH₂ ), 2.63-2.49 (quin, 2H, CH₂), 2.26 (s, 3H, CH₃).

### Beispiel 22

### 1-(4-Chlorphenyl)-2-(4-sulfamoyl-phenyl)-6,7-dihydro-5H-pyrrolizin-3-carbonsäure-ethylester

### a) 1-(4-Chlorphenyl)-2-phenyl-6,7-dihydro-5H-pyrrolizin-3-carbonsäure-ethylester

7-(4-Chlorphenyl)-6-phenyl-2,3-dihydro-1H-pyrralizin (Beispiel 21 b, 1.5 g, 5.1 mMol), gelöst in THF (10 mL), wird mit Triethylamin (0.7 mL, 0.51 g, 5 mMol) versetzt. Zu dieser Lösung wird bei RT die Lösung von Diphosgen (0.5 g, 2,5 mMol), gelöst in THF (5 mL), zugetropft, es wird 12 h gerührt, Ethanol (abs., 3 mL, 2.37 g, 51 mMol) und Triethylamin (0.7 mL, 0.51 g, 5 mMol) werden zugegeben, und es wird weitere 72 h bei RT gerührt. Die Reaktionsmischung wird im Vakuum eingeengt, der Rückstand wird aus EtOH kristallisiert und getrocknet.
Y: 81.5% (1.52 g), C₂₂H₂₀ClNO₂, MW = 365;
IR (NaCl): 1/λ (cm⁻¹) = 2985, 2969, 1694, 1545, 1473, 1415, 1385, 1311, 1221, 1126, 1097;
¹HNMR (CDCl₃): δ (ppm) = 7.28-7.17 (m, 5H, arom), 7.14-6.91 (AA'BB', 4H, arom), 4.40 (t, 2H, CH₂), 4.10 (q., OCH₂), 3.01 (t, 2H, CH₂), 2.56 (quin, 2H, CH₂), 1.055 (t, 3H, CH₃).

### b) 1-(4-Chlorphenyl)-2-(4-sulfamoyl-phenyl)-6,7-dihydro-5H-pyrrolizin-3-carbonsäure-ethylester

1-(4-Chlorphenyl)-2-phenyl-6,7-dihydro-5H-pyrrolizin-3-carbonsäure-ethylester (1.6 g, 4.4 mmol) werden in Chlorsulfonsäure (8 mL, 14.2 g, 120 mMol), die zuvor auf -10 °C temperiert wurde, suspendiert und die Mischung wird 16 h bei RT gerührt; danach wird kurz erwärmt (30 min, 60 °C) und anschließend wieder abgekühlt. Die Mischung wird mit Eis (20 mL) versetzt und mit CHCl₃ (20 mL) extrahiert. Die über Na₂SO₄ sicc. getrocknete CHCl₃-Phase wird mit einer in der Kälte gesättigten Lösung von NH₃ in CHCl₃ (20 mL) versetzt, und es wird weitere 12 h bei RT gerührt. Danach wird der unlösliche Bodenkörper abgesaugt. Dieser Rückstand wird in 120 mL Ethanol erhitzt, und ungelöste Salze werden abgesaugt. Beim Einengen der EtOH-Lösung auf 20% des Ausgangsvolumens kristallisieren 1.5 g Produkt aus. Nach weiterem Einengen werden in der Kälte zusätzliche 170 mg erhalten.
Y: 85.3 % (1.67 g), C₂₂H₂₁ClN₂O₄S, MW = 444.94;
Mp: Dec ab 110°;
IR (NaCl): 1/λ (cm⁻¹) = 3265, 2981, 1688, 1542, 1464, 1419, 1382, 1312, 1227, 1163, 1098;
¹HNMR (CDCl₃): δ (ppm) = 7.73-7.68 (AA', 2H, arom), 7.27-7.23 (AA', 2H, arom), 7.04-7.00 (BB', 2H, arom), 6.81-677 (BB', 2H, arom), 4.31 (t, 2H, CH₂), 4.02 (q, 2H, OCH₂), 2.94-2.83 (t, 4H, CH₂ + SO₂NH₂), 2.48 (quin, 2H, CH₂), 0.99 (t, 3H, CH₃).

### Beispiel 23

### 1-(4-Chlorphenyl)-2-(4-sulfamoyl-phenyl)-6,7-dihydro-5H-pyrrolizin-3-carbonsäure-methylester

### a) 1-(4-Chlorphenyl)-2-phenyl-6,7-dihydro-5H-pyrrolizin-3-carbonsäure-methylester

7-(4-Chlorphenyl)-6-phenyl-2,3-dihydro-1H-pyrrolizin (Beispiel 21 b, 4.0 g, 13.6 mMol), gelöst in THF (25 mL) wird mit Triethylamin (2.0 mL, 1.5 g, 15 mMol) versetzt. Zu dieser Lösung wird bei RT eine Lösung von Diphosgen (1.5 g, 7,5 mMol), gelöst in THF (15 mL), zugetropft, und es wird 12 h gerührt. Danach werden Methanol (abs., 8.0 mL, 6.3g, 200 mMol) und Triethylamin (2.0 mL, 1.5 g, 15 mMol) zugegeben, und es wird weitere 48 h bei RT gerührt. Die Reaktionsmischung wird im Vakuum eingeengt, Ammoniumsalze werden in Wasser (10 mL) aufgenommen, und der verbleibende Rückstand wird abgesaugt und getrocknet. Man erhält 4.7 g (98.3 %) Rohprodukt
Y: 98.3% (4.7 g), C₂₁H₁₈ClNO₂, MW = 351.84;
Mp: 147- 149 °C;
IR (NaCl): 1/λ (cm⁻¹) = 2951, 1703, 1545, 1468, 1435, 1405, 1394, 1311, 1218, 1162, 1094, 833, 727, 696;
¹HNMR (CDCl₃): δ (ppm) = 7.25-717 (m, 5H, arom), 7.14 - 6.89 (AA'BB', 4H, arom), 4.40 (t, 2H, CH₂), 3.63 (s, 3H, COOCH₃), 3.00 (t, 2H, CH₂), 2.55 (quin, 2H, CH₂).

### b) 1-(4-Chlorphenyl)-2-(4-sulfamoyl-phenyl)-6,7-dihydro-5H-pyrrolizin-3-carbonsäure-methylester

In Dichlorethan (40 mL) wird 2-(4-Chlorphenyl)-1-phenyl-6,7-dihydro-5H-pyrrolizin-3-carbonsäuremethylester (4.0 g, 11.4 mMol) gelöst, und die Lösung wird im Eisbad auf 0-5°C gekühlt. Hierzu tropft man langsam (IT < 10°C, 15 min) eine Lösung von Chlorsulfonsäure (2.0 g, 17.3 mMol) in Dichlorethan (20 mL) und rührt anschließend bei RT 12 h. Eine in der Kälte gesättigte Lösung von NH₃ in Dichlorethan (40 mL) wird zugegeben, und es wird weitere 12 h bei RT gerührt. Danach wird mit Wasser (20 mL) versetzt (pH 9), und die organische Phase wird im Scheidetrichter abgetrennt. In Wasser unlöslicher Feststoff wird aus der Wasserphase abgesaugt und im Vakuum getrocknet. Die getrocknete Festsubstanz wird aus MeOH kristallisiert: 0.76 g (Produkt A).
Die Dichlorethanphase wird über Na₂SO₄ sicc. getrocknet und im Vakuum eingeengt, es verbleibt ein Rückstand von 1.6 g (Produkt B).

### Produkt B:

Y: 32.6 % (1.6 g), C₂₁H₁₉CIN₂O₄S, MW = 430.91;
Mp: Dec 140 °C;
IR (NaCl): 1/λ (cm⁻¹) = 3022, 2951, 2886, 1708, 1596, 1545, 1488, 1459, 1399, 1220, 1121, 1098, 833;
¹HNMR (CD₃OD): δ (ppm) = 7.29-7.17 (AA'+AA', 4H, arom), 7.14-7.10 (BB', 2H, arom), 6.94-6.89 (BB', 2H, arom), 4.39 (t, 2H, CH₂), 3.63 (s, 3H, COOCH₃), 3.00 (t, 2H, CH₂), 2.56 (quin, 2H, CH₂).

Produkt A:
ist identisch mit Produkt aus Beispiel 24

### Beispiel 24

### 4-[1-(4-Chlorphenyl)-6,7-dihydro-5H-pyrrolizin-2-yl]-benzolsulfonsäureamid

### a) 1-(4-Chlorphenyl)-2-phenyl-6,7-dihydro-5H-pyrrolizin-3-carbonsäure-tert.-butylester

7-(4-Chlorphenyl)-6-phenyl-2,3-dihydro-1H-pyrrolizin (Beispiel 21 b, 0.75 g, 2.5 mmol), gelöst in THF (10 mL), wird mit Triethylamin (0.3 mL, 0.25 g, 2.5 mMol) versetzt. Zu dieser Lösung wird bei RT eine Lösung von Diphosgen (0.25 g, 1,25 mMol), gelöst in THF (5 mL), zugetropft, und es wird 18 h gerührt. Danach werden tert.-Butylalkohol (abs., 1.0 mL, 0.786 g, 10 mMol) und Triethylamin (0.7 mL, 0,51 g, 5 mMol) zugegeben, und es wird weitere 48 h bei RT gerührt. Die Reaktionsmischung wird zwischen Wasser (30 mL) und CHCl₃ (60 mL) verteilt, und die Etherphase wird mit Na₂CO₃-Lösung (5%, 20 mL) gewaschen. Aus der mit Na₂SO₄ sicc. getrockneten THF/CHCl₃-Phase wird nach Entfernen des Lösemittels im Vakuum ein öliger Rückstand (0.7 g) erhalten, der dc (SiO₂, Hexan/CH₂Cl₂ 1:1) neben Produkt (Rf 0.3) noch Edukt (Rf 0.75) enthält. Der Rückstand wird sc gereinigt (SiO₂, Hexan/CH₂Cl₂ 1:1); man erhält 190 mg des reinen Esters.
Y: 20 % (0.19 g), C₂₄H₂₄ClNO₂, MW = 393.92;
¹HNMR (CDCl₃): δ (ppm) = 7.27-7.13 (m, 5H, arom), 7.12-6.89 (qu, 4H, arom), 4.42-4.35 (t, 2H, CH₂), 3.02-2.94 (t, 2H, CH₂), 2.57-2.49 (qui, 2H, CH₂).

### b) 4-[1-(4-Chlorphenyl)-6,7-dihydro-5H-pyrrolizin-2-yl]-benzolsulfonsäureamid

1-(4-Chlorphenyl)-2-phenyl-6,7-dihydro-5H-pyrrolizin-3-carbonsäure-tert.-butylester (190 mg, 0.5 mMol) werden in CHCl₃ (10 mL) aufgenommen, die Lösung wird im Eisbad auf 0-5°C gekühlt, und man tropft dann langsam (IT < 10°C, 15 min.) eine Lösung von Chlorsulfonsäure (1 mL, 1.75 g, 15 mMol) in CHCl₃ (20 mL) zu. Man rührt anschließend bei RT 12 h, fügt eine in der Kälte gesättigte Lösung von NH₃ in Dichlorethan (40 mL) zu und rührt weitere 12 h bei RT. Danach wird mit Eiswasser (5 mL) versetzt, und man saugt das Phasengemisch durch eine G3-Fritte. Man trennt die CHCl₃-Phase im Scheidetrichter von der nun klaren Wasserphase ab. Der Filterrückstand wird mit wenig Wasser und CHCl₃ gewaschen und im Vakuum getrocknet. Die getrocknete Festsubstanz wird aus MeOH kristallisiert: 0.13 g (70 %).
Y 70 % (130 mg), C₁₉H₁₇ClN₂O₂S, MW = 372.88;
IR (NaCl): 1/λ (cm⁻¹) = 3432, 3178, 3055, 1596, 1547, 1489, 1445, 1426, 1384, 1288, 1185, 1091, 1042, 833, 731, 721, 637;
¹HNMR (DMSO-d6): δ (ppm) = 7.24-717 (AA'+AA'+CH, 5H, arom), 7.09-6.89 (BB'+BB', 4H, arom), 4.87 (s, 2H, SO₂NH₂), 4.29 (t, 2H, CH₂), 2.96 (t, 2H, CH₂), 2.52 (quin, 2H, CH₂).

Die Verbindungen der Beispiele 25 - 29 werden nach analogen Vorschriften hergestellt:
Spektroskopische Daten zu den Verbindungen 25 - 29 sind in den Figuren aufgeführt.

### Beispiel 30

### 4-(3-Methyl-6,7-dihydro-5H-pyrrolizin-1-yl)-benzolsulfonsäureamid

### a) 7-Phenyl-2,3-dihydro-1H-pyrrolizin

2-Benzyl-1-pyrrolin (Beispiel 1 b, 85 %, 74 g, 0.395 Mol), gelöst in MeOH (360 mL) wird mit NaHCO₃ (38 g, 0.45 Mol) versetzt, und die Mischung wird auf 5 °C gekühlt. Zu dieser Lösung wird bei dieser Temperatur eine Lösung von Chloracetaldehyd (45 %ig in Wasser, 68 g, 0.4 Mol), gelöst in MeOH (100 mL) zugetropft, und es wird 18 h unter Lichtausschluß gerührt.
Die Reaktionsmischung mit Bodenkörper wird zwischen halbgesättigter NaCl-Lösung (900 mL) und Ethylacetat (900 mL) verteilt, die Essigester-Phase wird mit gesättigter NaCl-Lösung (300 mL) gewaschen, mit Na₂SO₄ sicc. getrocknet, und das Lösemittel wird im Vakuum entfernt. Der Rückstand (82 g) besitzt die für die weiteren Umsetzungen notwendige Reinheit (dc SiO₂, Hexan/Diethylether 1:1, Rf 0.65).
Y: 112 % (82 g), C₁₃H₁₃N, MW = 183.26;
Mp: 60.4 °C;
IR (NaCl): 1/λ (cm⁻¹) = 2360, 1601, 1551, 1498, 1440, 1295, 1245, 1072, 958, 903, 764, 711, 690, 654, 609;
¹HNMR (CDCl₃): δ (ppm) = 7.50-7.07 (m, 5H, atom), 6.64 und 6.53 (2xCH), 4.00 (t, 2H, CH₂), 3.09 (t, 2H, CH₂), 2.62-2.52 (q, 2H, CH₂);
¹³CNMR (CDCl₃): δ (ppm) = 136.6, 134.2, 128.4, 124.9, 124.3, 115.0, 114.3, 110.0, 46.1, 27.8, 25.2.

### b) Oxo-(1-phenyl-6,7-dihydro-5H-pyrrolizin-3-yl)-essigsäure-ethylester

7-Phenyl-2,3-dihydro-1H-pyrrolizin (roh, 82 g, ca 0.4 Mol) wird in THF (400 mL) gelöst, die Lösung wird im Eisbad auf 0-5°C gekühlt; dann wird innerhalb von 30 min (IT < 10°C, 15 min.) die Lösung von Oxalsäure-ethylesterchlorid (50 mL, 61.1 g, 0.448 Mol) in THF (200 mL) zugetropft. Man rührt anschließend noch 1 h bei dieser Temperatur und 1h bei RT. Danach wird unter Eiskühlung mit Na₂CO₃-Lösung (gesättigt, 300 mL) versetzt und 3-mal extrahiert mit Ethylacetat (300 mL). Man wäscht die organische Phase im Scheidetrichter mit NaCl-Lösung (gesättigt, 300 mL) und trennt von der Wasserphase ab. Die Ethylacetat-THF-Phase wird mit Na₂SO₄ sicc. getrocknet, und das Lösemittel wird im Vakuum abgedampft. Der getrocknete Rückstand (99 g) in CH₂Cl₂ wird sc gereinigt (SiO₂, Ether):
46,4 g Reinsubstanz: (41,1 %).
Y über a und b: 41 % (46,4 g), C₁₇H₁₇NO₃, MW = 283.33;
Mp: 101.8 °C;
IR (NaCl): 1/λ (cm⁻¹) = 1734, 1630, 1450, 1428, 1217, 1148, 1046, 1006, 763, 696
¹HNMR (DMSO-d6): δ (ppm) = 7.63 (s, 1H, CH), 7.51-7.22 (m, 5H, arom), 4.46-4.35 (t, 2H, CH₃ und q, 2H, CH₂), 3.11 (t, 2H, CH₂), 2.62 (quin, 2H, CH₂), 1.43 (t, 3H, CH₃).
GC-MS: m/z (rel. Int. [%] =. 284 (06); 283 (M^{+•}, 30); 211 (20); 210 ((M-COOEt^{•})⁺;100); 167 (05); 154 (05); 153 (05); 141 (06); 127 (05); 115 (05).

### c) 1-Phenyl-6,7-dihydro-5H-pyrrolizin-3-yl-essigsäure

Oxo-(1-phenyl-6,7-dihydro-5H-pyrrolizin-3-yl)-essigsäure-ethylester (46 g, 0.163 Mol) wird in Diethylenglycol-THF (1:1, 750 mL) gelöst, Hydrazinhydrat (80 %ig, 110 mL, 113 g, 1.8 Mol) wird zugefügt, die Mischung wird im Ölbad auf 60 °C temperiert, und es wird 1,5 h bei dieser Temperatur gerürt. THF wird abdestilliert (350 mL) und die Sumpf-Temperatur wird schrittweise (60 min) auf 100 °C erhöht. Man läßt auf 60°C abkühlen, gibt KOH-Plätzchen (85%, 173 g, 2.62 Mol) zum Ansatz, erhöht die Ansatz-Temperatur auf 120 °C, rührt anschließend noch 2 h bei dieser Temperatur und kühlt dann. Wenn 50 °C erreicht sind, wird mit Eiswasser (1.2 L) versetzt und 3-mal mit Ethylacetat (900 mL) extrahiert. Man säuert die Wasserphase mit HCl (20%ig) auf pH3 an, extrahiert 3-mal mit Ethylacetat (900 mL) und wäscht die organische Phase im Scheidetrichter mit NaCl-Lösung (gesättigt, 300 mL). Die Ethylacetat-Phase wird mit Na₂SO₄ sicc. getrocknet, und das Lösemittel wird im Vakuum abgedampft. Der Rückstand kristallisiert aus Hexan-Ether 1:1: 10.7 g (27,2 %). Nach dem Einengen der Mutterlauge und Aufnahme des Rückstandes in Ethylacetat wird mit Wasser ausgewaschen, über Na₂SO₄ sicc. getrocknet und erneut eingeengt: es verbleiben weitere 11.3 g Produkt.
Y: 56 % (22 g), C₁₅H₁₅NO₂, MW = 241.29;
Mp: 108.8°C;
IR (NaCl): 1/λ (cm⁻¹) = 2891, 1708, 1604, 1419, 1398, 1296, 1235, 758, 696;
¹HNMR (DMSO-d6): δ (ppm) = 7.46-7.09 (m, 5H, arom), 6.40 (s, 1H, CH), 3.96-3.89 (t, 2H, CH₂), 3.11-3.04 (t, 2H, CH₂), 2.60-2.56 (q, 2H, CH₂);
¹³CNMR (DMSO-d6): δ (ppm) =171.9, 136.6, 133.1, 128.6, 124.1, 123.9, 120.9, 113.5, 108.6, 44.1, 32.4, 27.1, 25.4.

### c) 5-Methyl-7-phenyl-2,3-dihydro-1H-pyrrolizin

1-Phenyl-6,7-dihydro-5H-pyrrolizin-3-yl)-essigsäure (21 g, 0.087 Mol) wird unter Schutzgas in einen Kolben eingefüllt, und dieser wird in ein auf 180 °C temperiertes Ölbad getaucht. Man behandelt bei 180 °C, bis die Gasentwicklung nachläßt (45 min) und eine dc Probe (SiO₂, Ether-Hexan 1:1) vollständige Umwandlung anzeigt. Die abgekühlte Schmelze wird in der Wärme in Ethylacetat (300 mL) gelöst, 3-mal mit Na₂CO₃-Lösung (10%, 100 mL), einmal mit NaCl (50 mL) und einmal mit Wasser (50 mL) gewaschen. Die Ethylacetatphase wird über Na₂SO₄ sicc. getrocknet und eingeengt: es verbleiben 16.1 g gelb-brauner Featstoff (93.8%).
Y: 93.8 % (16.1 g), C₁₄H₁₅N, MW = 197.28;
Mp: 58.9°C;
IR (NaCl): 1/λ (cm⁻¹) = 2919, 2887, 1601, 1521, 1418, 1405, 1300, 1211, 1066, 750, 691, 653, 503; ¹HNMR (CDCl₃): δ (ppm) = 7.45-7.08 (m, 5H, arom), 6.20 (1H, CH), 3.87-3.80 (t, 2H, CH₂), 3.10-3.03 (t, 2H, CH₂), 2.58-2.51 (t, 2H, CH₂), 2.23 (s, 3H, CH₃);
¹³CNMR (CDCl₃): δ (ppm) = 136.8, 132.2, 128.4, 124.9, 124.1, 123.9, 114.4, 107.1, 44.1, 27.7, 25.7, 11.9.

### d) 4-(3-Methyl-6,7-dihydro-5H-pyrrolizin-1-yl)-benzolsulfonsäureamid

5-Methyl-7-phenyl-2,3-dihydro-1H-pyrrolizin (370 mg, 1.9 mMol) wird unter Feuchtigkeitsausschluß in Chlorsulfonsäure (1,5 mL, 2.62 g, 22.5 mMol) suspendiert, die auf -10 °C gekühlt wurde. Man rührt anschließend bei RT 72 h, versetzt, dann vorsichtig mit Eis (10 g), extrahiert mit Chloroform (3x 20 mL) und wäscht die vereinten Chloroformauszüge mit kalter NaCl-Lösung. Der gewaschene CHCl₃-Auszug wird über Na₂SO₄ sicc. getrocknet; dann wird in die filtrierte CHCl₃-Lösung des Sulfochlorids NH₃ aus einem Druckbehälter eingeleitet. Die mit NH₃ gesättigte Suspension wird noch 1h gerührt, danach mit Eiswasser (15 mL) versetzt und das Phasengemisch wird durch eine G3-Fritte abgesaugt. Man trennt die CHCl₃-Phase im Scheidetrichter von der nun klaren Wasserphase ab, extrahiert letztere noch zweimal mit CHCl₃ (150 mL), vereint die CHCl₃-Phasen, trocknet über Na₂SO₄ sicc. und konzentriert im Vakuum. Die nach dem Abziehen des Lösemittels zurückbleibende Festsubstanz (270 mg, 53%) wird aus EtOH 60 % kristallisiert: 75 mg (14,2 %).
Y: 53 % (270 mg), C₁₄H₁₆N₂O₂S, MW = 276.36;
Mp: 198- 207°C;
IR (NaCl): 1/λ (cm⁻¹) = 3348, 3253, 1596, 1528, 1430, 1327, 1302, 1151, 1095, 836, 543, 412;
¹HNMR (CDCl₃): δ (ppm) = 7.72-7.49 (AA'BB', 4H, arom), 7.20 (NH₂), 6.23 (s, 1H, CH), 3.84 (t, 2H, CH₂), 3.03 (t, 2H, CH₂), 2.49 (quin, 2H, CH₂), 2.17 (s, 3H, CH₃);
¹³CNMR (CDCl₃): δ (ppm) = 134, 127.1, 126.5, 126.1, 125, 124.2, 113, 107.1, 44.0, 27.3, 25.8, 11.6.

Die Verbindung des Beispiels 33 wird nach der für Beispiel 30 beschrieben Umsetzungsfolge aus 2-Benzyl-1-pyrrolin (Beispiel 1 b) und α-Brom-tert-Butyl-methyl-keton aufgebaut, die Einführung der 5-Methylgruppe erfolgt über Umsetzung des 6-tert-Butyl-7-phenyl-2,3-dihydro-1H-pyrrolizins in den 5-Oxoessigsäureethylester und dessen Reduktion zur Essigsäure, die bei erhöhter Temperatur decarboxyliert.
Die Verbindungen der Beispiele 31, und 32 werden analog zu der im folgenden beschriebenen Umsetzungsfolge der Methode A des Beispiels 34 aus 2-Benzyl-1-pyrrolin (Beispiel 1 b) und den Halogenketonen 2-Chlor-butanon (Beispiel 31), bzw. 2-Chlor-cyclohexanon (Beispiel 32) hergestellt. Die Substituenten in den Positionen 5 und 6 werden über ein entsprechendes Bromketon (R2-CO-CHBr-R3) eingeführt.

### Beispiel 34

### 4-(3-Trifluormethyl-6,7-dihydro-5H-pyrrolizin-1-yl)-benzolsulfonsäureamid

### a) 5-Trifluormethyl-7-phenyl-2,3-dihydro-1H-pyrrolizin

Methode A:
In einem 25 mL-Kolben werden 2-Benzyl-1-pyrrolin (Beispiel 1 b, 85 %, 4.4 g, 24 mmol) und 1-Brom-3,3,3-trifluoraceton (3.82 g, 20 mmol) miteinander gemischt (exotherm), und es wird anschließend 4 h bei 80 °C gerührt. Es bilden sich 2 isomere Produkte im Verhältnis 0.8 : 1 (DC: SiO₂ - Hexan/Ether 8:2, Rf 0.6 + Rf 0.3). Die Mischung wird mit Wasser (10 mL) versetzt und mit HCl (10 %, 5 mL) angesäuert; es mit Ethylacetat wird 3-mal extrahiert (100 mL). Die Ethylacetatphase wird über Na₂SO₄ sicc. getrocknet und eingeengt. Der Rückstand (2.0 g) wird sc (SiO₂, n-Hexan/Ether 8:2) gereinigt: TY: 1.28 g (25 %).

### Methode B:

In einem 100 mL-Kolben werden 2-Benzyl-1-pyrrolin (Beispiel 1 b, 85 %, 6.3 g, 34 mMol), gelöst in Methanol (60 mL), 1-Brom-3,3,3-trifluoraceton (3.82 g, 20 mmol) und NaHCO₃ (1.85 g, 22 mMol) miteinander gemischt, und es wird anschließend 88 h bei RT gerührt. Es bilden sich 2 isomere Produkte im Verhältnis 2:1 (DC: SiO₂ - Hexan/Ether 8:2, Rf 0.6 + Rf 0.3) . Die Mischung wird mit Wasser (50 mL) versetzt und mit HCl (10 %) angesäuert (pH 3-4); mit Diethylether wird 3-mal extrahiert (150 mL). Die Etherphase wird über Na₂SO₄ sicc. getrocknet und eingeengt. Der Rückstand (7.1 g) wird sc (SiO₂, n-Hexan/Ether 8:2) gereinigt: TY: 3.05 g (54 %).

Fraktionen 1-16: 6-Trifluormethyl-7-phenyl-2,3-dihydro-1H-pyrrolizin;
Y: 36 % (2.0 g), C₁₄H₁₂F₃N, MW = 251.25;
Mp: 67.8 °C;
IR (NaCl): 1/λ (cm⁻¹) = 3446, 2922, 2362, 1603, 1565, 1484, 1450, 1375, 1266, 1208, 1143, 1098, 758, 690;
¹HNMR (CDCl₃): δ (ppm) = 7.38-7.08 (5H, arom), 6.78 (s, 1H, CH), 4.02-3.95 (t, 2H, CH₂), 3.02-2.95 (t, 2H, CH₂), 2.59-2.48 (q, 2H, CH₂);
¹³CNMR (CDCl₃): δ (ppm) = 137, 135.2, 128.7, 125.3, 125.2, 124.2, 119, 115.6, 111.8, 46.0, 27.4, 25.2.

Fraktion 18-24: Mischfraktion.

Fraktion 26-56: 5-Trifluormethyl-7-phenyl-2,3-dihydro-1H-pyrrolizin, 520 mg;
Y: 18 % (1.0 g), C₁₄H₁₂F₃N, MW = 251.25;
Mp: 61.60°C;
IR (NaCl): 1/λ (cm⁻¹) = 2906, 2360, 1605, 1531, 1458, 1298, 1196, 1138, 1109, 1084, 971, 778, 765, 702;
¹HNMR (CDCl₃): δ (ppm) = 7.44-7.23 (5H, arom), 7.00 (s, 1H, CH), 4.03-3.96 (t, 2H, CH₂), 2.96-2.88 (t, 2H, CH₂), 2.55-2.48 (q, 2H, CH₂);
¹³CNMR (CDCl₃): δ (ppm) = 136.8, 134.6, 132.0, 128.3, 128.2, 126.6, 126.0, 121.3, 115, 46.7, 27.5, 24.3.

### b) 4-(3-Trifluormethyl-6,7-dihydro-5H-pyrrolizin-1-yl)-benzolsulfonsäureamid

5-Trifluormethyl-7-phenyl-2,3-dihydro-1H-pyrrolizin (750 mg, 3 mMol) wird unter Feuchtigkeitsausschluß in Chlorsulfonsäure (10 mL, 17,5 g, 150 mMol) suspendiert. Man rührt anschließend bei RT 16 h. Unter Eiskühlung wird vorsichtig mit Eis (10 g) versetzt, mit Chloroform (3x 30 mL) extrahiert und die vereinten Chloroformauszüge werden mit kalter NaCl-Lösung gewaschen. Der gewaschene CHCl₃-Auszug wird über Na₂SO₄ sicc. getrocknet, und dann wird die filtrierte CHCl₃-Lösung des Sulfochlorids auf 5 °C gekühlt. Aus einem Druckbehälter wird 1 h NH₃ eingeleitet. Die mit NH₃ gesättigte Suspension wird noch 2h gerührt und danach mit Eiswasser (15 mL) versetzt. Man trennt die CHCl₃-Phase im Scheidetrichter von der Wasserphase ab, extrahiert letztere noch zweimal mit CHCl₃ (150 mL), vereinigt die CHCl₃-Phasen, wäscht mit Wasser (20 mL), trocknet über Na₂SO₄ sicc. und konzentriert im Vakuum. Die nach Abziehen des Lösemittels zurückbleibende Festsubstanz (0.5 g, 50%, Gehalt 90%) wird aus Ethylacetat kristallisiert: 0.2 g (Gehalt 98 %).
Y: 45 % (0.5 g, 90 %ig), C₁₄H₁₃F₃N₂O₂S, MW = 330.33;
Mp: 153.5 °C;
IR (NaCl): 1/λ (cm⁻¹) 3388, 3282, 1594, 1536, 1296, 1198, 1149, 1112, 1091, 1079, 796, 598;
¹HNMR (CDCl₃): δ (ppm) = 7.95-7.44 (AA'BB'+ br, 6H, arom + NH₂), 7.04 (s, 1H, CH), 4.03 (t, 2H, CH₂), 2.95 (t, 2H, CH₂), 2.57 (quin, 2H, CH₂);
¹³CNMR (CDCl₃): δ (ppm) = 142.9, 135.5, 131.8, 128.7, 128.3, 126.1, 125.6, 123.5, 115.3, 46.7, 27.3, 24.1.

### Beispiel 35

### 4-(2-Isobutyl-3-methyl-6,7-dihydro-5H-pyrrolizin-1-yl)-benzolsulfonsäureamid

### a) 2-Methyl-1-(3-methyl-1-phenyl-6,7-dihydro-5H-pyrrolizin-2-yl)-propan-1-on

5-Methyl-7-phenyl-2,3-dihydro-1H-pyrrolizin (Beispiel 30 c, 7.8 g, 0.039 Mol), gelöst in Dichlorethan (140 mL) wird bei RT mit Isobuttersäurechlorid (4,6 mL, 4.8 g, 0.043 Mol) versetzt, und anschließend wird Zinntetrachlorid (5.3 mL, 11.8 g, 0.045 Mol) in Dichlorethan (30 mL) zugetropft. Die Mischung erwärmt sich auf 35 °C und wird für 2 h bei 73 °C unter Rückfluß gekocht. Danach wird auf Eis gegossen, mit NaOH (10 %, 200 mL) alkalisiert und die organische Phase im Scheidetrichter abgetrennt.

Die Alkaliphase wird noch zweimal mit Dichlormethan (400 mL) extrahiert, das zur Dichlorethan-Phase gegben wird. Dieser Extrakt wird über Na₂SO₄ sicc. getrocknet und im Vakuum zur Trockne eingedampft.
Der Rückstand (11.3 g) wird mit wenig Diisopropylether gewaschen, die Kristalle (7.56 g, 72.5 %), werden abgesaugt und getrocknet.
Y: 72.5 % (7.56 g), C₁₈H₂₁NO, MW = 267.37;
Mp: 158.7 °C;
IR (NaCl): 1/λ (cm⁻¹) = 2958, 1648, 1599, 1518, 1421, 1407, 1115, 977, 702;
¹HNMR (CDCl₃): δ (ppm) = 7.35-7.22 (m, 5H, arom), 3.91 (t, 2H, CH₂), 2.84 (t, 2H, CH₂), 2.70 (sept., 1H, CH(CH₃)₂), 2.52-2.40 (quin, 2H, CH₂), 2.39 (s, 3H, CH₃), 0.925 (d, 6H, CH(CH₃)₂);
¹³CNMR (CDCl₃): δ (ppm) = 205.2, 136.8, 133.3, 130.0, 128.9, 128.1, 125.8, 123.0, 116.0, 44.5, 38.1, 26.9, 23.8, 19.1, 12.1.

### b) 6-Isobutyl-5-methyl-7-phenyl-2,3-dihydro-1H-pyrrolizin

Eine Lösung von 2-Methyl-1-(3-methyl-1-phenyl-6,7-dihydro-5H-pyrrolizin-2-yl)-propan-1-on (7.5 g, 28 mMol) in CH₂Cl₂ (140 mL) wird nacheinander mit NaCNBH₃ (7.4 g, 112 mMol) und ZnI₂ (12.5 g, 40 mMol) versetzt und 2 h bei RT gerührt (DC: SiO₂/Diethylether; Edukt Rf 0.75; Produkt Rf 8.8). Die Mischung wird unter Eiskühlung mit H₃PO₄ (8%, 200 mL) überschichtet und 16 h gerührt. Die CH₂Cl₂-Phase wird abgetrennt und mit Wasser (50 mL) gewaschen, über Na₂SO₄ sicc. getrocknet und im Vakuum eingeengt. Der Rückstand (7.5 g, 106 %) wird aus MeOH umkristallisiert.
Y: 64.4 % (4.5 g), C₁₈H₂₃N, MW = 253.39;
Mp: 95.4 °C;
IR (NaCl): 1/λ (cm⁻¹) = 2916, 2861, 1599, 1527, 1453, 1421, 1305, 760, 698;
¹HNMR (CDCl₃): δ (ppm) = 7.35-7.23 (m, 5H, arom), 3.87 (t, 2H, CH₂), 2.94 (t, 2H, CH₂), 2.52 - 2.41 (quin + d, 4H, CH₂+ CH₂CH), 2.17 (s, 3H, CH₃), 1.62 (m., 1H, CH₂CH(CH₃)₂), 0.795 (d, 6H, CH(CH₃)₂);
¹³CNMR (CDCl₃): δ (ppm) = 137.9, 132.0, 128.1, 128.1, 124.5, 120.4, 120.4, 114.7, 44.4, 34.5, 30.3, 27.0, 24.7, 22.6, 10.4.

### c) 4-(2-Isobutyl-3-methyl-6,7-dihydro-5H-pyrrolizin-1-yl)-benzolsulfonsäureamid

6-Isobutyl-5-methyl-7-phenyl-2,3-dihydro-1H-pyrrolizin (4.0 g, 16 mMol) wird in CHCl₃ abs. (20 mL) gelöst, die Lösung wird auf 5°C gekühlt und Chlorsulfonsäure (10 mL, 17,5 g, 150 mMol) wird langsam zugetropft. Die Mischung wird 2.5 h (58 - 61 °C) erhitzt (DC: SiO₂, Ether-Hexan 1:1, Edukt Rf 0.9, Chlorsulfonsäure Rf 0.8). Die Reaktionsmiscbung wird auf Eis (100 mL) abgelöscht und in CHCl₃ (120 mL) aufgenommen. Die Eiswasserphase wird 3-mal mit CHCl₃ (120 mL) extrahiert, und die gesammelte CHCl₃-Lösung wird gewaschen (100 mL gesättigte NaCl-Lösung), getrocknet (Na₂SO₄ sicc.) und eingeengt (Rückstand: 7.2 g). Das Chlorsulfonierungsprodukt (7.2 g) wird in THF abs. gelöst, und es wird langsam konzentrierte NH₄OH (25%ig, 4 mL) zugetropft. Die Mischung wird 16 h bei RT gerührt, mit halbkonzentrierte NaCl-Lösung (60 mL) versetzt und mit Ethylacetat 3-mal extrahiert (150 mL). Die gewaschene (40 mL gesättigte NaCl-Lösung) Ethylacetatphase wird getrocknet (Na₂SO₄ sicc.) und eingeengt. Der Rückstand (5.0 g, Gehalt 83 % n. HPLC) wird mit Ether (10 mL) bei RT digeriert (2.76 g, 51 %, Gehalt 94%) und anschließend aus Ethanol (20 mL) kristallisiert und getrocknet:
Y: 34,4 %, (1.83 g, 99.7%ig), C₁₈H₂₄N₂O₂S, MW = 332.47;
Mp: 197.1 °C;
IR (NaCl): 1/λ (cm⁻¹) = 3337, 3235, 2948, 1595, 1332, 1161, 1093, 546;
¹HNMR (DMSO-d6): δ (ppm) = 7.74-7.42 (m, 5H, arom), 7.24 (NH₂), 3.83 (t, 2H, CH₂), 2.87 (t, 2H, CH₂), 2.41-2.37 (quin + d, 4H, CH₂+ CH₂CH), 2.09 (s, 3H, CH₃), 1.62 (m., 1H, CH₂CH(CH₃)₂), 0.735 (d, 6H, CH(CH₃)₂);
¹³CNMR (DMSO-d6): δ (ppm) = 141.7, 139.3, 133.1, 126.8, 125.8, 120.7, 119.1, 112.4, 44.2, 40.2, 34.3, 29.6, 26.5, 24.7, 22.4, 10.1.

Die Verbindungen der Beispiele 36 - 38 werden nach analogen Vorschriften hergestellt:
Spektroskopische Daten zu den Verbindungen 36 - 38 sind in den Figuren aufgeführt.

### Beispiel 39

### 4-[2-(Perfluorisopropyl)-3-methyl-6,7-dihydro-5H-pyrrolizin-1-yl]-benzolsulfonsäureamid

### a) 2,2,2-Trifluor-1-(3-Methyl-1-phenyl-6,7-dihydro-5H-pyrrolizin-2-yl)-ethan-1-on

5-Methyl-7-phenyl-2,3-dihydro-1H-pyrrolizin (Beispiel 30 c, 90%, 2.2 g, 0.01 Mol), gelöst in Dichlormethan (30 mL) wird bei RT mit Trifluoressigsäureanhydrid (1,4 mL, 2.14 g, 0.01 Mol) und Zinkchlorid (1,36 g, 0.01 Mol) versetzt. Die Mischung wird für 2 h bei RT gerührt, worauf eine DC-Probe (SiO₂, Hexan-Ether 1:1, Edukt Rf 0,8, Hauptprodukt Rf 0,4, Nebenprodukt Rf 0,6) vollständige Umsetzung anzeigt. Danach wird mit Wasser versetzt, mit NaOH (10 %, 200 mL) alkalisiert und noch 2-mal mit CH₂Cl₂ (50 mL) extrahiert. Die gesammelten organischen Phasen werden vereint, über Na₂SO₄ sicc. getrocknet und im Vakuum zur Trockne eingedampft.
Der Rückstand wird mittels SC (SiO₂, 200 g, Hexan-Ether 1:1) gereinigt: man erhält zunächst 0,34 g der Nebenproduktfraktion, anschließend 1,3 g (44,4 % Ausbeute) der gewünschten Verbindung.
Y: 44.4 % (1,3 g); C₁₆H₁₄F₃NO, MW = 293.29;
Mp.: 111.4 °C,
IR (NaCl): 1/λ (cm⁻¹) = 1662, 1601, 1446, 1423, 1383, 1236, 1194, 1124, 1080, 974, 759, 706, 698;
¹HNMR (CDCl₃): δ (ppm) = 7.37-7.21 (m, 5H, arom), 3.95 (t, 2H, CH₂), 2.84 (t, 2H, CH₂), 2.52 (quin, 2H, CH₂), 2.45 (s, 3H, CH₃);
GC-MS (70 eV): m/z (rel.Int. [%] = 293 (59); 224 (100); 207 (20); 225 (17); 294 (10); 127 (08); 167 (06); 194 (05).

### b 1,1,1,3,3,3-Hexafluor-2-(3-methyl-1-phenyl-6,7-dihydro-5H-pyrrolizin-2-yl)-propan-2-ol

Die Lösung von 2,2,2-Trifluor-1-(3-methyl-1-phenyl-6,7-dihydro-5H-pyrrolizin-2-yl)-ethan-1-on (1.8 g, 6 mMol) in THF (70 mL) wird nacheinander mit Trifluormethyl-trimethylsilan (7,5 mL, 2 M in THF, 15 mMol) und Tetrabutylammoniumfluorid (gebunden auf Kieselgel, 1.1 mmol/g SiO₂; 0.5 g, 0.55 mMol) versetzt und 2 h bei 40°C gerührt (DC: SiO₂/Hexan -Diethylether 1:1; Edukt Rf 0.4; Produkt Rf 8.5). Die Mischung wird im Eisbad gekühlt und mit NaOH (70 mL, 10%ige Lösung; 7 g, 175 mMol) versetzt und 1 h bei Raumtemperatur gerührt. Die THF-Phase wird mit Diethylether (50 mL) versetzt und im Scheidetrichter abgetrennt und die Wasserphase wird nochmals mit Diethylether (50 mL) extrahiert. Die vereinigten organischen Phasen werden mit Wasser (50 mL) gewaschen, über Na₂SO₄ sicc. getrocknet und im Vakuum eingeengt.
Y: 110 % (2.4 g); C₁₈H₂₃N, MW = 253.39;
Mp.: 106.1 °C
IR (NaCl): 1/λ (cm⁻¹) = 3522, 2958, 2924, 1603, 1423, 1303, 1263, 1223, 1203, 1132, 962, 948, 728, 710;
¹HNMR (CDCl₃): δ (ppm) = 7.35-7.25 (m, 5H, arom), 3.94 (t, 2H, CH₂), 3.57 (s, 1H, 0H), 2.67 (t, 2H, CH₂), 2.45 (quin + d, 4H, CH₂+ CH₂CH), 2.29 (s, 3H, CH₃);
GC-MS (EI-70 eV) m/z (rel.Int. [%] = 363 (97); 97 (100); 294 (98); 196 (24); 364 (19); 295 (18); 198 (17); 182 (13); 224 (12); 362 (06); 276 (06); 112 (06); 128 (05); 127 (05).

### c) 5-Methyl-6-perfluorisopropyl-7-phenyl-2,3-dihydro-1H-pyrrolizin

Die Lösung von 1,1,1,3,3,3-Hexafluor-2-(3-methyl-1-phenyl-6,7-dihydro-5H-pyrrolizin-2-yl)-propan-2-ol (2.2 g, 6 mMol) in CH₂Cl₂ abs. (25 mL) wird bei -50 °C langsam zu einer Lösug von Diethylaminoschwefel-trifluorid (DAST, 1,5 mL, 1,845 g, 11.5 mMol) in 25 mL CH₂Cl₂ abs. getropft. Man entfernt das Kältebad und läßt unter Rühren die Mischung innerhalb 2 h auf Raumtemperatur kommen. (DC: SiO₂/Hexan -Diethylether 1:1; Edukt Rf 0.4; Produkt Rf 8.5). Die Mischung wird im Eisbad gekühlt und mit NaHCO₃-Lösung (30 mL) alkalisiert und 10 min. gerührt. Die CH₂Cl₂-Phase wird im Scheidetrichter abgetrennt und die Wasserphase wird nochmals mit Diethylether (50 mL) extrahiert. Die vereinigten organischen Phasen werden mit ges. Kochsalzlösung. (50 mL) gewaschen, über Na₂SO₄ sicc. getrocknet und im Vakuum eingeengt. Der Rückstand wird sc gereinigt: SiO₂/Hexan -Diethylether 2:1; Edukt Rf 0.3; Produkt Rf 0.5).
Y: 69,9 % (1.53 g); C₁₇H₁₄F₇N, MW = 365.30;
Mp: 63.10 °C
IR (NaCl): 1/λ (cm⁻¹) = 2964, 2360, 1606, 1423, 1304, 1275, 1234, 1217, 1134, 966, 729, 706;
¹HNMR (CDCl₃): δ (ppm) = 7.3-7.19 (m, 5H, arom), 3.93 (t, 2H, CH₂), 2.68 (t, 2H, CH₂), 2.46 (quin + d, 4H, CH₂+ CH₂CH), 2.29 (s, 3H, CH₃);
GC-MS (EI-70 eV) m/z (rel.Int. [%] = 365 (100); 296 (57); 276 (26); 366 (19); 364 (15); 297 (11); 100(09); 268 (08); 212 (08); 196 (08); 207 (07); 199 (07); 294 (06); 227 (06); 226 (06); 277 (05); 114 (05).

### d) 4-(5-Methyl-6-perfluorisopropyl-7-phenyl-2,3-dihydro-1H-pyrrolizin -1-yl)-benzolsulfonsäureamid

5-Methyl-6-perfluorisopropyl-7-phenyl-2,3-dihydro-1H-pyrrolizin (0,7 g, 1,9 mMol) wird bei -30 °C in Chlorsulfonsäure (2.6 mL, 4.54 g, 39 mMol) eingetragen. Die Mischung wird während 2.5 h auf 0 - 10 °C erwärmt dann bei RT bis zur vollständigen Umwandlung ins Säurechlorid 42 h gerührt. Das Monitoring der Reaktion erfolgt mittels HPLC. Nach dieser Zeit liegen ca. 75 % der eingesetzten Substanz als Sulfochlorid vor neben 5 % Sulfonsäure und ca 4 % Edukt (DC: SiO₂, Ether-Hexan 1:1, Edukt Rf 0.7, Sulfonsäure Rf 0, Chlorsulfonsäure Rf 0.4). Die Reaktionsmischung wird auf Eiswasser (20 mL) abgelöscht und die Eiswasserphase 3 mal mit CHCl₃ (3x40 mL) extrahiert. Die gesammelten CHCl₃-Extrakte werden gewaschen (100 mL ges. NaCl-Lösung), getrocknet (Na₂SO₄ sicc.) und eingeengt (Rückstand: 0.94 g, 94 %, Gehalt nach HPLC: 88%). Das Chlorsulfonierungsprodukt wird in THF abs. (30 mL) gelöst und es wird unter Eiskühlung konz. NH₄OH (25%ig, 3 mL) zugegeben. Die Mischung wird 1 h bei RT gerührt, mit halbkonz. NaCl-Lösung (60 mL) versetzt und mit Ethylacetat 3-mal extrahiert (3x50 mL). Die Ethylacetatphase wird getrocknet (Na₂SO₄ sicc.) und eingeengt. Der Rückstand (0,91 g, Gehalt 83,5 % n. HPLC, 90 % Ausbeute) wird zunächst aus wäßrigem EtOH (50 % G/G; 6 mL) kristallisiert (Gehalt nach HPLC: 89,5%) und anschließend aus absolutem Ethanol (4 mL) kristallisiert und getrocknet: (0,25 g, Gehalt n. HPLC: 96.8%). Aus der Mutterlauge wurden weitere 0,15 g erhalten (96 % nach HPLC).
Y: 47,4 %, (0.4 g), C₁₇H₁₅F₇N₂O₂S, MW = 444.37;
Mp: 199.60 °C
IR (NaCl): 1/λ (cm⁻¹) = 3340, 3253, 1598, 1338, 1276, 1221, 1162, 1145, 1132, 1092, 982, 968, 841, 739, 729, 551;
¹HNMR (DMSO-d6): δ (ppm) = 7.85 und 7.37 (AA'BB', arom, 4H, J=8,4), 4.85 (NH₂), 3.95 (t, 2H, CH₂, J=7,0), 2.68 (t, 2H, CH₂, J=7,0), 2.49 (quin, 2H, CH₂, J=7,0), 2.29 (s, 3H, CH₃); GC-MS (EI, 70eV): m/z (rel Int. %)= 444 (100); 226 (52); 295 (50); 445 (21); 345 (11); 443 (09); 296 (09); 227 (09); 446 (07); 275 (07); 225 (07); 364 (06); 294 (05)

### Beispiel 40

### 4-[2-(1,1,1,2,2,3,3-Heptafluor-4-butyl)-3-methyl-6,7-dihydro-5H-pyrrolizin-1-yl]-benzolsulfonsäureamid

### a) 1,1,1,2,2,3,3-Heptafluor-4-(3-Methyl-1-phenyl-6,7-dihydro-5H-pyrrolizin-2-yl)-butan-4-on

5-Methyl-7-phenyl-2,3-dihydro-1H-pyrrolizin (Beispiel 30 c, 1.0 g, 0.005 Mol), gelöst in Dichlorethan (20 mL) wird bei RT mit Heptafluorbuttersäurechlorid (0.83 mL, 1.3 g, 0.0055 Mol) versetzt und zuletzt Zinntetrachlorid (0.7 mL, 1,56 g, 0.006 Mol) in Dichlorethan (5 mL) zugetropft. Die Mischung erwärmt sich auf 35 °C und wird 16 h bei RT gerührt. Die GC-MS Analyse zeigt eine 60%ige Umsetzung. Die Reaktionsmischung wird im Eisbad mit NaOH (25 %, 25 mL) alkalisiert und nach Zugabe von CH₂Cl₂ (25 mL) wird die organische Phase im Scheidetrichter abgetrennt. Die Alkaliphase wird noch zweimal mit Dichlormethan (2x20 mL) extrahiert. Diese CH₂Cl₂-Extrakte werden vereint, über Na₂SO₄ sicc. getrocknet und im Vakuum zur Trockne eingedampft.
Der Rückstand (1.4 g) wird sc gereinigt (SiO₂ (120g), Hexan-Diethylether 7:3): im Vorlauf werden 0,46 g Edukt zurückerhalten. Aus einer Zwischenfraktion werden in einem zweiten Trenngang 0,180 g und im Nachlauf der ersten Trennung 0,62 g Produkt, zusammen 0,8 g (40,7 % d. Th.) erhalten.
Y: 40,7 % (0.8 g); C₁₈H₁₄F₇NO, MW = 393.31;
Mp: 77.0 °C,
IR (NaCl): 1/λ (cm⁻¹) = 1667, 1505, 1422, 1384, 1343, 1239, 1213, 1175, 1117, 1093, 870, 764, 717, 703;
¹HNMR (CDCl₃): δ (ppm) = 7.37 (m, 5H, arom), 3.95 (t, 2H, CH₂), 2.89 (t, 2H, CH₂), 2.53 (quin, 2H, CH₂), 2.39 (s, 3H, CH₃);
GC-MS (EI, 70eV): m/z (rel. Int. %) = 224.1 (100); 393.1 (35); 225.1 (18); 394.1 (07); 127.0 (06).

### b) 2-(1,1,1,2,2,3,3-Heptafluor-4-butyl)-3-methyl-1-phenyl-6,7-dihydro-5H-pyrrolizin

Die Lösung von 1,1,1,2,2,3,3-Heptafluor-4-(3-Methyl-1-phenyl-6,7-dihydro-5H-pyrrolizin-2-yl)-butan-4-on (0.62 g, 1.6 mMol) in Dichlorethan (20 mL) wird nacheinander mit NaCNBH₃ (0.42 g, 6.4 mMol) und ZnI₂ (0.71 g, 2.24 mMol) versetzt und 4 Tage (d) unter Rückfluß gekocht. Nach 2 Tagen werden nochmals NaCNBH₃ (0.42 g, 6.4 mMol) und ZnI₂ (0.71 g, 2.24 mMol) zugesetzt. Die Umsetzung wird mittels GC-MS beobachtet. Nach 2 Tagen liegen 40%, nach 4 Tagen 89 % der eingesetzten Verbindung umgesetzt vor, neben weiteren 11% Edukt. Die Reaktion wird daraufhin durch Zugabe von verdünnter Phosphorsäure (8%, 50 mL) abgebrochen. Nach Beendigung der Gasentwicklung wird mit Methylenchlorid extrahiert, die organische Phase mit Wasser (50 mL) gewaschen und das Lösungsmittel nach Trocknen über Na₂SO₄ sicc. im Vakuum abgezogen. Der Rückstand (0.65 g, 82,4 %, 77% ig nach GC-MS(TIC)) wird ohne weitere Reinigung in der Folgereaktion eingesetzt..

Y: 82.4 % (0.65 g, 77% n. GC-MS (TIC)); C₁₈H₁₆F₇N, MW = 379.32;
Mp: 95.4 °C
IR (NaCl): 1/λ (cm⁻¹) = 2928,1604, 1446, 1425, 1352, 1221, 1112, 1059, 963, 763, 745, 702;
¹HNMR (CDCl₃): δ (ppm) = 7.36-7.20 (m, 5H, arom), 3.91 (t, 2H, CH₂, J=7,1), 3.28 (t, 2H, CH₂, J=20,0), 2.93 (t, 2H, CH₂, J=7,1), 2.47 (q, 2H, CH₂, J=7,1), 2.21 (s, 3H, CH₃);
MS(EI, 70eV): m/z (rel Int. %)= 379 (56); 210 (100); 211 (17); 208 (13); 380 (12).

### c) 4-[2-(1,1,1,2,2,3,3-Heptafluor-4-butyl)-3-methyl-6,7-dihydro-5H-pyrrolizin-1-yl]-benzolsulfonsäureamid

2-(1,1,1,2,2,3,3-Heptafluor-4-butyl)-3-methyl-1-phenyl-6,7-Mydro-5H-pyrrolizin (0.45 g, 77%, 1.1 mMol) wird in CHCl₃ abs. (3 mL) gelöst, die Lösung wird auf 0°C gekühlt und Chlorsulfonsäure (0,66 mL, 1,15 g, 10 mMol) wird langsam zugetropft. Die Mischung wird 2.5 h (58 - 61 °C) erhitzt (DC: SiO₂, Ether-Hexan 1:1, Edukt Rf 0.8, Sulfonsäure Rf 0.0, Chlorsulfonsäure Rf 0.5). Die Reaktionsmischung wird auf Eis (20 mL) abgelöscht, 3-mal mit CHCl₃ (3x25 mL) extrahiert und die gesammelte CHCl₃-Lösung wird gewaschen (30 mL ges. NaCl-Lösung), getrocknet (Na₂SO₄ sicc.) und eingeengt (Rückstand: 1.0 g). Das Chlorsulfonierungsprodukt (1.0 g) wird in THF abs. (10 mL) gelöst und es wird langsam konz. NH₄OH (25%ig, 2,5 mL) zugetropft. Die Mischung wird 16 h bei RT gerührt, mit halbkonz. NH₄Cl-Lösung (20 mL) versetzt und mit Ethylacetat 3-mal extrahiert (3x50 mL). Die gewaschene (40 mL ges. NaCl-Lösung) Ethylacetatphase wird getrocknet (Na₂SO₄ sicc.) und eingeengt. Der Rückstand (0.3 g) wird mit Ether als Fließmittel auf SiO₂ (30 g) getrennt: aus Fraktion 3-6: 0.15 g, (Gehalt 87%) und anschließend 2 x mit Ether (0,5 ml) gewaschen und getrocknet: 0.09 g (90.2 % n. HPLC) Fraktion 7-10: 0.05 g (91.2% n. HPLC).
Y: 29.7 %, (0.14 g, Gehalt 90.7%), C₁₈H₁₇F₇N₂O₂S, MW = 458.4;
Mp: 220.7 °C
IR (NaCl): 1/λ (cm⁻¹) = 3344, 3247, 1597, 1352, 1334, 1217, 1113, 1053, 747, 545;
¹HNMR (DMSO-d6): δ (ppm) = 7.89 und 7.39 (AA'BB', 4H, arom, J=8.6), 5.96 (s, 2H, NH₂), 3.93 (t, 2H, CH₂, J=7.0), 3.30 (t, 2H, CH₂, J=19.8), 2.93 (t, 2H, CH₂, J=7.1), 2.53 (q, 2H, CH₂, J=7.1), 2.21 (s, 3H, CH₃).

Die Verbindung des Beispiels 41 wird nach analogen Vorschriften hergestellt.

### Biologische Aktivität

### Testsystem zur Bestimmung der Hemmung der 5-Lipoxygenase

Als Quelle für die 5-Lipoxygenase dienen menschliche Granulozyten, Durch Stimulation mit Calcium-Ionophor A 23187 wird LTB₄ (Leukotrien B₄) aus endogener Arachidonsäure gebildet, Die Isolierung der Granulozyten und die Durchführung der Enzymreaktion erfolgt nach bekannten Verfahren (siehe Arch. Pharm. 330, 307 - 312 (1997)).

Das mit Heparin vor Gerinnung geschützte Blut wird über einem diskontinuierlichen Percoll®-Gradienten zentrifugiert und die Granulozytenschicht abpipettiert, Nach Lyse der Erythrozyten werden die Granulozyten mehrmals gewaschen und anschließend auf eine bestimmte Zellzahl eingestellt, Die Enzymreaktion wird dann in An- bzw, Abwesenheit der Testsubstanz nach Zugabe von Ca²⁺ mit Calcium-Ionophor A 23187 gestartet, Die Synthese der Leukotriene wird nach 1.5 Minuten gestoppt, Die Proben werden abzentrifugiert, der Überstand verdünnt, Die quantitative Bestimmung von LTB₄ erfolgt mittels ELISA.

### Testsystem zur Bestimmung der Hemmung der Cyclooxygenase-1

Bei diesem Testsystem wird die von menschlichen Thrombozyten nach Zusatz von Calcium-Ionophor gebildete Prostaglandin E₂-Menge mittels ELISA bestimmt, Dabei werden die Thrombozyten nach Zentrifugation über einen diskontinuierlichen Percoll®-Gradienten gewonnen, Die Enzymreaktion und die Bestimmung der gebildeten Metaboliten erfolgt prinzipiell wie bei der Bestimmung der 5-Lipoxygenase-Hemmung, Unterschiede bestehen hinsichtlich der Inkubationszeit, Weiterhin ist die Zugabe eines Thromboxansynthase-Hemmstoffes notwendig (siehe Arch. Pharm. Pharm. Med. Chem. 330, 307 - 312 (1997)).

### Testsystern zur Bestimmung der Hemmung der Cyclooxygenase-2

COX₂ (aus Placenta des Schafs) wird mit Testsubstanz 10 min bei 4°C vorinkubiert, dann mit Arachidonsäure (5 µM) bei 25°C 10 min, stimuliert, Als Referenz dient Diclofenac (IC₅₀(COX₂) = 3.0 10⁻⁶ M), Die Bestimmung erfolgt in 3 Verdünnungen (10⁻⁷, 10⁻⁶, 10⁻⁵ M), Die PGE₂-Konzentrationen werden mittels ELISA quantifiziert (siehe Mitchell J.A, et al. Proc. Nat. Acad. Sci. 90: 11693-11697 (1993)).

## Patentansprüche

1. [α]-anellierte Pyrrolverbindungen der Formel I 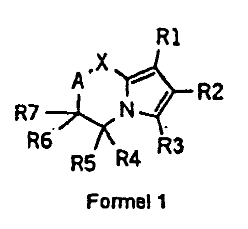 worin
X für CR8R9, S, O, NR12 oder C(O) steht;
A für CR10R11 oder eine Bindung zwischen X und dem die Reste R6 und R7 tragenden Atom steht;
der erste der Reste R1, R2, R3 für
4-substituiertes Phenyl steht, wobei der Substituent ausgewählt ist unter C₁₋₄-Alkylthio, C₁₋₄-Alkylsulfinyl, C₁₋₄-Alkylsulfonyl, Sulfamoyl, N-C₁₋₄-Alkylsulfamoyl, N,N-Di-C₁₋₄-Alkylsulfamoyl, C₁₋₄-Alkylsulfonamido oder C₁₋₄-Alkylsulfon-N-C₁₋₄-alkylamido;
der zweite der Reste R1, R2, R3 für
C₁₋₆-Alkyl, das gegebenenfalls einfach, zweifach oder dreifach bzw. mit Halogen auch mehrfach mit unter Halogen, C₃₋₇-Cycloalkyl, C₁₋₄-Alkoxy, Trifluormethoxy, Hydroxy oder Trifluormethyl ausgewählten, gleichen oder verschiedenen Substituenten substituiert ist,
C₃₋₇-Cycloalkyl, das gegebenenfalls einfach, zweifach oder dreifach bzw. mit Halogen auch mehrfach mit unter Halogen, C₁₋₄-Alkyl, Halogen-C₁₋₄-alkyl, C₃₋₇-Cycloalkyl, C₁₋₄-Alkoxy, Halogen-C₁₋₄-alkoxy oder Hydroxy ausgewählten, gleichen oder verschiedenen Substituenten substituiert ist,
Phenyl, das gegebenenfalls einfach, zweifach oder dreifach bzw. mit Halogen auch mehrfach mit unter Halogen, C₁₋₄-Alkyl, Halogen-C₁₋₄-alkyl, C₁₋₄-Alkoxy, Halogen-C₁₋₄-alkoxy, C₁₋₄-Alkylthio, Hydroxy, Nitro, C₁₋₄-Alkylsulfinyl, C₁₋₄-Alkylsulfonyl, Sulfamoyl, N-C₁₋₄-Alkylsulfamoyl, N,N-Di-C₁₋₄-Alkylsulfamoyl, C₁₋₄-Alkylsulfonamido oder C₁₋₄-Alkylsulfon-N-C₁₋₄-alkylamido ausgewählten, gleichen oder verschiedenen Substituenten substituiert ist; oder
einen 5- oder 6-gliedrigen, heterocyclischen Rest, der aromatisch oder nicht-aromatisch, mono- oder bicyclisch, und gegebenenfalls benzoanelliert sein kann und der 1, 2 oder 3, unter N, O und S unabhängig voneinander ausgewählte Heteroatome aufweist und gegebenenfalls einfach, zweifach oder dreifach bzw. mit Halogen auch mehrfach mit unter Halogen, C₁₋₄-Alkyl, Halogen-C₁₋₄-alkyl, C₁₋₄-Alkoxy, Halogen-C₁₋₄-alkoxy, C₁₋₄-Alkylthio, Hydroxy, Nitro, C₁₋₄-Alkylsulfinyl, C₁₋₄-Alkylsulfonyl, Sulfamoyl, N-C₁₋₄-Alkylsulfamoyl, N,N'-Di-C₁₋₄-Alkylsulfamoyl, C₁₋₄-Alkylsulfonamido oder C₁₋₄-Alkylsulfon-N-C₁₋₄-alkylamido ausgewählten, gleichen oder verschiedenen Substituenten substituiert ist, steht;
der dritte der Reste R1, R2, R3 für
H, C₁₋₆-Alkyl, Halogen-C₁₋₄-alkyl, Hydroxy-C₁₋₆-alkyl, -CHO, -COOH, -COCOOH, -COO-C₁₋₄-Alkyl, -COO-C₁₋₄-AlkPhenyl, -COCOO-C₁₋₄-Alkyl, Halogen, Cyano, C₁₋₄-Alkylsulfonyl, Sulfamoyl oder B-Y steht;
worin
B für C₁₋₈-Alkylen oder C₂₋₈-Alkenylen steht, das gegebenenfalls durch Hydroxyl oder C₁₋ ₄-Alkoxy substituiert sein kann;
Y für -COOH, -COO-C₁₋₄-Alkyl, -SO₃-C₁₋₄-Alkyl, -CHO oder Hydroxy steht; oder
der zweite und der dritte der Reste R1, R2, R3 zusammen mit den C-Atomen, an die sie gebunden sind, für gesättigtes oder ungesättigtes C₃-C₇-Cycloalkyl stehen;
R4-R11, die gleich oder verschieden sein können, für Wasserstoff, C₁₋₆-Alkyl, Hydroxy-C₁₋₆alkyl, C₁₋₄-Alkoxy-C₁₋₆-alkyl, Hydroxyl, COOH oder Acyloxy stehen, wobei vicinale Reste auch für Bindungen oder geminale Reste auch zusammen mit dem C-Atom, an das sie gebunden sind, für Carbonyl oder für C₃-C₇-Cycloalkyl stehen können;
R12 für Wasserstoff, C₁₋₆-Alkyl oder Phenyl steht,
und optische Isomere, physiologisch verträgliche Salze und physiologisch hydrolysierbare Ester davon, wobei die physiologisch hydrolysierbaren Ester ausgewählt sind unter Alkyl-, Pivaloyloxymethyl-, Acetoxymethyl-, Phthalidyl-, Indanyl- und Methoxymethylester.

2. Verbindungen nach Anspruch 1, worin X für CR8R9, A für eine Bindung zwischen X und dem die Reste R6 und R7 tragenden Atom und R4, R5, R6, R7, R8, R9, die gleich oder verschieden sein können, für Wasserstoff oder C₁₋₄-Alkyl stehen.

3. Verbindungen nach Anspruch 1 oder 2, worin der zweite der Reste R1, R2, R3 für 4-substituiertes Phenyl steht.

4. Verbindungen nach Anspruch 3, worin der Substituent Fluor, Methyl oder Trifluormethyl ist.

5. Verbindungen nach Anspruch 1 oder 2, worin der zweite der Reste R1, R2, R3 für C₁₋₆-Alkyl, C₃₋₇-Cycloalkylmethyl oder C₃₋₇-Cycloalkyl steht.

6. Verbindungen nach Anspruch 1 oder 2, worin der zweite der Reste R1, R2, R3 für polyfluoriertes C₁₋₆-Alkyl, C₃₋₇-Cycloalkylmethyl oder C₃₋₇-Cycloalkyl steht.

7. Verbindungen nach einem der vorhergehenden Ansprüche, worin der dritte der Reste R1, R2, R3 für Wasserstoff, C₁₋₆-Alkyl, CF₃ oder Halogen steht.

8. Verbindungen nach einem der vorhergehenden Ansprüche, worin der erste und der zweite der Reste R1, R2, R3 vicinal zueinander stehen.

9. Verbindungen nach einem der vorhergehenden Ansprüche, worin R1 der erste der Reste R1, R2, R3 ist.

10. Pharmazeutisches Mittel, enthaltend wenigstens eine Verbindung nach einem der Ansprüche 1 bis 9, gegebenenfalls zusammen mit pharmazeutisch akzeptablen Hilfsstoffen.

11. Verwendung wenigstens einer Verbindung nach einem der Ansprüche 1 bis 9 zur Herstellung eines pharmazeutischen Mittels zur Behandlung von Erkrankungen des rheumatischen Formenkreises.

## Claims

1. [α]-Fused pyrrole compounds of the formula 1 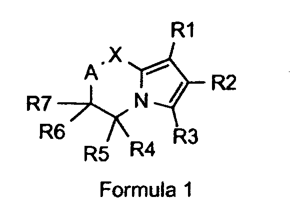 in which
X is CR8R9, S, O, NR12 or C(O);
A is CR10R11 or a bond between X and the atom carrying the radicals R6 and R7;
the first of the radicals R1, R2, R3 is
4-substituted phenyl, the substituent being selected from C₁₋₄-alkylthio, C₁₋₄-alkylsulphinyl, C₁₋₄-alkylsulphonyl, sulphamoyl, N-C₁₋₄-alkylsulphamoyl, N,N-di-C₁₋₄-alkylsulphamoyl, C₁₋₄-alkylsulphonamido or C₁₋₄-alkylsulphone-N-C₁₋₄-alkylamido;
the second of the radicals R1, R2, R3 is
C₁₋₆-alkyl which is optionally mono-, di- or tri- and, with halogen, also poly-substituted by identical or different substituents selected from halogen, C₃₋₇-cycloalkyl, C₁₋₄-alkoxy, trifluoromethoxy, hydroxyl or trifluoromethyl,
C₃₋₇-cycloalkyl which is optionally mono-, di- or tri- and, with halogen, also poly-substituted by identical or different substituents selected from halogen, C₁₋₄-alkyl, halo-C₁₋₄-alkyl,
C₃₋₇-cycloalkyl, C₁₋₄-alkoxy, halo-C₁₋₄-alkoxy or hydroxyl,
phenyl which is optionally mono-, di- or tri- and, with halogen, also poly-substituted by identical or different substituents selected from halogen, C₁₋₄-alkyl, halo-C₁₋₄-alkyl, C₁₋₄-alkoxy, halo-C₁₋₄-alkoxy, C₁₋₄-alkylthio, hydroxyl, nitro, C₁₋₄-alkylsulphinyl, C₁₋₄-alkylsulphonyl, sulphamoyl, N-C₁₋₄-alkylsulphamoyl, N,N-di-C₁₋₄-alkylsulphamoyl, C₁₋₄-alkylsulphonamido or C₁₋₄-alkylsulphone-N-C₁₋₄-alkylamido; or
a 5- or 6-membered heterocyclic radical which may be aromatic or non-aromatic, mono- or bicyclic, and optionally benzo-fused, and which contains 1, 2 or 3 heteroatoms independently of one another selected from N, O and S and is optionally mono-, di- or tri- and, with halogen, also poly-substituted by identical or different substituents selected from halogen, C₁₋₄-alkyl, halo-C₁₋₄-alkyl, C₁₋₄-alkoxy, halo-C₁₋₄-alkoxy, C₁₋₄-alkylthio, hydroxyl, nitro, C₁₋₄-alkylsulphinyl, C₁₋₄-alkylsulphonyl, sulphamoyl, N-C₁₋₄-alkylsulphamoyl, N,N-di-C₁₋₄-alkylsulphamoyl, C₁₋₄-alkylsulphonamido or C₁₋₄-alkylsulphone-N-C₁₋₄-alkylamido;
the third of the radicals R1, R2, R3 is
H, C₁₋₆-alkyl, halo-C₁₋₄-alkyl, hydroxy-C₁₋₆-alkyl, -CHO, -COOH, -COCOOH, - COO-C₁₋₄-alkyl, -COO-C₁₋₄-Alkphenyl, -COCOO-C₁₋₄-alkyl, halogen, cyano, C₁₋₄-alkylsulphonyl, sulphamoyl or B-Y;
in which
B is C₁₋₈-alkylene or C₂₋₈-alkyenylene, each of which can optionally be substituted by hydroxyl or C₁₋₄-alkoxy;
Y is -COOH, -COO-C₁₋₄-alkyl, -SO₃-C₁₋₄-alkyl, -CHO or hydroxyl; or
the second and the third of the radicals R1, R2, R3, together with the C atoms to which they are bonded, are saturated or unsaturated C₃-C₇-cycloalkyl;
R4-R11, which can be identical or different, are hydrogen, C₁₋₆-alkyl, hydroxy-C₁₋₆-alkyl, C₁₋₄-alkoxy-C₁₋₆-alkyl, hydroxyl, COOH or acyloxy, where vicinal radicals can also represent bonds or geminal radicals, also together with the C atom to which they are bonded, can represent carbonyl or C₃-C₇-cycloalkyl;
R12 is hydrogen, C₁₋₆-alkyl or phenyl,
and optical isomers, physiologically tolerable salts and physiologically hydrolysable esters thereof, wherein the physiologically hydrolysable esters are selected from alkyl, pivaloyloxymethyl, acetoxymethyl, phthalidyl, indanyl and methoxymethyl esters.

2. Compounds according to Claim 1, in which X is CR8R9, A is a bond between X and the atom carrying the radicals R6 and R7 and R4, R5, R6, R7, R8, R9, which can be identical or different, are hydrogen or C₁₋₄-alkyl.

3. Compounds according to Claim 1 or 2, in which the second of the radicals R1, R2, R3 is 4-substituted phenyl.

4. Compounds according to Claim 3, in which the substituent is fluoro, methyl or trifluoromethyl.

5. Compounds according to Claim 1 or 2, in which the second of the radicals R1, R2, R3 is C₁₋₆-alkyl, C₃₋₇-cycloalkylmethyl or C₃₋₇-cycloalkyl.

6. Compounds according to Claim 1 or 2, in which the second of the radicals R1, R2, R3 is polyfluorinated C₁₋₆-alkyl, C₃₋₇-cycloalkylmethyl or C₃₋₇-cycloalkyl.

7. Compounds according to one of the preceding claims, in which the third of the radicals R1, R2, R3 is hydrogen, C₁₋₆-alkyl, CF₃ or halogen.

8. Compounds according to one of the preceding claims, in which the first and the second of the radicals R1, R2, R3 are vicinal to one another.

9. Compounds according to one of the preceding claims, in which R1 is the first of the radicals R1, R2, R3.

10. Pharmaceutical composition, comprising at least one compound according to one of Claims 1 to 9, if appropriate together with pharmaceutically acceptable excipients.

11. Use of at least one compound according to one of Claims 1 to 9 for the production of a pharmaceutical composition for the treatment of disorders of the rheumatic type.

## Revendications

1. Dérivés [α]-condensés du pyrrole, de formule : 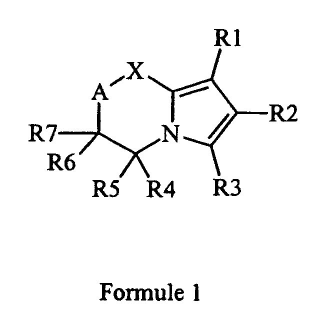 dans laquelle
X représente CR8R9, S, O, NR12 ou C(O) ;
A représente CR10R11 ou une liaison entre X et l'atome portant les restes représentés par R6 et R7 ;
un premier des restes représentés par R1, R2 et R3 est un groupe phényle portant, en position 4, un substituant choisi parmi les groupes alkylthio en C₁₋₄, alkylsulfinyle en C₁₋₄, alkylsulfonyle en C₁₋₄, sulfamyle, N-(alkyle en C₁₋₄)sulfamyle, N,N-di(alkyle en C₁₋₄)sulfamyle, (alkyle en C₁₋₄)sulfonamido, ou (alkyle en C₁₋₄)sulfon-N-(alkyle en C₁₋₄)amido ;
un deuxième des restes représentés par R1, R2 et R3 est :
- un groupe alkyle en C₁₋₆, qui porte éventuellement un, deux ou trois sub-stituants (ou encore plus s'il s'agit d'atomes d'halogène), identiques
ou différents, choisis parmi les atomes d'halogène et les groupes cycloalkyle en C₃₋₇, alcoxy en C₁₋₄, trifluorométhoxy, hydroxy et trifluorométhyle,
- un groupe cycloalkyle en C₃₋₇, qui porte éventuellement un, deux ou trois substituants (ou encore plus s'il s'agit d'atomes d'halogène), identiques ou différents, choisis parmi les atomes d'halogène et les groupes alkyle en C₁₋₄, halogénoalkyle en C₁₋₄, cycloalkyle en C₃₋₇, alcoxy en C₁₋₄, halogénoalcoxy en C₁₋₄ et hydroxy,
- un groupe phényle, qui porte éventuellement un, deux ou trois substituants (ou encore plus s'il s'agit d'atomes d'halogène), identiques ou différents, choisis parmi les atomes d'halogène et les groupes alkyle en C₁₋₄, halogénoalkyle en C₁₋₄, alcoxy en C₁₋₄, halogénoalcoxy en C₁₋₄, alkylthio en C₁₋₄, hydroxy, nitro, alkylsulfinyle en C₁₋₄, alkylsulfonyle en C₁₋₄, sulfamyle, N-(alkyle en C₁₋₄)sulfamyle, N,N-di(alkyle en C₁₋₄)-sulfamyle, (alkyle en C₁₋₄)sulfonamido et (alkyle en C₁₋₄)sulfon-N-(alkyle en C₁₋₄)-amido,
- ou un groupe hétérocyclique à 5 ou 6 chaînons, qui peut être aromatique ou non-aromatique, monocyclique ou bicyclique, et le cas échéant, benzo-condensé, qui comporte 1, 2 ou 3 hétéroatomes, choisis indépendamment l'un de l'autre parmi les atomes N, O et S, et qui porte éventuellement un, deux ou trois substituants (ou encore plus s'il s'agit d'atomes d'halogène), identiques ou différents, choisis parmi les atomes d'halogène et les groupes alkyle en C₁₋₄, halogénoalkyle en C₁₋₄, alcoxy en C₁₋₄, halogénoalcoxy en C₁₋₄, alkylthio en C₁₋₄, hydroxy, nitro, alkylsulfinyle en C₁₋₄, alkylsulfonyle en C₁₋₄, sulfamyle, N-(alkyle en C₁₋₄)sulfamyle, N,N-di(alkyle en C₁₋₄)sulfamyle, (alkyle en C₁₋₄)sulfonamido et (alkyle en C₁₋₄)sulfon-N-(alkyle en C₁₋₄)amido ;
et le troisième des restes représentés par R1, R2 et R3 est un atome d'hydrogène ou d'halogène ou un groupe alkyle en C₁₋₆, halogénoalkyle en C₁₋₄, hydroxyalkyle en C₁₋₆, -CHO, -COOH, -COCOOH, -COO-(alkyle en C₁₋₄), -COO-(alkylène en C₁₋₄)-phényle, -COCOO-(alkyle en C₁₋₄), cyano, alkylsulfonyle en C₁₋₄ ou sulfamyle, ou encore un groupe B-Y où B représente un groupe alkylène en C₁₋₈ ou alcénylène en C₂₋₈, qui porte éventuellement un substituant hydroxy ou alcoxy en C₁₋₄, et Y représente un groupe -COOH, -COO-(alkyle en C₁₋₄), -SO₃-(alkyle en C₁₋₄), -CHO ou hydroxy ;
ou bien le deuxième et le troisième des restes représentés par R1, R2 et R3 forment ensemble, conjointement avec les atomes de carbone auxquels ils sont liés, un groupe cycloalkyle en C₃₋₇, saturé ou insaturé ;
les restes représentés par R4 à R11, qui peuvent être identiques ou différents, sont chacun un atome d'hydrogène ou un groupe alkyle en C₁₋₆, hydroxyalkyle en C₁₋₆, (alcoxy en C₁₋₄)alkyle en C₁₋₆, hydroxy, -COOH ou acyloxy, les groupes vicinaux pouvant aussi former ensemble des liaisons, et les groupes géminés pouvant aussi former, conjointement avec l'atome de carbone auquel ils sont liés, un groupe carbonyle ou cycloalkyle en C₃₋₇ ;
et R12 représente un atome d'hydrogène ou un groupe alkyle en C₁₋₆ ou phényle ;
et isomères optiques, sels physiologiquement compatibles et esters physiologiquement hydrolysables de ces dérivés, ces esters physiologiquement hydrolysables étant choisis parmi les esters d'alkyle, de pivaloyloxyméthyle, d'acétoxyméthyle, de phtalidyle, d'indanyle et de méthoxyméthyle.

2. Dérivés conformes à la revendication 1, dans lesquels X représente CR8R9, A représente une liaison entre X et l'atome qui porte les restes représentés par R6 et R7, et R4, R5, R6, R7, R8 et R9, dont les significations peuvent être identiques ou différentes, représentent chacun un atome d'hydrogène ou un groupe alkyle en C₁₋₄.

3. Dérivés conformes à la revendication 1 ou 2, dans lesquels le deuxième des restes représentés par R1, R2 et R3 est un groupe phényle portant un substituant en position 4.

4. Dérivés conformes à la revendication 3, dans lesquels le substituant est un atome de fluor ou un groupe méthyle ou trifluorométhyle.

5. Dérivés conformes à la revendication 1 ou 2, dans lesquels le deuxième des restes représentés par R1, R2 et R3 est un groupe alkyle en C₁₋₆, (cycloalkyle en C₃₋₇)méthyle ou cycloalkyle en C₃₋₇.

6. Dérivés conformes à la revendication 1 ou 2, dans lesquels le deuxième des restes représentés par R1, R2 et R3 est un groupe alkyle en C₁₋₆, (cycloalkyle en C₃₋₇)méthyle ou cycloalkyle en C₃₋₇, polyfluoré.

7. Dérivés conformes à l'une des revendications précédentes, dans lesquels le troisième des restes représentés par R1, R2 et R3 est un atome d'hydrogène ou d'halogène ou un groupe alkyle en C₁₋₆ ou CF₃.

8. Dérivés conformes à l'une des revendications précédentes, dans lesquels le premier et le deuxième des restes représentés par R1, R2 et R3 sont placés en positions vicinales l'un par rapport à l'autre.

9. Dérivés conformes à l'une des revendications précédentes, dans lesquels c'est R1 qui est le premier des restes représentés par R1, R2 et R3.

10. Agent pharmaceutique qui contient au moins un dérivé conforme à l'une des revendications 1 à 9, éventuellement associé à des adjuvants pharmacologiquement admissibles.

11. Utilisation d'au moins un dérivé conforme à l'une des revendications 1 à 9 pour la préparation d'un agent pharmaceutique destiné au traitement de maladies faisant partie du groupe des affections rhumatismales.
